# EUROPEAN PATENT SPECIFICATION

(11) **EP 3 285 751 B1**
(45) Date of publication and mention of the grant of the patent: **07.10.2020**
(21) Application number: 16783970.3
(22) Date of filing: 22.04.2016
(51) Int. Cl.: C07J 41/00, A61K 31/10, A61K 31/56, A61P 31/02

(54) **CATIONIC STEROIDAL ANTIMICROBIAL SALTS**
KATIONISCHE STEROIDALE ANTIMIKROBIELLE SALZE
SELS ANTIMICROBIENS STÉROÏDIENS CATIONIQUES

(30) Priority: 22.04.2015 US 201562151019 P; 21.05.2015 US 201562165013 P; 13.07.2015 US 201562191916 P
(43) Date of publication of application: 28.02.2018
(73) Proprietor: Brigham Young University, Provo, UT 84602 (US)
(72) Inventor: SAVAGE, Paul B., Mapleton, Utah 84664 (US); CHITRE, Saurabh Shashikant, Midlothian EH19 2DP (GB); VARIA, Kunal Arvind, Ghatkopar (East) Maharashtra Mumbai 400 047 (IN); REECE, Hayley Ann, Edinburgh EH16 6AW (GB); JACKS, Thomas Elliot, Hillsborough, New Jersey 08844 (US); MILLER, Ross Allen, Fanwood, New Jersey 07023 (US); RANDALL, Jared Lynn, Smyrna, New York 13464 (US)
(74) Representative: Schwarz & Partner Patentanwälte OG
(86) International application number: PCT/US2016/028921
(87) International publication number: WO 2016/172534

(56) References cited:
- WO-A1-2013/040265
- WO-A1-2014/062960
- WO-A1-2014/151411
- WO-A2-2014/107740
- US-A1- 2014 336 131

## Description

### BACKGROUND

### 1. Field

The present application relates to the fields of pharmaceutical chemistry, biochemistry, and medicine. In particular, the present application relates to acid addition salts of cationic steroidal antimicrobials ("CSAs" or "ceragenins").

### 2. Related Technology

Endogenous antimicrobial peptides, such as the human cathelicidin LL-37, play key roles in innate immunity. LL-37 is found in airway mucus and is believed to be important in controlling bacterial growth in the lung. Antimicrobial peptides are found in organisms ranging from mammals to amphibians to insects to plants. The ubiquity of antimicrobial peptides has been used as evidence that these compounds do not readily engender bacterial resistance. In addition, considering the varied sequences of antimicrobial peptides among diverse organisms, it is apparent that they have evolved independently multiple times. Thus, antimicrobial peptides appear to be one of "Nature's" primary means of controlling bacterial growth. However, clinical use of antimicrobial peptides presents significant issues including the relatively high cost of producing peptide-based therapeutics, the susceptibility of peptides to proteases generated by the host and by bacterial pathogens, and deactivation of antimicrobial peptides by proteins and DNA in lung mucosa.

An attractive means of harnessing the antibacterial activities of antimicrobial peptides without the issues delineated above is to develop non-peptide mimics of antimicrobial peptides that display the same broad-spectrum antibacterial activity utilizing the same mechanism of action. Non-peptide mimics would offer lower-cost synthesis and potentially increased stability to proteolytic degradation. In addition, control of water solubility and charge density may be used to control association with proteins and DNA in lung mucosa.

With over 1,600 examples of antimicrobial peptides known, it is possible to categorize the structural features common to them. While the primary sequences of these peptides vary substantially, morphologies adopted by a vast majority are similar. Those that adopt alpha helix conformations juxtapose hydrophobic side chains on one face of the helix with cationic (positively charged) side chains on the opposite side. As similar morphology is found in antimicrobial peptides that form beta sheet structures: hydrophobic side chains on one face of the sheet and cationic side chains on the other.

We have developed small molecule, non-peptide mimics of antimicrobial peptides, termed ceragenins or CSAs. These compounds reproduce the amphiphilic morphology in antimicrobial peptides, represented above by CSA-13, and display potent, as well as diverse, biological activities (including, but not limited to anti-bacterial, anti-cancer, antiinflammatory, promoting bone growth, promoting wound healing, etc.). Lead ceragenins can be produced at a large scale, and because they are not peptide based, they are not substrates for proteases. Consequently, the ceragenins represented an attractive compound class for producing pharmaceutically-relevant treatments. WO 2014/107740 A2 discloses a 1,5-naphthalene disulfonic acid addition salt.

### SUMMARY

The present invention relates to a 1,5-naphthalenedisulfonic acid addition salt of a cationic steroidal antimicrobial (CSA) as defined in the claims.

In some embodiments, the acid addition salt is a solid. In some embodiments, the solid is a flowable solid. In some embodiments, the acid addition salt is crystalline. In some embodiments, the acid addition salt is storage stable. In some embodiments, the salt is micronized.

Some embodiments provide a formulation comprising an acid addition salt of a CSA and a pharmaceutically acceptable excipient.

Some embodiments provide a process for preparing a CSA salt, comprising diluting the free base of a CSA with a solvent; adding at least one equivalent of an acid to the diluted CSA in solvent to afford a reaction mixture; precipitating or temperature cycling the reaction mixture; and isolating a CSA salt.

In some embodiments, the temperature cycling is conducted for at least about 48 hours. In some embodiments, the process further comprises utilizing an anti-solvent or evaporation of solvent when isolating the CSA salt.

In some embodiments, the CSA salt is a solid. In some embodiments, the CSA salt is crystalline. In some embodiments, the CSA salt is amorphous. In some embodiments, the CSA salt is storage stable. In some embodiments, the CSA salt is flowable. In some embodiments, the CSA salt is micronized.

Advantanges of the CSA compounds disclosed herein include, but are not limited to, comparable and/or improved antimicrobial activity, stability, and/or pharmaceutical administerability compared to existing CSA compounds and/or simplified synthetis of final CSA compounds and/or intermediate CSA compounds compared to existing synthetic routes.

Additional features and advantages will be set forth in part in the description that follows, and in part will be obvious from the description, or may be learned by practice of the embodiments disclosed herein. It is to be understood that both the foregoing brief summary and the following detailed description are exemplary and explanatory only and are not restrictive of the embodiments disclosed herein or as claimed.

### BRIEF DESCRIPTION OF THE DRAWINGS

To further clarify the above and other advantages and features of the present invention, a more particular description of the invention will be rendered by reference to specific embodiments thereof which are illustrated in the appended drawings. It is appreciated that these drawings depict only illustrated embodiments of the invention and are therefore not to be considered limiting of its scope. The invention will be described and explained with additional specificity and detail through the use of the accompanying drawings in which:
Figures 1-6 illustrate x-ray powder diffraction (XRPD) spectrum of various CSA salt compounds according to the present disclosure;
Figure 7 illustrates a dynamic vapor sorption (DVS) isotherm plot of a CSA salt of the present disclosure;
Figure 8 illustrates an XRPD spectrum of a CSA salt embodiment after being subjected to a DVS analysis; and
Figure 9 illustrates an overlay of XRPD spectrums of a CSA salt composition embodiment showing results before and after DVS analysis of the salt composition.

### DETAILED DESCRIPTION

The embodiments disclosed herein will now be described by reference to some more detailed embodiments, with occasional reference to any applicable accompanying drawings. These embodiments may, however, be embodied in different forms and should not be construed as limited to the embodiments set forth herein. Rather, these embodiments are provided so that this disclosure will be thorough and complete, and will fully convey the scope of the embodiments to those skilled in the art.

### Definitions

Unless otherwise defined, all technical and scientific terms used herein have the same meaning as commonly understood by one of ordinary skill in the art to which these embodiments belong. The terminology used in the description herein is for describing particular embodiments only and is not intended to be limiting of the embodiments. As used in the specification and the appended claims, the singular forms "a," "an," and "the" are intended to include the plural forms as well, unless the context clearly indicates otherwise.

Terms and phrases used in this application, and variations thereof, especially in the appended claims, unless otherwise expressly stated, should be construed as open ended as opposed to limiting. As examples of the foregoing, the term "including" should be read to mean "including, without limitation," "including but not limited to," or the like; the term "comprising" as used herein is synonymous with "including," "containing," or "characterized by," and is inclusive or open-ended and does not exclude additional, unrecited elements or method steps; the term "having" should be interpreted as "having at least"; the term "includes" should be interpreted as "includes but is not limited to"; the term "example" is used to provide exemplary instances of the item in discussion, not an exhaustive or limiting list thereof; and use of terms like "preferably," "preferred," "desired," or "desirable," and words of similar meaning should not be understood as implying that certain features are critical, essential, or even important to the structure or function of the invention, but instead as merely intended to highlight alternative or additional features that may or may not be utilized in a particular embodiment. In addition, the term "comprising" is to be interpreted synonymously with the phrases "having at least" or "including at least". When used in the context of a process, the term "comprising" means that the process includes at least the recited steps, but may include additional steps. When used in the context of a compound, composition or device, the term "comprising" means that the compound, composition or device includes at least the recited features or components, but may also include additional features or components. Likewise, a group of items linked with the conjunction "and" should not be read as requiring that each and every one of those items be present in the grouping, but rather should be read as "and/or" unless expressly stated otherwise. Similarly, a group of items linked with the conjunction "or" should not be read as requiring mutual exclusivity among that group, but rather should be read as "and/or" unless expressly stated otherwise.

It is understood that, in any compound described herein having one or more chiral centers, if an absolute stereochemistry is not expressly indicated, then each center may independently be of R-configuration or S-configuration or a mixture thereof. Thus, the compounds provided herein may be enantiomerically pure, enantiomerically enriched, racemic mixture, diastereomerically pure, diastereomerically enriched, or a stereoisomeric mixture. In addition it is understood that, in any compound described herein having one or more double bond(s) generating geometrical isomers that can be defined as E or Z, each double bond may independently be E or Z a mixture thereof.

Likewise, it is understood that, in any compound described, all tautomeric forms are also intended to be included.

It is to be understood that where compounds disclosed herein have unfilled valencies, then the valencies are to be filled with hydrogens or isotopes thereof, e.g., hydrogen-1 (protium) and hydrogen-2 (deuterium).

It is understood that the compounds described herein can be labeled isotopically. Substitution with isotopes such as deuterium may afford certain therapeutic advantages resulting from greater metabolic stability, such as, for example, increased *in vivo* half-life or reduced dosage requirements. Each chemical element as represented in a compound structure may include any isotope of said element. For example, in a compound structure a hydrogen atom may be explicitly disclosed or understood to be present in the compound. At any position of the compound that a hydrogen atom may be present, the hydrogen atom can be any isotope of hydrogen, including but not limited to hydrogen-1 (protium) and hydrogen-2 (deuterium). Thus, reference herein to a compound encompasses all potential isotopic forms unless the context clearly dictates otherwise.

Unless otherwise indicated, all numbers expressing quantities of ingredients, reaction conditions, and so forth used in the specification and claims are to be understood as being modified in all instances by the term "about." Accordingly, unless indicated to the contrary, the numerical parameters set forth in the specification and attached claims are approximations that may vary depending upon the desired properties sought to be obtained by the present embodiments. At the very least, and not as an attempt to limit the application of the doctrine of equivalents to the scope of the claims, each numerical parameter should be construed in light of the number of significant digits and ordinary rounding approaches.

Notwithstanding that the numerical ranges and parameters setting forth the broad scope of the embodiments are approximations, the numerical values set forth in the specific examples are reported as precisely as possible. Any numerical value, however, inherently contains certain errors necessarily resulting from the standard deviation found in their respective testing measurements. Every numerical range given throughout this specification and claims will include every narrower numerical range that falls within such broader numerical range, as if such narrower numerical ranges were all expressly written herein. Where a range of values is provided, it is understood that the upper and lower limit, and each intervening value between the upper and lower limit of the range is encompassed within the embodiments.

As used herein, any "R" group(s) such as, without limitation, R₁, R₂, R₃, R₄, R₅, R₆, R₇, R₈, R₉, R₁₀, R₁₁, R₁₂, R₁₃, R₁₄, R₁₅, R₁₆, R₁₇, and R₁₈ represent substituents that can be attached to the indicated atom. Unless otherwise specified, an R group may be substituted or unsubstituted.

A "ring" as used herein can be heterocyclic or carbocyclic. The term "saturated" used herein refers to a ring having each atom in the ring either hydrogenated or substituted such that the valency of each atom is filled. The term "unsaturated" used herein refers to a ring where the valency of each atom of the ring may not be filled with hydrogen or other substituents. For example, adjacent carbon atoms in the fused ring can be doubly bound to each other. Unsaturation can also include deleting at least one of the following pairs and completing the valency of the ring carbon atoms at these deleted positions with a double bond, such as R₅ and R₉; R₈ and R₁₀; and R₁₃ and R₁₄.

Whenever a group is described as being "substituted" that group may be substituted with one, two, three or more of the indicated substituents, which may be the same or different, each replacing a hydrogen atom. If no substituents are indicated, it is meant that the indicated "substituted" group may be substituted with one or more group(s) individually and independently selected from alkyl, alkenyl, alkynyl, cycloalkyl, cycloalkenyl, cycloalkynyl, acylalkyl, alkoxyalkyl, aminoalkyl, amino acid, aryl, heteroaryl, heteroalicyclyl, aralkyl, heteroaralkyl, (heteroalicyclyl)alkyl, hydroxy, protected hydroxyl, alkoxy, aryloxy, acyl, mercapto, alkylthio, arylthio, cyano, halogen (e.g., F, Cl, Br, and I), thiocarbonyl, O-carbamyl, N-carbamyl, O-thiocarbamyl, N-thiocarbamyl, C-amido, N-amido, S-sulfonamido, N-sulfonamido, C-carboxy, protected C-carboxy, O-carboxy, isocyanato, thiocyanato, isothiocyanato, nitro, oxo, silyl, sulfenyl, sulfinyl, sulfonyl, haloalkyl, haloalkoxy, trihalomethanesulfonyl, trihalomethanesulfonamido, an amino, a mono-substituted amino group and a di-substituted amino group, RₐO(CH₂)ₘO-, R_{b}(CH₂)ₙO-, R_{c}(O)O(CH₂)ₚO-, and protected derivatives thereof. The substituent may be attached to the group at more than one attachment point. For example, an aryl group may be substituted with a heteroaryl group at two attachment points to form a fused multicyclic aromatic ring system. Biphenyl and naphthalene are two examples of an aryl group that is substituted with a second aryl group. A group that is not specifically labeled as substituted or unsubstituted may be considered to be either substituted or unsubstituted.

As used herein, "Cₐ" or "Cₐ to C_{b}" in which "a" and "b" are integers refer to the number of carbon atoms in an alkyl, alkenyl or alkynyl group, or the number of carbon atoms in the ring of a cycloalkyl, cycloalkenyl, cycloalkynyl, aryl, heteroaryl or heteroalicyclyl group. That is, the alkyl, alkenyl, alkynyl, ring of the cycloalkyl, ring of the cycloalkenyl, ring of the cycloalkynyl, ring of the aryl, ring of the heteroaryl or ring of the heteroalicyclyl can contain from "a" to "b", inclusive, carbon atoms. Thus, for example, a "C₁ to C₄ alkyl" group refers to all alkyl groups having from 1 to 4 carbons, that is, CH₃-, CH₃CH₂-, CH₃CH₂CH₂-, (CH₃)₂CH-, CH₃CH₂CH₂CH₂-, CH₃CH₂CH(CH₃)- and (CH₃)₃C-. If no "a" and "b" are designated with regard to an alkyl, alkenyl, alkynyl, cycloalkyl cycloalkenyl, cycloalkynyl, aryl, heteroaryl or heteroalicyclyl group, the broadest range described in these definitions is to be assumed.

As used herein, "alkyl" refers to a straight or branched hydrocarbon chain that comprises a fully saturated (no double or triple bonds) hydrocarbon group. The alkyl group may have 1 to 25 carbon atoms (whenever it appears herein, a numerical range such as "1 to 25" refers to each integer in the given range; *e.g.,* "1 to 25 carbon atoms" means that the alkyl group may consist of 1 carbon atom, 2 carbon atoms, 3 carbon atoms, *etc.,* up to and including 25 carbon atoms, although the present definition also covers the occurrence of the term "alkyl" where no numerical range is designated). The alkyl group may also be a medium size alkyl having 1 to 15 carbon atoms. The alkyl group could also be a lower alkyl having 1 to 6 carbon atoms. The alkyl group of the compounds may be designated as "C₄" or "C₁-C₄ alkyl" or similar designations. By way of example only, "C₁-C₄ alkyl" indicates that there are one to four carbon atoms in the alkyl chain, i.e., the alkyl chain is selected from methyl, ethyl, propyl, iso-propyl, n-butyl, iso-butyl, sec-butyl, and t-butyl. Typical alkyl groups include, but are in no way limited to, methyl, ethyl, propyl, isopropyl, butyl, isobutyl, tertiary butyl, pentyl and hexyl. The alkyl group may be substituted or unsubstituted.

As used herein, "alkenyl" refers to an alkyl group that contains in the straight or branched hydrocarbon chain one or more double bonds. The alkenyl group may have 2 to 25 carbon atoms (whenever it appears herein, a numerical range such as "2 to 25" refers to each integer in the given range; *e.g.,* "2 to 25 carbon atoms" means that the alkenyl group may consist of 2 carbon atom, 3 carbon atoms, 4 carbon atoms, *etc.,* up to and including 25 carbon atoms, although the present definition also covers the occurrence of the term "alkenyl" where no numerical range is designated). The alkenyl group may also be a medium size alkenyl having 2 to 15 carbon atoms. The alkenyl group could also be a lower alkenyl having 1 to 6 carbon atoms. The alkenyl group of the compounds may be designated as "C₄" or "C₂-C₄ alkyl" or similar designations. An alkenyl group may be unsubstituted or substituted.

As used herein, "alkynyl" refers to an alkyl group that contains in the straight or branched hydrocarbon chain one or more triple bonds. The alkynyl group may have 2 to 25 carbon atoms (whenever it appears herein, a numerical range such as "2 to 25" refers to each integer in the given range; *e.g.,* "2 to 25 carbon atoms" means that the alkynyl group may consist of 2 carbon atom, 3 carbon atoms, 4 carbon atoms, *etc.,* up to and including 25 carbon atoms, although the present definition also covers the occurrence of the term "alkynyl" where no numerical range is designated). The alkynyl group may also be a medium size alkynyl having 2 to 15 carbon atoms. The alkynyl group could also be a lower alkynyl having 2 to 6 carbon atoms. The alkynyl group of the compounds may be designated as "C₄" or "C₂-C₄ alkyl" or similar designations. An alkynyl group may be unsubstituted or substituted.

As used herein, "aryl" refers to a carbocyclic (all carbon) monocyclic or multicyclic aromatic ring system (including fused ring systems where two carbocyclic rings share a chemical bond) that has a fully delocalized pi-electron system throughout all the rings. The number of carbon atoms in an aryl group can vary. For example, the aryl group can be a C₆-C₁₄ aryl group, a C₆-C₁₀ aryl group, or a C₆ aryl group (although the definition of C₆-C₁₀ aryl covers the occurrence of "aryl" when no numerical range is designated). Examples of aryl groups include, but are not limited to, benzene, naphthalene and azulene. An aryl group may be substituted or unsubstituted.

As used herein, "aralkyl" and "aryl(alkyl)" refer to an aryl group connected, as a substituent, via a lower alkylene group. The aralkyl group may have 6 to 20 carbon atoms (whenever it appears herein, a numerical range such as "6 to 20" refers to each integer in the given range; *e.g.,* "6 to 20 carbon atoms" means that the aralkyl group may consist of 6 carbon atom, 7 carbon atoms, 8 carbon atoms, *etc.,* up to and including 20 carbon atoms, although the present definition also covers the occurrence of the term "aralkyl" where no numerical range is designated). The lower alkylene and aryl group of an aralkyl may be substituted or unsubstituted. Examples include but are not limited to benzyl, 2-phenylalkyl, 3-phenylalkyl, and naphthylalkyl.

"Lower alkylene groups" refer to a C₁-C₂₅ straight-chained alkyl tethering groups, such as -CH₂- tethering groups, forming bonds to connect molecular fragments via their terminal carbon atoms. Examples include but are not limited to methylene (-CH₂-), ethylene (-CH₂CH₂-), propylene (-CH₂CH₂CH₂-), and butylene (-CH₂CH₂CH₂CH₂-). A lower alkylene group can be substituted by replacing one or more hydrogen of the lower alkylene group with a substituent(s) listed under the definition of "substituted."

As used herein, "cycloalkyl" refers to a completely saturated (no double or triple bonds) mono- or multi- cyclic hydrocarbon ring system. When composed of two or more rings, the rings may be joined together in a fused fashion. Cycloalkyl groups can contain 3 to 10 atoms in the ring(s) or 3 to 8 atoms in the ring(s). A cycloalkyl group may be unsubstituted or substituted. Typical cycloalkyl groups include, but are in no way limited to, cyclopropyl, cyclobutyl, cyclopentyl, cyclohexyl, cycloheptyl and cyclooctyl.

As used herein, "cycloalkenyl" refers to a mono- or multi- cyclic hydrocarbon ring system that contains one or more double bonds in at least one ring; although, if there is more than one, the double bonds cannot form a fully delocalized pi-electron system throughout all the rings (otherwise the group would be "aryl," as defined herein). When composed of two or more rings, the rings may be connected together in a fused fashion. A cycloalkenyl group may be unsubstituted or substituted.

As used herein, "cycloalkynyl" refers to a mono- or multi- cyclic hydrocarbon ring system that contains one or more triple bonds in at least one ring. If there is more than one triple bond, the triple bonds cannot form a fully delocalized pi-electron system throughout all the rings. When composed of two or more rings, the rings may be joined together in a fused fashion. A cycloalkynyl group may be unsubstituted or substituted.

As used herein, "alkoxy" or "alkyloxy" refers to the formula -OR wherein R is an alkyl, an alkenyl, an alkynyl, a cycloalkyl, a cycloalkenyl or a cycloalkynyl as defined above. A non-limiting list of alkoxys are methoxy, ethoxy, n-propoxy, 1-methylethoxy (isopropoxy), n-butoxy, iso-butoxy, sec-butoxy and tert-butoxy. An alkoxy may be substituted or unsubstituted.

As used herein, "acyl" refers to a hydrogen, alkyl, alkenyl, alkynyl, aryl, or heteroaryl connected, as substituents, via a carbonyl group. Examples include formyl, acetyl, propanoyl, benzoyl, and acryl. An acyl may be substituted or unsubstituted.

As used herein, "alkoxyalkyl" or "alkyloxyalkyl" refers to an alkoxy group connected, as a substituent, via a lower alkylene group. Examples include alkyl-O-alkyl- and alkoxy-alkyl- with the terms alkyl and alkoxy defined herein.

As used herein, "hydroxyalkyl" refers to an alkyl group in which one or more of the hydrogen atoms are replaced by a hydroxy group. Exemplary hydroxyalkyl groups include but are not limited to, 2-hydroxyethyl, 3-hydroxypropyl, 2-hydroxypropyl, and 2,2-dihydroxyethyl. A hydroxyalkyl may be substituted or unsubstituted.

As used herein, "haloalkyl" refers to an alkyl group in which one or more of the hydrogen atoms are replaced by a halogen (e.g., mono-haloalkyl, di-haloalkyl and trihaloalkyl). Such groups include but are not limited to, chloromethyl, fluoromethyl, difluoromethyl, trifluoromethyl and 1-chloro-2-fluoromethyl, 2-fluoroisobutyl. A haloalkyl may be substituted or unsubstituted.

The term "amino" as used herein refers to a -NH₂ group.

As used herein, the term "hydroxy" refers to a -OH group.

A "cyano" group refers to a "-CN" group.

A "carbonyl" or an "oxo" group refers to a C=O group.

The term "azido" as used herein refers to a -N₃ group.

As used herein, "aminoalkyl" refers to an amino group connected, as a substituent, via a lower alkylene group. Examples include H₂N-alkyl- with the term alkyl defined herein.

As used herein, "alkylcarboxyalkyl" refers to an alkyl group connected, as a substituent, to a carboxy group that is connected, as a substituent, to an alkyl group. Examples include alkyl-C(=O)O-alkyl- and alkyl-O-C(=O)-alkyl- with the term alkyl as defined herein.

As used herein, "alkylaminoalkyl" refers to an alkyl group connected, as a substituent, to an amino group that is connected, as a substituent, to an alkyl group. Examples include alkyl-NH-alkyl-, with the term alkyl as defined herein.

As used herein, "dialkylaminoalkyl" or "di(alkyl)aminoalkyl" refers to two alkyl groups connected, each as a substituent, to an amino group that is connected, as a substituent, to an alkyl group. Examples include 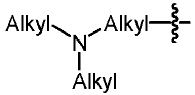 with the term alkyl as defined herein.

As used herein, "alkylaminoalkylamino" refers to an alkyl group connected, as a substituent, to an amino group that is connected, as a substituent, to an alkyl group that is connected, as a substituent, to an amino group. Examples include alkyl-NH-alkyl-NH-, with the term alkyl as defined herein.

As used herein, "alkylaminoalkylaminoalkylamino" refers to an alkyl group connected, as a substituent, to an amino group that is connected, as a substituent, to an alkyl group that is connected, as a substituent, to an amino group that is connected, as a substituent, to an alkyl group. Examples include alkyl-NH-alkyl-NH-alkyl-, with the term alkyl as defined herein.

As used herein, "arylaminoalkyl" refers to an aryl group connected, as a substituent, to an amino group that is connected, as a substituent, to an alkyl group. Examples include aryl-NH-alkyl-, with the terms aryl and alkyl as defined herein.

As used herein, "aminoalkyloxy" refers to an amino group connected, as a substituent, to an alkyloxy group. Examples include H₂N-alkyl-O- and H₂N-alkoxy- with the terms alkyl and alkoxy as defined herein.

As used herein, "aminoalkyloxyalkyl" refers to an amino group connected, as a substituent, to an alkyloxy group connected, as a substituent, to an alkyl group. Examples include H₂N-alkyl-O-alkyl- and H₂N-alkoxy-alkyl- with the terms alkyl and alkoxy as defined herein.

As used herein, "aminoalkylcarboxy" refers to an amino group connected, as a substituent, to an alkyl group connected, as a substituent, to a carboxy group. Examples include H₂N-alkyl-C(=O)O- and H₂N-alkyl-O-C(=O)- with the term alkyl as defined herein.

As used herein, "aminoalkylaminocarbonyl" refers to an amino group connected, as a substituent, to an alkyl group connected, as a substituent, to an amino group connected, as a substituent, to a carbonyl group. Examples include H₂N-alkyl-NH-C(=O)- with the term alkyl as defined herein.

As used herein, "aminoalkylcarboxamido" refers to an amino group connected, as a substituent, to an alkyl group connected, as a substituent, to a carbonyl group connected, as a substituent to an amino group. Examples include H₂N-alkyl-C(=O)-NH- with the term alkyl as defined herein.

As used herein, "azidoalkyloxy" refers to an azido group connected as a substituent, to an alkyloxy group. Examples include N₃-alkyl-O- and N₃-alkoxy- with the terms alkyl and alkoxy as defined herein.

As used herein, "cyanoalkyloxy" refers to a cyano group connected as a substituent, to an alkyloxy group. Examples include NC-alkyl-O- and NC-alkoxy- with the terms alkyl and alkoxy as defined herein.

A "sulfenyl" group refers to an "-SR" group in which R can be hydrogen, alkyl, alkenyl, alkynyl, cycloalkyl, cycloalkenyl, cycloalkynyl, aryl, heteroaryl, heteroalicyclyl, aralkyl, or (heteroalicyclyl)alkyl. A sulfenyl may be substituted or unsubstituted.

A "sulfinyl" group refers to an "-S(=O)-R" group in which R can be the same as defined with respect to sulfenyl. A sulfinyl may be substituted or unsubstituted.

A "sulfonyl" group refers to an "SO₂R" group in which R can be the same as defined with respect to sulfenyl. A sulfonyl may be substituted or unsubstituted.

An "O-carboxy" group refers to a "RC(=O)O-" group in which R can be hydrogen, alkyl, alkenyl, alkynyl, cycloalkyl, cycloalkenyl, cycloalkynyl, aryl, heteroaryl, heteroalicyclyl, aralkyl, or (heteroalicyclyl)alkyl, as defined herein. An O-carboxy may be substituted or unsubstituted.

The terms "ester" and "C-carboxy" refer to a "-C(=O)OR" group in which R can be the same as defined with respect to O-carboxy. An ester and C-carboxy may be substituted or unsubstituted.

A "thiocarbonyl" group refers to a "-C(=S)R" group in which R can be the same as defined with respect to O-carboxy. A thiocarbonyl may be substituted or unsubstituted.

A "trihalomethanesulfonyl" group refers to an "X₃CSO₂-" group wherein X is a halogen.

An "S-sulfonamido" group refers to a "-SO₂N(RARB)" group in which RA and RB can be independently hydrogen, alkyl, alkenyl, alkynyl, cycloalkyl, cycloalkenyl, cycloalkynyl, aryl, heteroaryl, heteroalicyclyl, aralkyl, or (heteroalicyclyl)alkyl. An S-sulfonamido may be substituted or unsubstituted.

An "N-sulfonamido" group refers to a "RSO₂N(RA)-" group in which R and RA can be independently hydrogen, alkyl, alkenyl, alkynyl, cycloalkyl, cycloalkenyl, cycloalkynyl, aryl, heteroaryl, heteroalicyclyl, aralkyl, or (heteroalicyclyl)alkyl. An N-sulfonamido may be substituted or unsubstituted.

An "O-carbamyl" group refers to a "-OC(=O)N(RARB)" group in which RA and RB can be independently hydrogen, alkyl, alkenyl, alkynyl, cycloalkyl, cycloalkenyl, cycloalkynyl, aryl, heteroaryl, heteroalicyclyl, aralkyl, or (heteroalicyclyl)alkyl. An O-carbamyl may be substituted or unsubstituted.

An "N-carbamyl" group refers to an "ROC(=O)N(RA)-" group in which R and RA can be independently hydrogen, alkyl, alkenyl, alkynyl, cycloalkyl, cycloalkenyl, cycloalkynyl, aryl, heteroaryl, heteroalicyclyl, aralkyl, or (heteroalicyclyl)alkyl. An N-carbamyl may be substituted or unsubstituted.

An "O-thiocarbamyl" group refers to a "-OC(=S)-N(RARB)" group in which RA and RB can be independently hydrogen, alkyl, alkenyl, alkynyl, cycloalkyl, cycloalkenyl, cycloalkynyl, aryl, heteroaryl, heteroalicyclyl, aralkyl, or (heteroalicyclyl)alkyl. An O-thiocarbamyl may be substituted or unsubstituted.

An "N-thiocarbamyl" group refers to an "ROC(=S)N(RA)-" group in which R and RA can be independently hydrogen, alkyl, alkenyl, alkynyl, cycloalkyl, cycloalkenyl, cycloalkynyl, aryl, heteroaryl, heteroalicyclyl, aralkyl, or (heteroalicyclyl)alkyl. An N-thiocarbamyl may be substituted or unsubstituted.

A "C-amido" group refers to a "-C(=O)N(RARB)" group in which RA and RB can be independently hydrogen, alkyl, alkenyl, alkynyl, cycloalkyl, cycloalkenyl, cycloalkynyl, aryl, heteroaryl, heteroalicyclyl, aralkyl, or (heteroalicyclyl)alkyl. A C-amido may be substituted or unsubstituted.

An "N-amido" group refers to a "RC(=O)N(RA)-" group in which R and RA can be independently hydrogen, alkyl, alkenyl, alkynyl, cycloalkyl, cycloalkenyl, cycloalkynyl, aryl, heteroaryl, heteroalicyclyl, aralkyl, or (heteroalicyclyl)alkyl. An N-amido may be substituted or unsubstituted.

As used herein, "guanidinoalkyloxy" refers to a guanidinyl group connected, as a substituent, to an alkyloxy group. Examples include 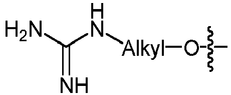 and 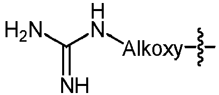 with the terms alkyl and alkoxy as defined herein.

As used herein, "guanidinoalkylcarboxy" refers to a guanidinyl group connected, as a substituent, to an alkyl group connected, as a substituent, to a carboxy group. Examples include 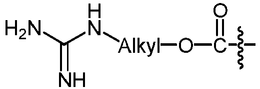 and 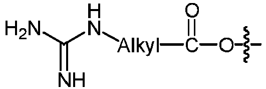 with the term alkyl as defined herein.

As used herein, "quaternary ammonium alkylcarboxy" refers to a quaternized amino group connected, as a substituent, to an alkyl group connected, as a substituent, to a carboxy group. Examples include 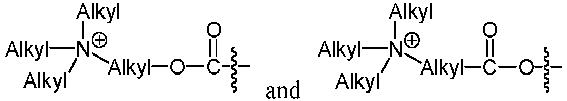 with the term alkyl as defined herein.

The term "halogen atom" or "halogen" as used herein, means any one of the radio-stable atoms of column 7 of the Periodic Table of the Elements, such as, fluorine, chlorine, bromine and iodine.

Where the numbers of substituents is not specified (e.g. haloalkyl), there may be one or more substituents present. For example "haloalkyl" may include one or more of the same or different halogens.

As used herein, the term "amino acid" refers to any amino acid (both standard and non-standard amino acids), including, but not limited to, □-amino acids, □-amino acids, □-amino acids and □-amino acids. Examples of suitable amino acids include, but are not limited to, alanine, asparagine, aspartate, cysteine, glutamate, glutamine, glycine, proline, serine, tyrosine, arginine, histidine, isoleucine, leucine, lysine, methionine, phenylalanine, threonine, tryptophan and valine. Additional examples of suitable amino acids include, but are not limited to, ornithine, hypusine, 2-aminoisobutyric acid, dehydroalanine, gamma-aminobutyric acid, citrulline, beta-alanine, alpha-ethyl-glycine, alpha-propyl-glycine and norleucine.

A linking group is a divalent moiety used to link one steroid to another steroid. In some embodiments, the linking group is used to link a first CSA with a second CSA (which may be the same or different). An example of a linking group is (C₁-C₁₀) alkyloxy-(Ci-Cio) alkyl.

The terms "P.G." or "protecting group" or "protecting groups" as used herein refer to any atom or group of atoms that is added to a molecule in order to prevent existing groups in the molecule from undergoing unwanted chemical reactions. Examples of protecting group moieties are described in T. W. Greene and P. G. M. Wuts, Protective Groups in Organic Synthesis, 3. Ed. John Wiley & Sons, 1999, and in J.F.W. McOmie, Protective Groups in Organic Chemistry Plenum Press, 1973. The protecting group moiety may be chosen in such a way, that they are stable to certain reaction conditions and readily removed at a convenient stage using methodology known from the art. A non-limiting list of protecting groups include benzyl; substituted benzyl; alkylcarbonyls and alkoxycarbonyls (e.g., t-butoxycarbonyl (BOC), acetyl, or isobutyryl); arylalkylcarbonyls and arylalkoxycarbonyls (e.g., benzyloxycarbonyl); substituted methyl ether (e.g. methoxymethyl ether); substituted ethyl ether; a substituted benzyl ether; tetrahydropyranyl ether; silyls (e.g., trimethylsilyl, triethylsilyl, triisopropylsilyl, t-butyldimethylsilyl, tri-*iso-*propylsilyloxymethyl, [2-(trimethylsilyl)ethoxy]methyl or t-butyldiphenylsilyl); esters (e.g. benzoate ester); carbonates (e.g. methoxymethylcarbonate); sulfonates (e.g. tosylate or mesylate); acyclic ketal (e.g. dimethyl acetal); cyclic ketals (e.g., 1,3-dioxane, 1,3-dioxolanes, and those described herein); acyclic acetal; cyclic acetal (e.g., those described herein); acyclic hemiacetal; cyclic hemiacetal; cyclic dithioketals (e.g., 1,3-dithiane or 1,3-dithiolane); orthoesters (e.g., those described herein) and triarylmethyl groups (e.g., trityl; monomethoxytrityl (MMTr); 4,4'-dimethoxytrityl (DMTr); 4,4',4"-trimethoxytrityl (TMTr); and those described herein). Amino-protecting groups are known to those skilled in the art. In general, the species of protecting group is not critical, provided that it is stable to the conditions of any subsequent reaction(s) on other positions of the compound and can be removed at the appropriate point without adversely affecting the remainder of the molecule. In addition, a protecting group may be substituted for another after substantive synthetic transformations are complete. Clearly, where a compound differs from a compound disclosed herein only in that one or more protecting groups of the disclosed compound has been substituted with a different protecting group, that compound is within the disclosure.

### CSA Compounds

Cationic steroidal anti-microbial (CSA) compounds, sometimes referred to as "CSA compounds" or "ceragenin" compounds, are synthetically produced, small molecule chemical compounds that include a sterol backbone having various charged groups (e.g., amine and cationic groups) attached to the backbone. The sterol backbone can be used to orient amine or guanidine groups on a face or plane of the sterol backbone. CSAs are cationic and amphiphilic, based upon the functional groups attached to the backbone. They are facially amphiphilic with a hydrophobic face and a polycationic face.

Without wishing to be bound to theory, the CSA molecules described herein act as anti-microbial agents (e.g., anti-bacterial, anti-fungal, and anti-viral). It is believed, for example, that anti-microbial CSA molecules may act as an anti-microbial by binding to the cellular membrane of bacteria and other microbes and modifying the cell membrane, e.g., such as by forming a pore that allows the leakage of ions and cytoplasmic materials critical to the microbe's survival, and leading to the death of the affected microbe. In addition, anti-microbial CSA molecules may also act to sensitize bacteria to other antibiotics. For example, at concentrations of anti-microbial CSA molecules below the corresponding minimum bacteriostatic concentration (MIC), the CSA compound may cause bacteria to become more susceptible to other antibiotics by disrupting the cell membrane, such as by increasing membrane permeability. It is postulated that charged cationic groups may be responsible for disrupting the bacterial cellular membrane and imparting anti-microbial properties. CSA molecules may have similar membrane- or outer coating-disrupting effects on fungi and viruses.

Compounds useful in accordance with this disclosure are described herein, both generically and with particularity, and in U.S. Patent Nos. 6,350,738, 6,486,148, 6,767,904, 7,598,234, 7,754,705, U.S. Application Nos. 61/786301, 13/288892, 61/642431, 13/554930, 61/572714, 13/594608, 61/576903, 13/594612, 13/288902, 61/605639, 13/783131, 61/605642, 13/783007, 61/132361, 13/000010, 61/534185, 13/615244, 61/534194, 13/615324, 61534205, 61/637402, 13/841549, 61/715277, PCT/US13/37615, 61/749800, 61/794721, and 61/814816. The skilled artisan will recognize the compounds within the generic formula set forth herein and understand their preparation in view of the references cited herein and the Examples.

CSA compounds can be a compound of Formula (I), Formula (II), or salt thereof, having a steroidal backbone:

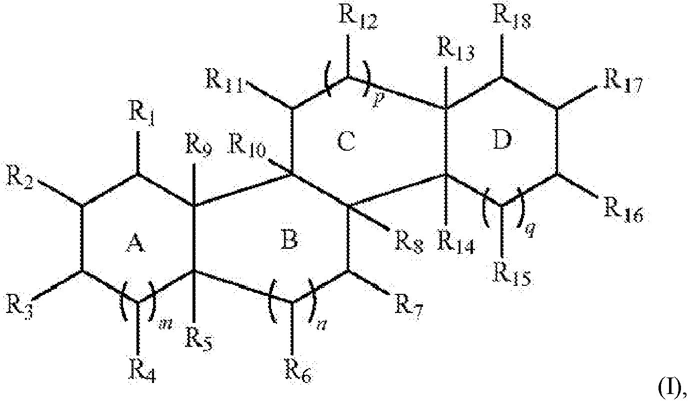

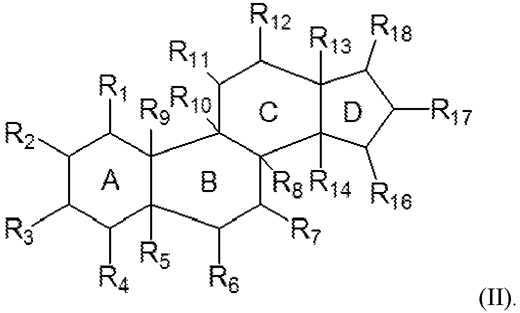

CSA compounds of Formula (I), Formula (II), and salts thereof can be characterized wherein:
rings A, B, C, and D are independently saturated, or are fully or partially unsaturated, provided that at least two of rings A, B, C, and D are saturated;
m, n, p, and q are independently 0 or 1;
R₁ through R₄, R₆ , R₇ , R₁₁ , R₁₂, R₁₅, R₁₆, and R₁₈ are independently selected from the group consisting of hydrogen, hydroxyl, alkyl, hydroxyalkyl, alkyloxyalkyl, alkylcarboxyalkyl, alkylaminoalkyl, alkylaminoalkylamino, alkylaminoalkylaminoalkylamino, aminoalkyl, aryl, arylaminoalkyl, haloalkyl, alkenyl, alkynyl, oxo, a linking group attached to a second steroid, aminoalkyloxy, aminoalkyloxyalkyl, aminoalkylcarboxy, aminoalkylaminocarbonyl,aminoalkylcarboxamido, di(alkyl)aminoalkyl, H₂N-HC(Q₅)-C(O)-O-, H₂N-HC(Q₅)-C(O)-N(H)-, azidoalkyloxy, cyanoalkyloxy, P.G.-HN-HC(Q₅)-C(O)-O-, guanidinoalkyloxy, quaternary ammonium alkylcarboxy, and guanidinoalkyl carboxy, where Q₅ is a side chain of any amino acid (including a side chain of glycine, i.e., H), and P.G. is an amino protecting group; and
R₅, R₈, R₉, R₁₀, R₁₃, R₁₄ and R₁₇ are independently deleted when one of rings A, B, C, or D is unsaturated so as to complete the valency of the carbon atom at that site, or R₅, R₈, R₉, R₁₀, R₁₃, and R₁₄ are independently selected from the group consisting of hydrogen, hydroxyl, alkyl, hydroxyalkyl, alkyloxyalkyl, aminoalkyl, aryl, haloalkyl, alkenyl, alkynyl, oxo, a linking group attached to a second steroid, aminoalkyloxy, aminoalkylcarboxy, aminoalkylaminocarbonyl, di(alkyl)aminoalkyl, H₂N-HC(Q₅)-C(O)-O-, H₂N-HC(Q₅)-C(O)-N(H)-, azidoalkyloxy, cyanoalkyloxy, P.G.-HN-HC(Q₅)-C(O)-O-, guanidinoalkyloxy, and guanidinoalkyl-carboxy, where Q₅ is a side chain of any amino acid, P.G. is an amino protecting group.

According to the invention there is provided a 1,5-naphthalenedisulfonic acid di-addition salt of a cationic steroidal antimicrobial (CSA), wherein the CSA compound is a compound of Formula (III), having a steroidal backbone: wherein 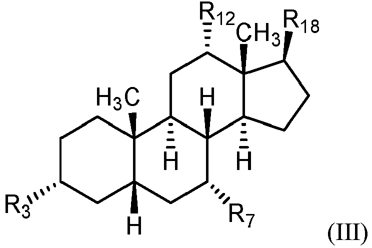 , R₃, R₇, R₁₂ and R₁₈ are as defined in claim 1, and wherein R₃, R₇, and R₁₂ are preferably independently selected from the group consisting of hydrogen, an unsubstituted (C₁-C₂₂) alkyl, unsubstituted (C₁-C₂₂) hydroxyalkyl, unsubstituted (C₁-C₂₂) alkyloxy-(C₁-C₂₂) alkyl, unsubstituted (C₁-C₂₂) alkylcarboxy-(C₁-C₂₂) alkyl, unsubstituted (C₁-C₂₂) alkylamino-(C₁-C₂₂)alkyl, unsubstituted (C₁-C₂₂) alkylamino-(C₁-C₂₂) alkylamino, unsubstituted (C₁-C₂₂) alkylamino-(C₁-C₂₂) alkylamino-(C₁-C₁₈) alkylamino, an unsubstituted (C₁-C₂₂) aminoalkyl, an unsubstituted arylamino-(C₁-C₂₂) alkyl, an unsubstituted (C₁-C₂₂) aminoalkyloxy, an unsubstituted (C₁-C₂₂) aminoalkyloxy-(C₁-C₂₂) alkyl, an unsubstituted (C₁-C₂₂) aminoalkylcarboxy, an unsubstituted (C₁-C₂₂) aminoalkylaminocarbonyl, an unsubstituted (C₁-C₂₂) aminoalkylcarboxamido, an unsubstituted di(C₁-C₂₂ alkyl)aminoalkyl, unsubstituted (C₁-C₂₂) guanidinoalkyloxy, unsubstituted (C₁-C₂₂) quaternary ammonium alkylcarboxy, and unsubstituted (C₁-C₂₂) guanidinoalkyl carboxy.

In some embodiments, R₃, R₇, and R₁₂ are independently selected from the group consisting of hydrogen, an unsubstituted (C₁-C₆) alkyl, unsubstituted (C₁-C₆) hydroxyalkyl, unsubstituted (C₁-C₁₆) alkyloxy-(C₁-C₅) alkyl, unsubstituted (C₁-C₁₆) alkylcarboxy-(C₁-C₅) alkyl, unsubstituted (C₁-C₁₆) alkylamino-(C₁-C₅)alkyl, unsubstituted (C₁-C₁₆) alkylamino-(C₁-C₅) alkylamino, unsubstituted (C₁-C₁₆) alkylamino-(C₁-C₁₆) alkylamino-(C₁-C₅) alkylamino, an unsubstituted (C₁-C₁₆) aminoalkyl, an unsubstituted arylamino-(C₁-C₅) alkyl, an unsubstituted (C₁-C₅) aminoalkyloxy, an unsubstituted (C₁-C₁₆) aminoalkyloxy-(C₁-C₅) alkyl, an unsubstituted (C₁-C₅) aminoalkylcarboxy, an unsubstituted (C₁-C₅) aminoalkylaminocarbonyl, an unsubstituted (C₁-C₅) aminoalkylcarboxamido, an unsubstituted di(C₁-C₅ alkyl)amino-(C₁-C₅) alkyl, unsubstituted (C₁-C₅) guanidinoalkyloxy, unsubstituted (C₁-C₁₆) quaternary ammonium alkylcarboxy, and unsubstituted (C₁-C₁₆) guanidinoalkylcarboxy.

In some embodiments, R₃, R₇, and R₁₂ are independently selected from the group consisting of aminoalkyloxy; aminoalkylcarboxy; alkylaminoalkyl; alkoxycarbonylalkyl; alkylcarbonylalkyl; di(alkyl)aminoalkyl; alkylcarboxyalkyl; and hydroxyalkyl.

In some embodiments, R₃, R₇, and R₁₂ are independently selected from the group consisting of aminoalkyloxy and aminoalkylcarboxy.

In some embodiments, R₃, R₇, and R₁₂ are the same. In some embodiments, R₃, R₇, and R₁₂ are aminoalkyloxy. In some embodiments, R₃, R₇, and R₁₂ are aminoalkylcarboxy.

In some embodiments, R₃, R₇, and R₁₂ are independently selected from the group consisting of amino-C₃-alkyloxy; amino-C₃-alkyl-carboxy; C₈-alkylamino-C₅-alkyl; C₈-alkoxy-carbonyl-C₄-alkyl; C₈-alkyl-carbonyl-C₄-alkyl; di-(C₅-alkyl)amino-C₅-alkyl; C₁₃-alkylamino-Cs-alkyl; C₆-alkoxy-carbonyl-C₄-alkyl; C₆-alkyl-carboxy-C₄-alkyl; and C₁₆-alkylamino-C₅-alkyl.

In some embodiments, CSA salt compounds of the present invention can be the 1,5-naphthalenedisulfonic acid di-addition salt of a compound of Formula (III), wherein at least R₁₈ of the steroidal backbone includes amide functionality in which the carbonyl group of the amide is positioned between the amido nitrogen of the amide and fused ring D of the steroidal backbone. For example, any of the embodiments described above can substitute R₁₈ for an R₁₈ including amide functionality in which the carbonyl group of the amide is positioned between the amido nitrogen of the amide and fused ring D of the steroidal backbone.

In some embodiments, at least R₁₈ can have the following structure:

-R₂₀-(C=O)-N-R₂₁R₂₂

wherein R₂₀ is omitted or alkyl, alkenyl, alkynyl, or aryl, and R₂₁ and R₂₂ are independently selected from the group consisting of hydrogen, alkyl, alkenyl, alkynyl, or aryl, provided that at least one of R₂₁ and R₂₂ is not hydrogen.

In some embodiments, R₂₁ and R₂₂ are independently selected from the group consisting of hydrogen, C₁-C₂₄ alkyl, C₂-C₂₄ alkenyl, C₂-C₂₄ alkynyl, C₆ or C₁₀ aryl, 5 to 10 membered heteroaryl, 5 to 10 membered heterocyclyl, C₇₋₁₃ aralkyl, (5 to 10 membered heteroaryl)-C₁-C₆ alkyl, C₃₋₁₀ carbocyclyl, C₄₋₁₀ (carbocyclyl)alkyl, (5 to 10 membered heterocyclyl)-C₁-C₆ alkyl, amido, and a suitable amine protecting group, provided that at least one of R₂₁ and R₂₂ is not hydrogen. In some embodiments, R₂₁ and R₂₂, together with the atoms to which they are attached, form a 5 to 10 membered heterocyclyl ring.

In some embodiments, the CSA is selected from the group consisting of: 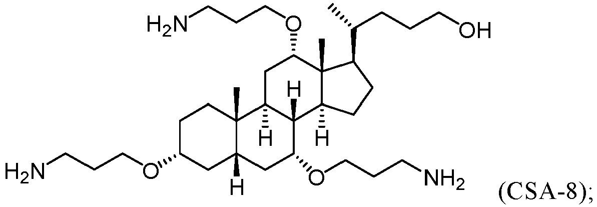 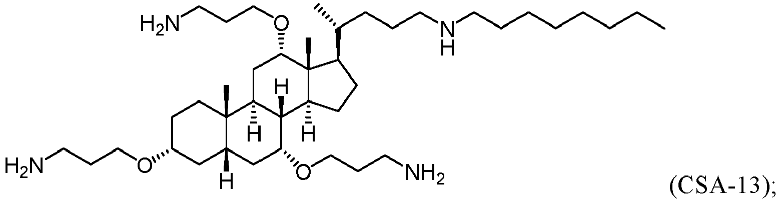 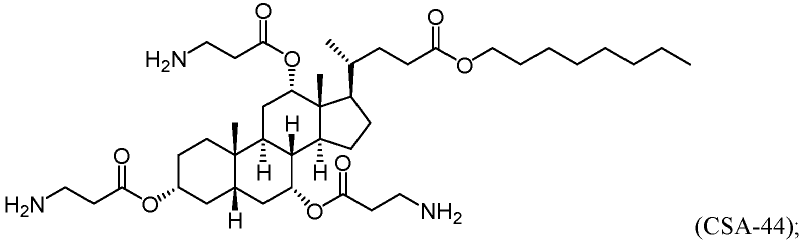 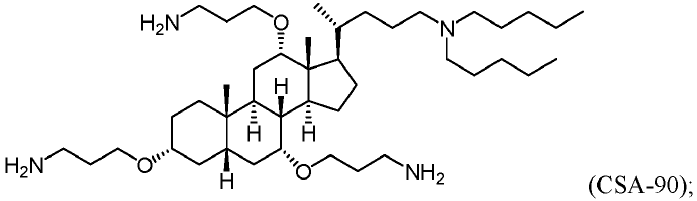 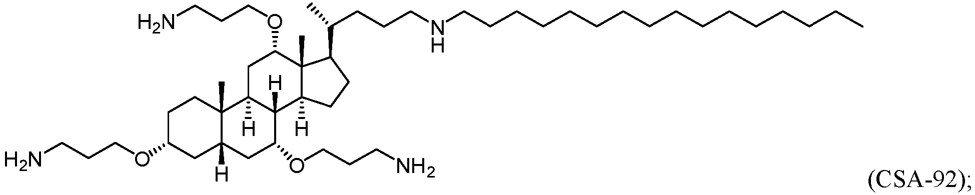 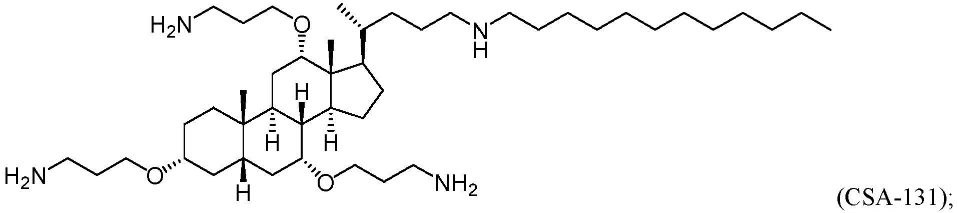 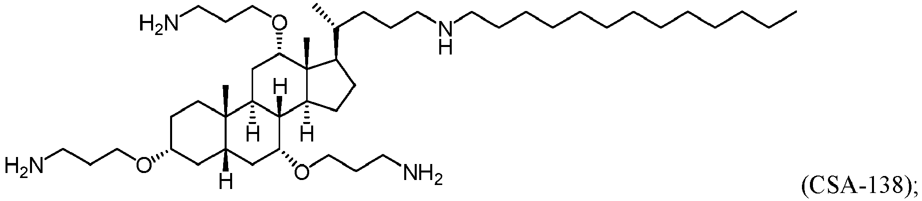 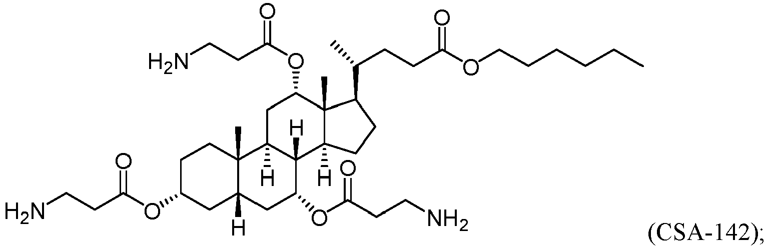 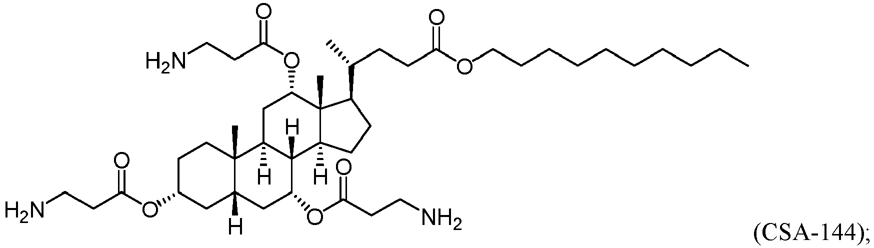 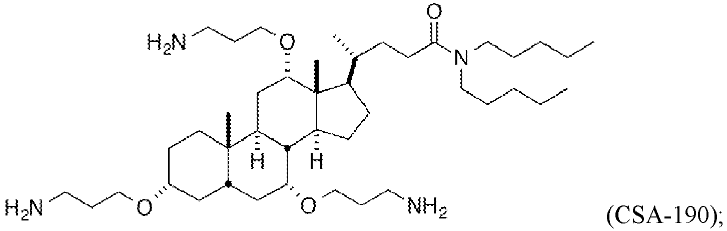 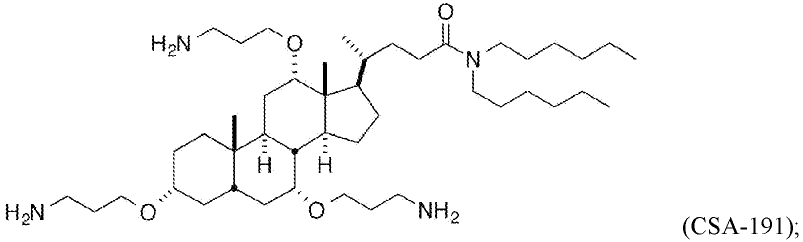 and

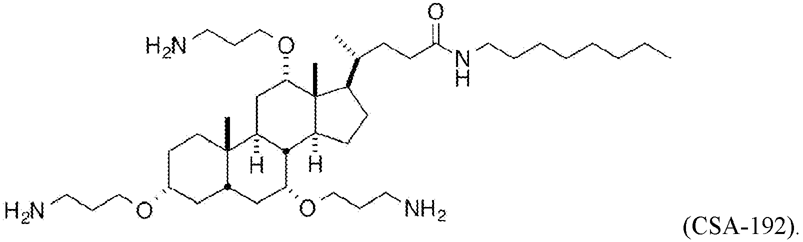

In some embodiments, the CSA is

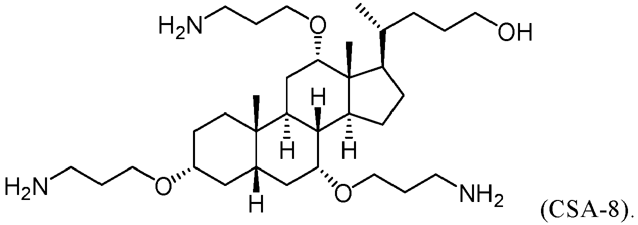

In some embodiments, the CSA is

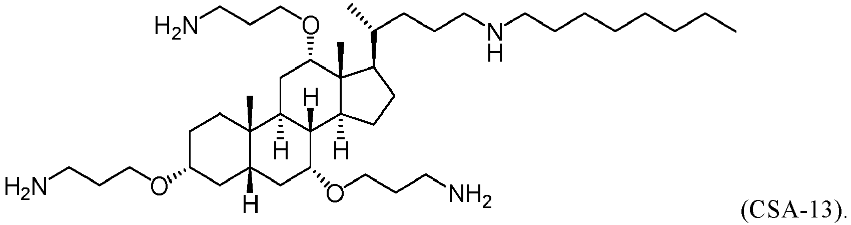

In some embodiments, the CSA is

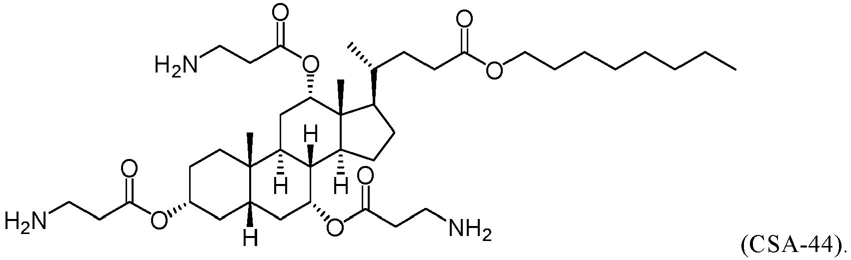

In some embodiments, the CSA is

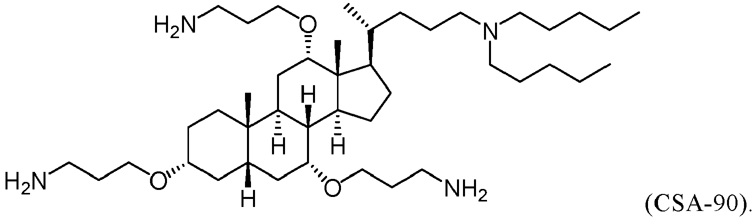

In some embodiments, the CSA is

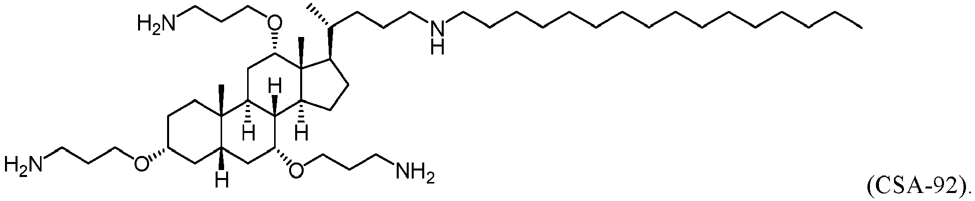

In some embodiments, the CSA is

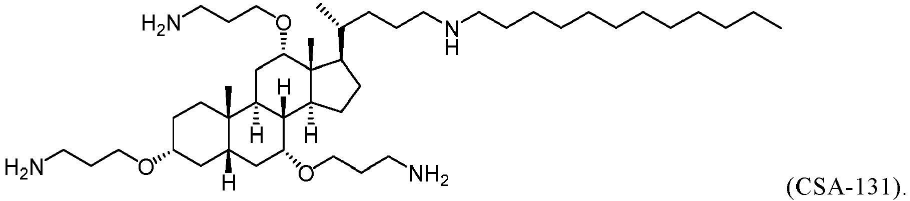

In some embodiments, the CSA is

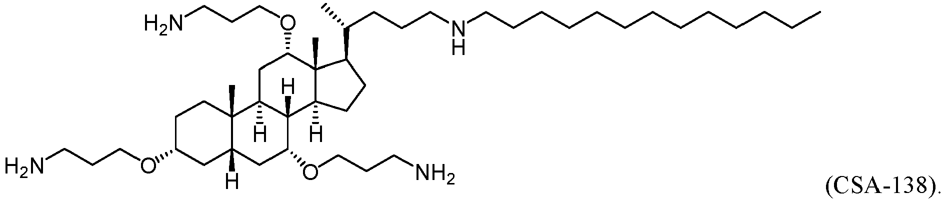

In some embodiments, the CSA is

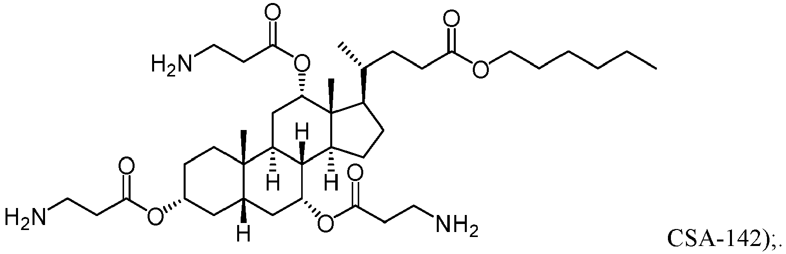

In some embodiments, the CSA is

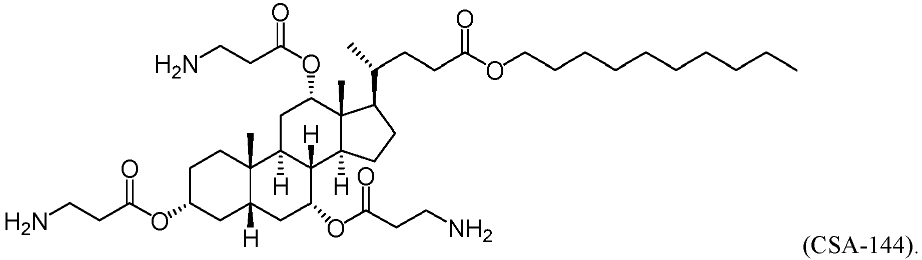

In some embodiments, the CSA is

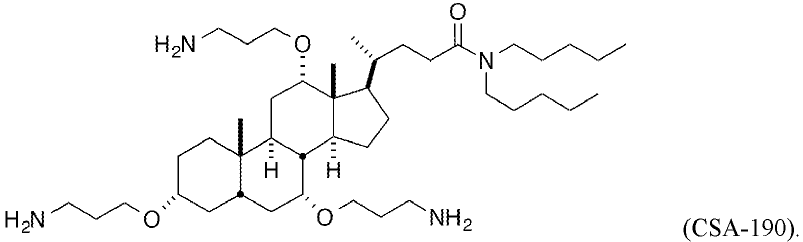

In some embodiments, the CSA is

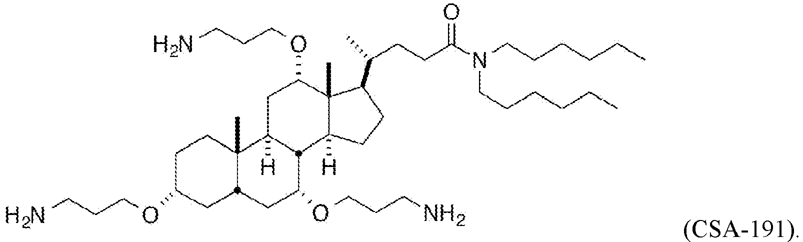

In some embodiments, the CSA is

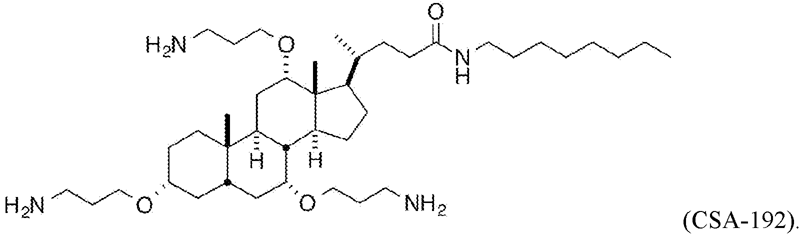

### CSA Salts

It has been discovered that the CSA salt form can be manipulated by the choice of counterion to afford CSA salts having pharmaceutically beneficial properties such as improved solubility, crystallinity, flow, and storage stability. Such properties are of critical concern for the handling and use of CSAs as pharmaceutical agents. For example, poor solubility can influence the ultimate formulation of a CSA, while storage stability can influence efficient manufacturing protocols and shelf life of the CSA formulation. Morever, crystallinity of the CSA can affect purification and significantly influence the synthesis and handling of the CSA during manufacturing. Likewise, the flow properties of a CSA can influence the the equipment and handling of a CSA during manufacturing. Thus, the ability to manipulate and control these properties through the selection of an appropriate counterion represents a significant step toward the commercialization of a CSA pharmaceutical product.

Described herein is a sulfuric acid addition salt or sulfonic acid addition salt of a CSA. Further described herein is the sulfonic acid addition salt which is a disulfonic acid addition salt. Also described herein is the sulfonic acid addition salt which is a 1,5-naphthalenedisulfonic acid addition salt. Also described herein is a compound wherein the acid addition salt is a mono-addition salt. According to the invention, the acid addition salt is a di-addition salt with 1,5-naphthalenedisulfonic acid. According to an additionally described feature, the acid addition salt is a tetra-addition salt.

In some embodiments, the acid addition salt described above is a solid.

In some embodiments, the acid addition salt described above is a flowable solid.

In some embodiments, the acid addition salt described above is crystalline.

In some embodiments, the acid addition salt described above is storage stable. In some embodiments, the acid addition salt is storage stable for a period of 5 days, 1 week, 2 weeks, 1 month, 3 months, 6 months, 1 year, or about any of the aforementioned numbers, or a range bounded by any two of the aforementioned numbers. In some embodiments, storage stability is measured by degradation that is less than 0.5%, 1%, 2%, 3%, 4%, 5%, 10% or about any of the aforementioned numbers, or a range bounded by any two of the aforementioned numbers for a given period of time, as described above. In some embodiments, storage stability is measured qualitatively by a change in crystallinity, such as loss of crystallinity and/or the concomitant increase in amorphous materials such as amorphous solids, gums, and the like, for a given period of time, as described above.

### CSA Salts Synthesis

Disclosed herein is a process for preparing a CSA acid addition salt, in which 1-4 equivalents of sulfuric acid or a sulfonic acid is contacted with a CSA. In some embodiments, the sulfonic acid addition salt is a disulfonic acid addition salt. In some embodiments, the sulfonic acid addition salt is a 1,5-naphthalenedisulfonic acid addition salt. In some embodiments, the acid addition salt is a mono-addition salt. In other embodiments, the acid addition salt is a di-addition salt. In other embodiments, the acid addition salt is a tetra-addition salt. In some embodiments, 1, 2, 3, or 4 equivalents of acid, or about any of the aforementioned numbers, or a range bounded by any of the aforementioned numbers is contacted with the CSA.

The process for preparing the above-described CSA salt includes diluting the free base of a CSA with a solvent; adding at least one equivalent of an acid to the diluted CSA in solvent to afford a reaction mixture; precipitating or temperature cycling the reaction mixture; and isolating a CSA salt. The CSA salt is precipitated. Alternatively, the CSA salt is isolated after temperature cycling. The temperature cycling may be conducted for at least about 1, 2, 3, 6, 8, 12, 16, 18, 20, 24, 36, or 48 hours, or a range bounded by any two of the aforementioned numbers. The CSA salt may be isolated after the addition of an anti-solvent. The CSA salt may be isolated after evaporation of solvent.

### Pharmaceutical Compositions

While it is possible for the compounds described herein to be administered alone, it may be preferable to formulate the compounds as pharmaceutical compositions (i.e., formulations). As such, in yet another aspect, pharmaceutical compositions useful in the methods and uses of the disclosed embodiments are provided. A pharmaceutical composition is any composition that may be administered in vitro or in vivo or both to a subject in order to treat or ameliorate a condition. In a preferred embodiment, a pharmaceutical composition may be administered in vivo. A subject may include one or more cells or tissues, or organisms. In some exemplary embodiments, the subject is an animal. In some embodiments, the animal is a mammal. The mammal may be a human or primate in some embodiments. A mammal includes any mammal, such as by way of non-limiting example, cattle, pigs, sheep, goats, horses, camels, buffalo, cats, dogs, rats, mice, and humans.

As used herein the terms "pharmaceutically acceptable" and "physiologically acceptable" mean a biologically compatible formulation, gaseous, liquid or solid, or mixture thereof, which is suitable for one or more routes of administration, in vivo delivery, or contact. A formulation is compatible in that it does not destroy activity of an active ingredient therein (e.g., a CSA compound), or induce adverse side effects that far outweigh any prophylactic or therapeutic effect or benefit.

In some embodiments, pharmaceutical compositions may be formulated with pharmaceutically acceptable excipients such as carriers, solvents, stabilizers, adjuvants, diluents, etc., depending upon the particular mode of administration and dosage form. The pharmaceutical compositions should generally be formulated to achieve a physiologically compatible pH, and may range from a pH of about 3 to a pH of about 11, preferably about pH 3 to about pH 7, depending on the formulation and route of administration. In alternative embodiments, it may be preferred that the pH is adjusted to a range from about pH 5.0 to about pH 8. More particularly, the pharmaceutical compositions may comprise a therapeutically or prophylactically effective amount of at least one compound as described herein, together with one or more pharmaceutically acceptable excipients. Optionally, the pharmaceutical compositions may comprise a combination of the compounds described herein, or may include a second active ingredient useful in the treatment or prevention of bacterial infection (e.g., anti-bacterial or anti-microbial agents). Optionally, the composition is formulated as a coating. In some embodiments, the coating is on a medical device. In some embodiments, the coating is on medical instrumentation.

Formulations, e.g., for parenteral or oral administration, are most typically solids, liquid solutions, emulsions or suspensions, while inhalable formulations for pulmonary administration are generally liquids or powders, with powder formulations being generally preferred. A preferred pharmaceutical composition may also be formulated as a lyophilized solid that is reconstituted with a physiologically compatible solvent prior to administration. Alternative pharmaceutical compositions may be formulated as syrups, creams, ointments, tablets, and the like.

Compositions may contain one or more excipients. Pharmaceutically acceptable excipients are determined in part by the particular composition being administered, as well as by the particular method used to administer the composition. Accordingly, there exists a wide variety of suitable formulations of pharmaceutical compositions (see, e.g., Remington's Pharmaceutical Sciences).

Suitable excipients may be carrier molecules that include large, slowly metabolized macromolecules such as proteins, polysaccharides, polylactic acids, polyglycolic acids, polymeric amino acids, amino acid copolymers, and inactive virus particles. Other exemplary excipients include antioxidants such as ascorbic acid; chelating agents such as EDTA; carbohydrates such as dextrin, hydroxyalkylcellulose, hydroxyalkylmethylcellulose, stearic acid; liquids such as oils, water, saline, glycerol and ethanol; wetting or emulsifying agents; pH buffering substances; and the like. Liposomes are also included within the definition of pharmaceutically acceptable excipients.

Pharmaceutical compositions may be formulated in any form suitable for the intended method of administration. When intended for oral use for example, tablets, troches, lozenges, aqueous or oil suspensions, non-aqueous solutions, dispersible powders or granules (including micronized particles or nanoparticles), emulsions, hard or soft capsules, syrups or elixirs may be prepared. Compositions intended for oral use may be prepared according to any method known to the art for the manufacture of pharmaceutical compositions, and such compositions may contain one or more agents including sweetening agents, flavoring agents, coloring agents and preserving agents, in order to provide a palatable preparation.

Pharmaceutically acceptable excipients particularly suitable for use in conjunction with tablets include, for example, inert diluents, such as celluloses, calcium or sodium carbonate, lactose, calcium or sodium phosphate; disintegrating agents, such as cross-linked povidone, maize starch, or alginic acid; binding agents, such as povidone, starch, gelatin or acacia; and lubricating agents, such as magnesium stearate, stearic acid or talc.

Tablets may be uncoated or may be coated by known techniques including microencapsulation to delay disintegration and adsorption in the gastrointestinal tract and thereby provide a sustained action over a longer period. For example, a time delay material such as glyceryl monostearate or glyceryl distearate alone or with a wax may be employed.

Formulations for oral use may be also presented as hard gelatin capsules where the active ingredient is mixed with an inert solid diluent, for example celluloses, lactose, calcium phosphate or kaolin, or as soft gelatin capsules wherein the active ingredient is mixed with non-aqueous or oil medium, such as glycerin, propylene glycol, polyethylene glycol, peanut oil, liquid paraffin or olive oil.

In another embodiment, pharmaceutical compositions may be formulated as suspensions comprising a compound of the embodiments in admixture with at least one pharmaceutically acceptable excipient suitable for the manufacture of a suspension.

In yet another embodiment, pharmaceutical compositions may be formulated as dispersible powders and granules suitable for preparation of a suspension by the addition of suitable excipients.

Excipients suitable for use in connection with suspensions include suspending agents, such as sodium carboxymethylcellulose, methylcellulose, hydroxypropyl methylcellulose, sodium alginate, polyvinylpyrrolidone, gum tragacanth, gum acacia, dispersing or wetting agents such as a naturally occurring phosphatide (e.g., lecithin), a condensation product of an alkylene oxide with a fatty acid (e.g., polyoxyethylene stearate), a condensation product of ethylene oxide with a long chain aliphatic alcohol (e.g., heptadecaethyleneoxycethanol), a condensation product of ethylene oxide with a partial ester derived from a fatty acid and a hexitol anhydride (e.g., polyoxyethylene sorbitan monooleate); polysaccharides and polysaccharide-like compounds (e.g. dextran sulfate); glycoaminoglycans and glycosaminoglycan-like compounds (e.g., hyaluronic acid); and thickening agents, such as carbomer, beeswax, hard paraffin or cetyl alcohol. The suspensions may also contain one or more preservatives such as acetic acid, methyl and/or n-propyl p-hydroxy-benzoate; one or more coloring agents; one or more flavoring agents; and one or more sweetening agents such as sucrose or saccharin.

Pharmaceutical compositions may also be in the form of oil-in water emulsions. The oily phase may be a vegetable oil, such as olive oil or arachis oil, a mineral oil, such as liquid paraffin, or a mixture of these. Suitable emulsifying agents include naturally-occurring gums, such as gum acacia and gum tragacanth; naturally occurring phosphatides, such as soybean lecithin, esters or partial esters derived from fatty acids; hexitol anhydrides, such as sorbitan monooleate; and condensation products of these partial esters with ethylene oxide, such as polyoxyethylene sorbitan monooleate. The emulsion may also contain sweetening and flavoring agents. Syrups and elixirs may be formulated with sweetening agents, such as glycerol, sorbitol or sucrose. Such formulations may also contain a demulcent, a preservative, a flavoring or a coloring agent.

Additionally, pharmaceutical compositions may be in the form of a sterile injectable preparation, such as a sterile injectable aqueous emulsion or oleaginous suspension. This emulsion or suspension may be formulated according to the known art using those suitable dispersing or wetting agents and suspending agents which have been mentioned above. The sterile injectable preparation may also be a sterile injectable solution or suspension in a nontoxic parenterally acceptable diluent or solvent, such as a solution in 1,2-propane-diol.

Sterile injectable preparations may also be prepared as a lyophilized powder. Among the acceptable vehicles and solvents that may be employed are water, Ringer's solution, and isotonic sodium chloride solution. In addition, sterile fixed oils may be employed as a solvent or suspending medium. For this purpose any bland fixed oil may be employed including synthetic mono- or diglycerides. In addition, fatty acids such as oleic acid may likewise be used in the preparation of injectables.

Many therapeutics have undesirably short half-lives and/or undesirable toxicity. Thus, the concept of improving half-life or toxicity is applicable to various treatments and fields. Pharmaceutical compositions can be prepared, however, by complexing the therapeutic with a biochemical moiety to improve such undesirable properties. Proteins are a particular biochemical moiety that may be complexed with a CSA for administration in a wide variety of applications. In some embodiments, one or more CSAs are complexed with a protein. In some embodiments, one or more CSAs are complexed with a protein to increase the CSA's half-life. In other embodiments, one or more CSAs are complexed with a protein to decrease the CSA's toxicity. Albumin is a particularly preferred protein for complexation with a CSA. In some embodiments, the albumin is fat-free albumin.

With respect to the CSA therapeutic, the biochemical moiety for complexation can be added to the pharmaceutical composition as 0.25, 0.5, 0.75, 1, 1.5, 2, 2.5, 3, 3.5, 4, 4.5, 5, 10, 20, 50, or 100 weight equivalents, or a range bounded by any two of the aforementioned numbers, or about any of the numbers. In some embodiments, the weight ratio of albumin to CSA is about 18:1 or less, such as about 9:1 or less. In some embodiments, the CSA is coated with albumin.

Alternatively, or in addition, non-biochemical compounds can be added to the pharmaceutical compositions to reduce the toxicity of the therapeutic and/or improve the half-life. Suitable amounts and ratios of an additive that can reduce toxicity can be determined via a cellular assay. With respect to the CSA therapeutic, toxicity reducing compounds can be added to the pharmaceutical composition as 0.25, 0.5, 0.75, 1, 1.5, 2, 2.5, 3, 3.5, 4, 4.5, 5, 10, 20, 50, or 100 weight equivalents, or a range bounded by any two of the aforementioned numbers, or about any of the numbers. In some embodiments, the toxicity reducing compound is a cocoamphodiacetate such as Miranol® (disodium cocoamphodiacetate). In other embodiments, the toxicity reducing compound is an amphoteric surfactant. In some embodiments, the toxicity reducing compound is a surfactant. In other embodiments, the molar ratio of cocoamphodiacetate to CSA is between about 8:1 and 1:1, preferably about 4:1. In some embodiments, the toxicity reducing compound is allantoin.

In some embodiments, a CSA composition is prepared utilizing one or more sufactants. In specific embodiments, the CSA is complexed with one or more poloxamer surfactants. Poloxamer surfactants are nonionic triblock copolymers composed of a central hydrophobic chain of polyoxypropylene (poly(propylene oxide)) flanked by two hydrophilic chains of polyoxyethylene (poly(ethylene oxide)). In some embodiments, the poloxamer is a liquid, paste, or flake (solid). Examples of suitable poloxamers include those by the trade names Synperonics, Pluronics, or Kolliphor. In some embodiments, one or more of the poloxamer surfactant in the composition is a flake poloxamer. In some embodiments, the one or more poloxamer surfactant in the composition has a molecular weight of about 3600 g/mol for the central hydrophobic chain of polyoxypropylene and has about 70% polyoxyethylene content. In some embodiments, the ratio of the one or more poloxamer to CSA is between about 50 to 1; about 40 to 1; about 30 to 1; about 20 to 1; about 10 to 1; about 5 to 1; about 1 to 1; about 1 to 10; about 1 to 20; about 1 to 30; about 1 to 40; or about 1 to 50. In other embodiments, the ratio of the one or more poloxamer to CSA is between 50 to 1; 40 to 1; 30 to 1; 20 to 1; 10 to 1; 5 to 1; 1 to 1; 1 to 10; 1 to 20; 1 to 30; 1 to 40; or 1 to 50. In some embodiments, the ratio of the one or more poloxamer to CSA is between about 50 to 1 to about 1 to 50. In other embodiments, the ratio of the one or more poloxamer to CSA is between about 30 to 1 to about 3 to 1. In some embodiments, the poloxamer is Pluronic F127.

The amount of poloxamer may be based upon a weight percentage of the composition. In some embodiments, the amount of poloxamer is about 10%, 15%, 20%, 25%, 30%, 35%, 40%, about any of the aforementioned numbers, or a range bounded by any two of the aforementioned numbers or the formulation. In some embodiments, the one or more poloxamer is between about 10% to about 40% by weight of a formulation administered to the patient. In some embodiments, the one or more poloxamer is between about 20% to about 30% by weight of the formulation. In some embodiments, the formulation contains less than about 50%, 40%, 30%, 20%, 10%, 5%, or 1% of CSA, or about any of the aforementioned numbers. In some embodiments, the formulation containes less than about 20% by weight of CSA.

The above described poloxamer formulations are particularly suited for the methods of treatment, device coatings, preparation of unit dosage forms (i.e., solutions, mouthwashes, injectables), etc.

In one embodiment, the compounds described herein may be formulated for oral administration in a lipid-based formulation suitable for low solubility compounds. Lipid-based formulations can generally enhance the oral bioavailability of such compounds.

A pharmaceutical composition may comprise a therapeutically or prophylactically effective amount of a compound described herein, together with at least one pharmaceutically acceptable excipient selected from the group consisting of- medium chain fatty acids or propylene glycol esters thereof (e.g., propylene glycol esters of edible fatty acids such as caprylic and capric fatty acids) and pharmaceutically acceptable surfactants such as polyoxyl 40 hydrogenated castor oil.

In an alternative embodiment, cyclodextrins may be added as aqueous solubility enhancers. Preferred cyclodextrins include hydroxypropyl, hydroxyethyl, glucosyl, maltosyl and maltotriosyl derivatives of α-, β-, and γ-cyclodextrin. A particularly preferred cyclodextrin solubility enhancer is hydroxypropyl-o-cyclodextrin (BPBC), which may be added to any of the above-described compositions to further improve the aqueous solubility characteristics of the compounds of the embodiments. In one embodiment, the composition comprises about 0.1% to about 20% hydroxypropyl-o-cyclodextrin, more preferably about 1% to about 15% hydroxypropyl-o-cyclodextrin, and even more preferably from about 2.5% to about 10% hydroxypropyl-o-cyclodextrin. The amount of solubility enhancer employed will depend on the amount of the compound of the embodiments in the composition.

In some exemplary embodiments, a CSA comprises a multimer (e.g., a dimer, trimer, tetramer, or higher order polymer). In some exemplary embodiments, the CSAs can be incorporated into pharmaceutical compositions or formulations. Such pharmaceutical compositions/formulations are useful for administration to a subject, in vivo or ex vivo. Pharmaceutical compositions and formulations include carriers or excipients for administration to a subject.

Such formulations include solvents (aqueous or non-aqueous), solutions (aqueous or non-aqueous), emulsions (e.g., oil-in-water or water-in-oil), suspensions, syrups, elixirs, dispersion and suspension media, coatings, isotonic and absorption promoting or delaying agents, compatible with pharmaceutical administration or in vivo contact or delivery. Aqueous and non-aqueous solvents, solutions and suspensions may include suspending agents and thickening agents. Such pharmaceutically acceptable carriers include tablets (coated or uncoated), capsules (hard or soft), microbeads, powder, granules and crystals. Supplementary active compounds (e.g., preservatives, antibacterial, antiviral and antifungal agents) can also be incorporated into the compositions.

Cosolvents and adjuvants may be added to the formulation. Non-limiting examples of cosolvents contain hydroxyl groups or other polar groups, for example, alcohols, such as isopropyl alcohol; glycols, such as propylene glycol, polyethyleneglycol, polypropylene glycol, glycol ether; glycerol; polyoxyethylene alcohols and polyoxyethylene fatty acid esters. Adjuvants include, for example, surfactants such as, soya lecithin and oleic acid; sorbitan esters such as sorbitan trioleate; and polyvinylpyrrolidone.

A pharmaceutical composition and/or formulation contains a total amount of the active ingredient(s) sufficient to achieve an intended therapeutic effect.

### CSA Synthesis

The methods disclosed herein may be as described below, or by modification of these methods. Ways of modifying the methodology include, among others, temperature, solvent, reagents etc., known to those skilled in the art. In general, during any of the processes for preparation disclosed herein, it may be necessary and/or desirable to protect sensitive or reactive groups on any of the molecules concerned. This may be achieved by means of conventional protecting groups, such as those described *in* Protective Groups in Organic Chemistry (ed. J.F.W. McOmie, Plenum Press, 1973); and P.G.M. Green, T.W. Wutts, Protecting Groups in Organic Synthesis (3rd ed.) Wiley, New York (1999). The protecting groups may be removed at a convenient subsequent stage using methods known from the art. Synthetic chemistry transformations useful in synthesizing applicable compounds are known in the art and include e.g. those described in R. Larock, Comprehensive Organic Transformations, VCH Publishers, 1989**,** or L. Paquette, ed., Encyclopedia of Reagents for Organic Synthesis, John Wiley and Sons, 1995. The routes shown and described herein are illustrative only and are not intended, nor are they to be construed, to limit the scope of the claims in any manner whatsoever. Those skilled in the art will be able to recognize modifications of the disclosed syntheses and to devise alternate routes based on the disclosures herein; all such modifications and alternate routes are within the scope of the claims.

Compounds described herein can be prepared by known methods, such as those disclosed in U.S. Patent No. 6,350,738. A skilled artisan will readily understand that minor variations of starting materials and reagents may be utilized to prepare known and novel cationic steroidal antimicrobials. For example, the preparation of CSA-13 disclosed in U.S. Patent No. 6,350,738 (compound 133) can be used to prepare CSA-92 by using hexadecylamine rather than octyl amine as disclosed. Schematically, for example, the preparation of certain compounds can be accomplished as follows:

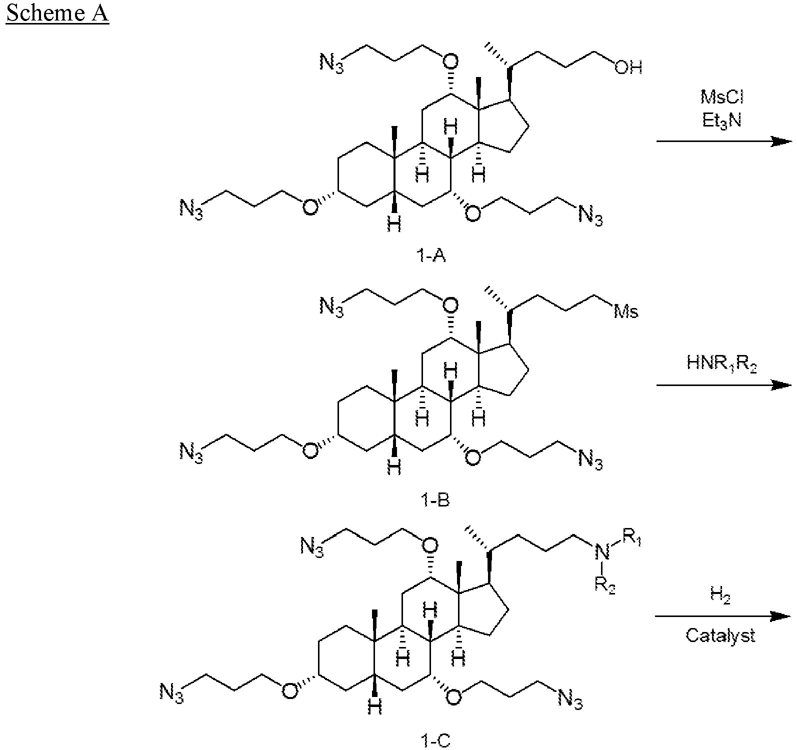

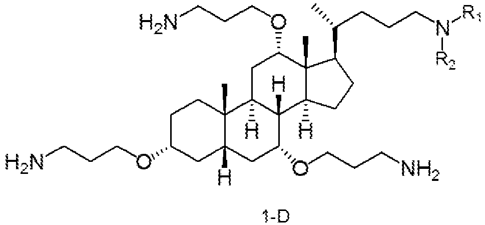

As shown above, compound 1-A is converted to the mesylate, compound 1-B using known conditions. Treatment of compound 1-B with a secondary amine, such as HNR₁R₂, results in the formation of compound 1-C, whose azido functional groups are reduced with hydrogen gas in the presence of a suitable catalyst to afford compound 1-D. Suitable catalysts include Palladium on Carbon and Lindlar catalyst. The reagent HNR₁R₂ is not particularly limited under this reaction scheme. For example, when R₁ is hydrogen and R₂ is a C₈-alkyl, CSA-13 is obtained from the synthesis. When R₁ is hydrogen and R₂ is a C₁₆-alkyl, CSA-92 is obtained from the synthesis. When R₁ and R₂ are both C₅-alkyl, CSA-90 is obtained from the synthesis. A skilled artisan will readily appreciate that this general synthetic scheme can be modified to prepare the CSAs described hereing, including CSAs with substituents and functional groups that are different from those generally described above.

An exemplary but non-limiting general synthetic scheme for preparing compounds of Formula (I), Formula (II), and/or Formula (III) is shown in Scheme B, below. Unless otherwise indicated, the variable definitions are as above for Formulae (I), (II) and/or (III).

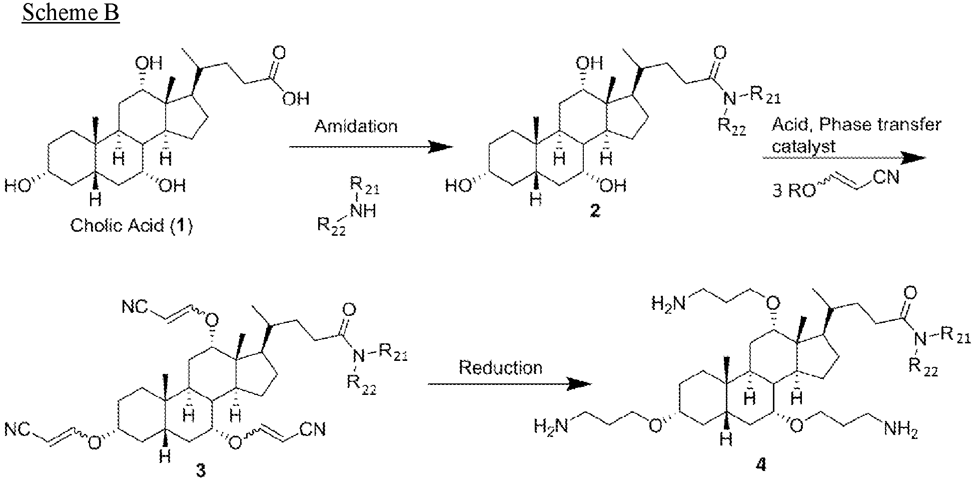

This process begins with cholic acid (1), or a derivative thereof. Treatment of (1) with a primary or secondary amine R₂₁R₂₂NH under amide bond forming conditions yields a final or intermediate CSA compound (2), or a derivative thereof. Amide bond forming conditions include, but are not limited to EDAC [N-(3-dimethylaminopropyl)-N'-ethylcarbodiimide hydrochloride] in the presence of HOBT (1-hydroxybenzotriazole), or HATU [N,N,N',N'-tetramethyl-O-(7-azabenzotriazol-1-yl)uronium hexafluorophosphate) in the presence of diisopropylethylamine, and the like.

In some embodiments, R₂₁ and R₂₂ are independently selected from the group consisting of hydrogen, C₁-C₂₄ alkyl, C₂-C₂₄ alkenyl, C₂-C₂₄ alkynyl, C₆ or C₁₀ aryl, 5 to 10 membered heteroaryl, 5 to 10 membered heterocyclyl, C₇₋₁₃ aralkyl, (5 to 10 membered heteroaryl)-C₁-C₆ alkyl, C₃₋₁₀ carbocyclyl, C₄₋₁₀ (carbocyclyl)alkyl, (5 to 10 membered heterocyclyl)-C₁-C₆ alkyl, and a suitable amine protecting group, provided that at least one of R₂₁ or R₂₂ is not a hydrogen.

In some embodiments, CSA compound (2), or a derivative thereof, can be treated with an alkoxyacroylonitrile reagent in the presence of acid and a phase transfer catalyst to yield a final or intermediate CSA compound of Formula (3), or a derivative thereof. In some embodiments, the acid is an organic acid. In some embodiments, the acid is an inorganic acid. In some embodiments, the acid is used in catalytic amounts. In some embodiments, the acid is used in stoichiometric amounts. In some embodiments, the acid is used in greater than stoichiometric amounts. In some embodiments, the phase transfer catalyst is tetrabutylammonium iodide. In some embodiments, the phase transfer catalyst is tetrabutylammonium bromide.

In some embodiments, CSA Compound (3), or a derivative thereof, can be subjected to reducing conditions suirable for forming CSA compound (4), or a derivative thereof. Suitable reducing conditions include, but are not limited to RedAl, lithium aluminum hydride, lithium borohydride, sodium borohydride, or treatment with hydrogen in the presence of a suitable metal catalyst (e.g., Raney cobolt), or treatment with silyl hydrides in the presence of a suitable metal catalyst. Suitable metal catalysts are known in the art.

An exemplary synthetic scheme for preparing CSA-192 is shown in Scheme C below.

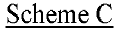

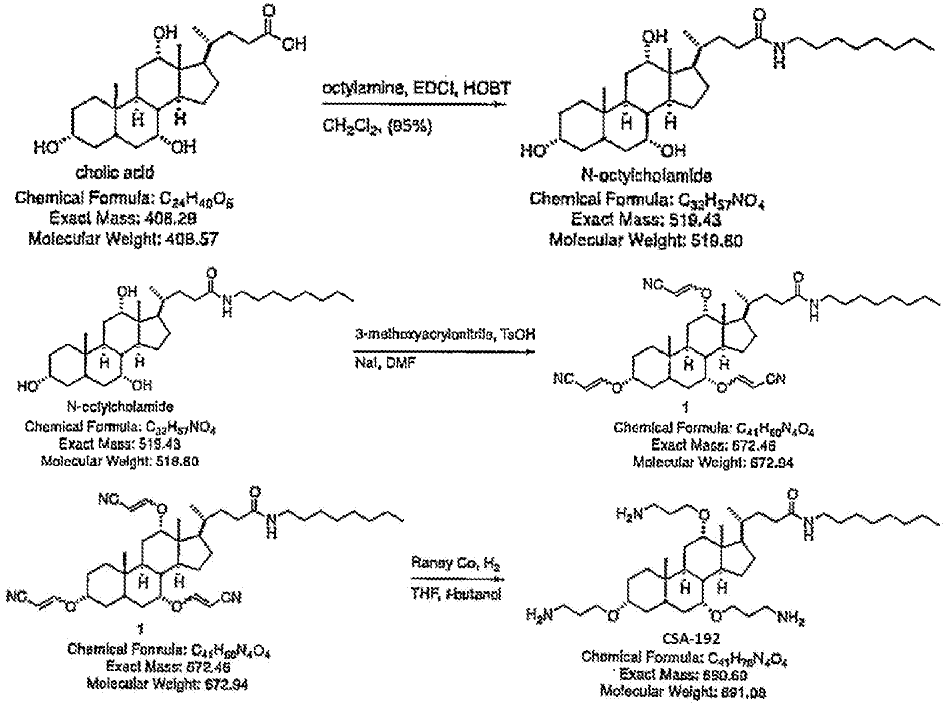

In some embodiments, CSA compounds as disclosed herein can be converted into a mesylate salt form, such as to form a pro-drug or hydrolysable intermediate, by reacting one or more amine groups with methylsulfonic acid or derivative thereof (*e*.*g*., acid halide). For example, CSA-192 can be converted into its mesylate salt form (CSA-192MS) by reacting CSA-192 with 3 equivalents of methylsulfonic acid.

### EXAMPLES

### Counterion Selection

Counterions were selected based upon toxicity information (i.e., Merck Class 1, 2, and 3), as well as pKa values, known solubilities of CSA free bases, and the anticipated mode of administration for the drug product. However, only those examples relating to the preparation, characterisation or use of 1,5-naphthalenedisulfonic acid di-addition salts of CSA compounds form part of the present invention.

### CSA-13

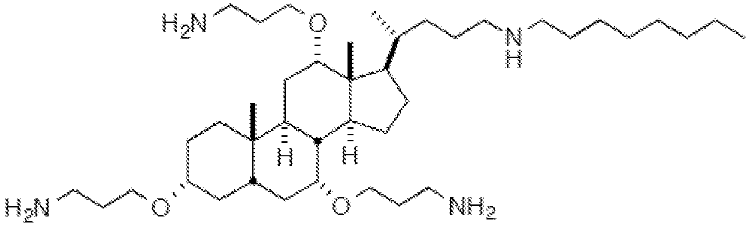

The free base of CSA-13 is obtained by neutralizing the hydrochloride salt as described in U.S. Patent No. 6,350,738.

### pKa Measurements of CSA-13

CSA-13 has four basic functional groups. pKa analysis was performed using the pH-metric method, with the sample being titrated in a triple titration from pH 2.0 to 12.1. CSA-13 pKa values were measured as 10.77 ± 0.05, 10.01 ± 0.09, 9.65 ± 0.04, and 9.01 ± 0.05.

### Solvent Solubility Test

Preliminary solubility tests were performed on the free base of CSA-13, reported in Table 1 below:

**Table 1**

| **Solvent** | **Approximate Solubility (mg/mL)** | **Observations** |
|---|---|---|
| Acetone | *ca*.335 | Dissolution was observed. Solvent colour changed to dark brown, after 24 hours at ambient. |
| Acetonitrile | < 10 | Initial gum-like material converted to a white solid. After 100 vol., the mixture was cloudy. |
| 1-Butanol | *ca.*165 | Dissolution was observed. |
| Cyclohexane | *ca.*199 | Dissolution was observed. |
| Dichloromethane | *ca*.415 | Dissolution was observed. |
| Diisopropyl ether | < 10 | Initial gum-like material converted to a white solid. After 100 vol., the mixture was cloudy. |
| Dimethylformamide | *ca.*343 | Dissolution was observed. |
| Dimethylsulfoxide | *ca.*246 | Dissolution was observed. |
| 1,4-Dioxane | < 10 | Initial gum-like material converted to a white solid. After 100 vol., the mixture was cloudy. |
| Ethanol | *ca*.406 | Dissolution was observed. |
| Ethyl acetate | < 10 | Initial gum-like material converted to a white solid. After 100 vol., the mixture was cloudy. |
| Heptane | < 10 | Initial gum-like material converted to a white solid. After 100 vol., the mixture was cloudy. |
| Isopropyl acetate | < 10 | Initial gum-like material converted to a white solid. After 100 vol., the mixture was cloudy. |
| Methanol | *ca.*400 | Dissolution was observed. |
| Methyl ethyl ketone | *ca*.413 | Dissolution was observed. Pale yellow after 24 hours at ambient. |
| Methyl isobutyl ketone | *ca*.340 | Dissolution was observed. Pale yellow after 24 hours at ambient. |
| *N*-Methyl-2-pyrrolidone | *ca*.248 | Dissolution was observed. |
| Nitromethane | < 10 | Complete dissolution was not observed and the colour of the mixture was yellow. |
| 2-Propanol | *ca.*263 | Dissolution was observed. |
| *tert*-Butylmethyl ether | *ca.*199 | Dissolution was observed. |
| Tetrahydrofuran | < 10 | Initial gum-like material converted to a white solid. After 100 vol., the mixture was cloudy. |
| Toluene | *ca.*250 | Dissolution was observed. |
| Water | *ca.*205 | Dissolution was observed. |
| Acetonitrile: Water (10%) | *ca.*198 | Dissolution was observed. |

Solubility values were estimated by a solvent addition technique, based on the following protocol: CSA-13 (20 mg) was weighed and individually distributed to 24 vials. Each solvent was added to the appropriate vial in 10 aliquots of 10 µL, 5 aliquots of 20 µL, 3 aliquots of 100 µL, and 1 aliquot of 500 µL. If complete dissolution was observed, the additions were stopped. Between additions, the sample was stirred to further encourage dissolution. If 2000 µL of solvent was added without dissolution, the solubility was calculated to be below this point. Polarized light microscopy analysis was performed on solids obtained from acetonitrile, 1,4-dioxane, ethyl acetate, isopropanol, and THF.

Based upon the solubility, diversity, toxicity, and stability of CSA-13 in the preliminary solubility tests, the following ICH Class 2 solvents were selected for salt screening experiments: Acetonitrile: Water (10%), Methanol, Tetrahydrofuran, and Toluene. Additionally, 2-Propanol and *tert*-Butylmethyl ether were also selected.

### Counterions for CSA-13 Salt Screening:

Counterions/acids for the proposed salt screening of CSA-13 were selected on the basis of CSA-13's measured pKa values, described above, and the likelihood of salt formation, which was estimated in part by a greater than about 2 pKa unit difference between the CSA pKA and the free acid pKa of the counterion. Table 2 below lists the counterions/acids identified for preliminary salt screening experiments of CSA-13:

**Table 2**

| **Counterion/acid** | **Class** | **pKa 1** | **pKa 2** | **pKa 3** | **Equivalents** |
|---|---|---|---|---|---|
| Benzoic acid | 2 | 4.19 | - | - | 4 |
| Benzenesulphonic acid | 2 | 0.70 | - | - | 2 |
| Benzenesulphonic acid | 2 | 0.70 | - | - | 4 |
| Citric acid | 1 | 3.13 | 4.76 | 6.40 | 1 |
| Citric acid | 1 | 3.13 | 4.76 | 6.40 | 2 |
| Fumaric acid | 1 | 3.03 | 4.38 | - | 2 |
| Galactaric acid (Mucic Acid) | 1 | 3.08 | 3.63 | - | 2 |
| Hydrochloric acid | 1 | -6.10 | - | - | 2 |
| Hydrochloric acid | 1 | -6.10 | - | - | 4 |
| 1-Hydroxy-2-Naphthoic acid | 2 | 2.70 | 13.50 | - | 2 |
| 1,5-Naphthalenedisulfonic acid | 2 | -3.37 | -2.64 | - | 2 |
| Pamoic acid | 2 | 2.51 | 3.10 | - | 2 |
| Phosphoric acid | 1 | 1.96 | 7.12 | 12.32 | 4 |
| Succinic acid | 1 | 4.21 | 5.64 | - | 2 |
| Sulphuric acid | 1 | -3.00 | 1.92 | - | 2 |
| L-Tartaric acid | 1 | 3.02 | 4.36 | - | 2 |

Salt screening was carried out using the following protocol: CSA-13 (approximately 25 mg) was slurried or dissolved in the respective solvent, and then mixed with the appropriate equivalents of the acid counterion (specified in Table 2, above). The mixtures of CSA-13 / counterion / solvent were temperature cycled between ambient and 40° C in four hour cycles for a period of approximately 48 hours. The following counterions and solvent combinations were identified from the preliminary screening and advanced to secondary screening:

**Table 3**

| **Counterion/acid** | **Equivalent** | **Solvent System** |
|---|---|---|
| 1,5-Naphthalenedisulfonic acid | 2 | Acetonitrile:water (10%) |
| Sulphuric acid | 2 | tetrahydrofuran |
| Hydrochloric acid | 2 | tetrahydrofuran |
| Hydrochloric acid | 4 | *tert*-Butylmethyl ether |
| Fumaric acid | 2 | *tert*-Butylmethyl ether |

### Secondary Salt Screening: 1,5-Naphthalenedisulfonic Acid

Approximately 300 mg of CSA-13 was weighed into a scintillation vial. 1.2 mL of acetonitrile:water (10%) was added to the vial. 1,5-Naphthalenedisulfonic acid (2 equivalents) was then added to the vial, resulting in precipitation. A further 1.2 mL of acetonitrile:water (10%) was then added to the vial. The reaction mixture of CSA-13 / counterion / solvent was then temperature cycled (40° C / RT, four hour cycles) for approximately 48 hours. Solids were isolated and dried at ambient temperature prior to analysis. Polarized light microscopy of the 1,5-naphthalenedisulfonate salt of CSA-13 prepared from the secondary salt screening indicated that the material was birefringent and needle-like. FTIR analysis afforded the following results: peaks were identified at about 2925, 2866, 1625, 1500, 1468, 1363, 1240, 1221, 1153, 1108, 1061, 906, 791, 765, 665, 612, 569, 527, and 465 cm⁻¹. The ¹H NMR spectrum for the 1,5-naphthalenedisulfonate salt of CSA-13 was also obtained. In addition to peaks attributable to the 1,5-naphthalenedisulfonate counterion, shifts in peaks were observed as compared to the free base of CSA-13. HPLC analysis indicated a purity of about 99 percent.

### Secondary Salt Screening: Sulfuric Acid

Approximately 300 mg of CSA-13 was weighed into a scintillation vial. 6 mL of tetrohydrofuran was added to the vial. Sulfuric acid (2 equivalents) was then added to the vial, resulting in slight precipitation. The reaction mixture of CSA-13 / counterion / solvent was then temperature cycled (40° C / RT, four hour cycles) for approximately 48 hours. After cycling, a very thin slurry was observed. The solvent was filtered and the solid was dried, affording a gum. The gum was then re-dissolved in 2-propanol, resulting in a slurry that was then temperature cycled (40° C / RT, four hour cycles) for approximately 48 hours. Solids were isolated and dried at ambient temperature prior to analysis.

Approximately 1 g of CSA-13 was weighed into a scintillation vial. 7 mL of 2-propanol was added to the vial. Sulfuric acid (1 equivalent) was then added to 0.5 mL of 2-propanol, and this solution was added to the vial. The reaction mixture of CSA-13 / counterion / solvent was then temperature cycled (40° C / RT, four hour cycles) for approximately 48 hours. After cycling, solvent was evaporated to afford a slurry, which was further temperature cycled (40° C / RT, four hour cycles) for approximately 48 hours. Solids were isolated and analysed wet by PXRD and then dried at ambient temperature prior to further analysis.

Analysis of the sulfate salt of CSA-13 prepared from the secondary salt screening indicated that the material was highly crystalline, with no clearly defined morphology. FTIR analysis afforded the following results: peaks were identified at about 2925, 2864, 1618, 1533, 1466, 1364, 1155, 1093, 1027, 854, 611, 579, and 434 cm⁻¹. The ¹H NMR spectrum for the sulfate salt of CSA-13 was also obtained. Shifts in peaks were observed as compared to the free base of CSA-13. HPLC analysis indicated a purity of about 99 percent. Ion chromatography analysis indicates that the ratio of CSA-13 to sulfate counterion was about 1:1.

A solubility screen was performed as described above for the sulphate salt of CSA-13. The results are provided in Table 4, below:

**Table 4**

| **Solvent** | **Approximate Solubility at 22° C (mg/mL)** |
|---|---|
| Acetone | < 10.5 |
| Acetonitrile | < 10.4 |
| 1-butanol | > 37.7 |
| Dichloromethane | > 193.9 |
| 1,4-dioxane | < 11.3 |
| Ethanol | > 98.4 |
| Methanol | > 199.7 |
| 2-propanol | > 20.9 |
| TBME | < 10.1 |
| Tetrahydrofuran | < 11.0 |
| Toluene | > 102.8 |

### Secondary Salt Screening: Hydrochloride Salt (2 Equivalents)

Approximately 300 mg of CSA-13 was weighed into a scintillation vial. 6 mL of tetrohydrofuran was added to the vial. Hydrochloric acid (2 equivalents) was then added to the vial. The reaction mixture of CSA-13 / counterion / solvent was then temperature cycled (40° C / RT, four hour cycles) for approximately 48 hours. After cycling, a thin slurry was observed. The solvent was filtered and the solid was dried, affording a gum. The gum was then re-dissolved in 2-propanol, resulting in a slurry that was then temperature cycled (40° C / RT, four hour cycles) for approximately 48 hours. Solids were isolated and dried at ambient temperature prior to analysis. Analysis indicated that the material was not fully crystalline and lacked a defined morphology. Ion chromatography analysis indicated that the ratio of CSA-13 to hydrochloride counterion was about 1:2.5. The material further appeared amorphous after 1 week stability study under all tested conditions.

### Secondary Salt Screening: Hydrochloride Salt (4 Equivalents)

Approximately 300 mg of CSA-13 was weighed into a scintillation vial. 6 mL of *tert-*butyl methyl ether was added to the vial. Hydrochloric acid (4 equivalents) was then added to the vial. The reaction mixture of CSA-13 / counterion / solvent was then temperature cycled (40° C / RT, four hour cycles) for approximately 48 hours. After cycling, heptane anti-solvent addition was performed, resulting in the formation of a gum. The gum was then re-dissolved in 2-propanol and evaporated to afford a solid. The solid was re-slurried in *tert-*butyl methyl ether and then temperature cycled (40° C / RT, four hour cycles) for approximately 72 hours.

Analysis indicated that the material was amorphous upon evaporation from the temperature cycle. Further slurrying and temperature cycling for 72 hours failed for afford crystallization.

### Additional Salt Screening

### Salt No. 1

Approximately 300 mg of CSA-13 freebase is dissolved in 1.5 mL of *tert-*Butylmethyl ether at about 22°C. A sulfuric acid solution is prepared by adding about 1 equivalent (0.44 mmol) of sulfuric acid to 500µL of *tert*-Butylmethyl ether at about 22°C. The crystallization is seeded using approximately 3-6 mg of seed Form 3. The sulfuric acid solution in *tert*-Butylmethyl ether is added in 50µL aliquots. The solution is then stirred at about 22°C for 1 hour. Ethyl acetate (*ca.* 1.35 mL) is added as an anti-solvent at about 22°C. After anti-solvent addition, the solution is cooled down to 0 °C and the precipitated material is isolated using a centrifuge. The isolated material is dried under vacuum at ambient for 2 hours to provide 285 mg (83% yield) of CSA-13 monosulfate salt as a partially crystalline Form 1 material with 98% purity by HPLC.

### Salt No. 2

Approximately 300 mg of CSA-13 freebase is dissolved in 1.5 mL of *tert-*Butylmethyl ether at about 22°C. A sulfuric acid solution is prepared by adding about 1 equivalent (0.44 mmol) of sulfuric acid to 500µL of *tert*-Butylmethyl ether at about 22°C. The crystallization is seeded using approximately 3-6 mg of seed Form 3. The sulfuric acid solution in *tert*-Butylmethyl ether is added in 50µL aliquots. The solution is then stirred at about 22°C for 1 hour. The solution is cooled to 5 °C and ethyl acetate (*ca.* 1.35 mL) is added as an anti-solvent. After anti-solvent addition, the solution is cooled down to 0 °C and the precipitated material is isolated using a centrifuge. The isolated material is dried under vacuum at ambient for 2 hours to provide 248 mg (72% yield) of CSA-13 monosulfate salt as a partially crystalline Form 1 material with 99% purity by HPLC.

### Salt No. 3

Approximately 100 mg of CSA-13 sulfate salt No. 1 is dissolved in 0.75 mL of methanol at ambient (22°C). The solution is seeded with 1-2mg of seed (Form 3). About 0.71 mL of ethyl acetate is added and the solution is stirred at about 22°C for about 1 hour. The solution is cooled down from 22°C to 5°C and isolated by centrifugation. The isolated material is dried under vacuum at ambient for 2 hours to provide 90 mg (90% yield) of CSA-13 monosulfate salt as a highly crystalline Form 3 material with 99% purity by HPLC.

### Salt No. 4

Approximately 100 mg of CSA-13 sulfate salt No. 2 is dissolved in 0.75 mL of methanol at ambient (22°C). The solution is seeded with 1-2mg of seed (Form 3). About 0.71 mL of ethyl acetate is added and the solution is stirred at about 22°C for about 1 hour. The solution is cooled down from 22°C to 5°C and isolated by centrifugation. The isolated material is dried under vacuum at ambient for 2 hours to provide 86 mg (86% yield) of CSA-13 monosulfate salt as a highly crystalline Form 3 material with 99% purity by HPLC.

### Salt No. 5

Approximately 300 mg of CSA-13 was weighed into a scintillation vial. 6 mL of *tert-*butyl methyl ether was added to the vial. Fumaric acid (2 equivalents) was then added to the vial. A further 2 mL of tert-butyl methyl ether was added and the reaction mixture of CSA-13 / counterion / solvent was then temperature cycled (40° C / RT, four hour cycles) for approximately 48 hours. After cycling, solids were isolated and dried at ambient temperature. PXRD indicated that the material corresponded to fumaric acid. Solids were re-slurried in the mother liquor and then temperature cycled (40° C / RT, four hour cycles) for approximately 72 hours, with the resulting solid determined to be amorphous.

### Salt No. 6

CSA-13 free base is dissolved in EtOH (360 mL) and heated to 60-65°C. A solution of NDSA (27.8 g, 77.1 mmol, 2.3 eq) in EtOH/H₂O (1/1 vol/vol; 150 mL) is added over an hour. At the end of the addition, the mixture is cooled to 45°C, seeded (110 mg) and aged overnight at 45°C. The thick slurry obtained is cooled slowly to 0-5°C, held at that temperature for 1-2 hours then isolated by filtration. The cake is washed with cold EtOH (2 x 40 mL), dried on the funnel under vacuum and a rubber dam until no further filtrates were observed, then dried in a vacuum oven at 30-40°C overnight to provide 31.9 g of CSA-13 di-NDSA salt as a white solid.

### Salt No. 7

Approximately 125 mL of ethanol is added to 124 g of CSA-13 free base and the mixture is stirred for 30 minutes at 40°C for 30 minutes. The mixture is then cooled to 5-10°C. Separately, 125 mL of ethanol is cooled to 5-10°C and 11.2 mL of concentrated sulfuric acid is added. The sulfuric acid solution is then added slowly to the CSA-13 free base solution and an exotherm to about 35°C is observed. The reaction mixture is then stirred at 40°C for 4 hours. The mixture is allowed to cool overnight to ambient temperature. CSA-13 monosulfate seeds are added and the mixture is cooled to 0-5° and stirred for 4 hours. The mixture is then heated to 40°C and stirred for 4 hours. The mixture is then allowed to cool overnight to ambient temperature. 1.88 L of MTBE is added to the reaction mixture and the mixture is cooled to 0-5°C and stirred for 4 hours. The mixture is then heated to 40 °C and stirred for 4 hours. The mixture is then cooled to 0-5 °C and stirred for hours. The reaction mixture is then filtered to obtain 113 g of CSA-13 monosulfate salt with a purity of 97.0 % (AUC).

### Salt No. 8

CSA-13 free base (488 mg) is taken up in 10.0 mL of acetonitrile. The mixture was heated to 60-65°C at which time a solution of NDSA (640 mg, 2.5 eq) in 6.0 mL of 1:1 acetonitrile/water is added over about 45 minutes, with solids forming almost immediately (no seeds added). After holding at 60-65°C for about an hour the batch is slowly cooled to ambient temperature for an overnight stir period. The mixture is cooled in an ice bath and the solids isolated by filtration on a Buchner funnel. After drying (air drying then in a vacuum drying oven), a total of 532 mg of CSA-13 di-NDSA salt was obtained as a pure white solid.

### Conversion back to CSA-13 free base

CSA-13 di-NDSA salt (0.75 g, 520-068) is combined with 2-MeTHF (7.5 mL) and then an aqueous solution of KOH (0.41 g in 4 mL water) is added. The slurry is aged for 1 h at room temperature during which time a noticeable form change in the slurry is observed. The solids are removed by filtration and the filtrate layers were separated. Toluene (7.5 mL) is added to the organic layer and then washed twice with water (5 mL) before concentrating to an oil to obtain CSA-13 free base (0.5g). Analysis of the oil and solids indicated no CSA-13 is lost on the solid and that no NDSA remained in the CSA-13 free base.

All x-ray powder diffraction 2θ values are measured with an error of ± 0.2 units.

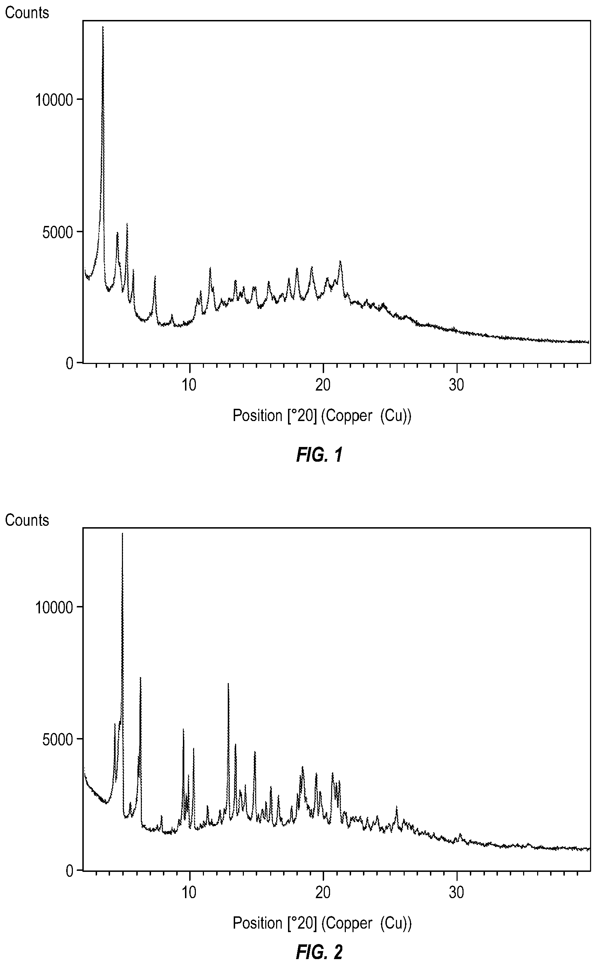

The CSA-13 monosulfate salt formed herein (as in Salt No. 1 or No. 2) is subjected to XRPD analysis and the pattern shown in Figure 1 and tabulated in Table 5 is obtained. This material is described as the Form 1 polymorph of the CSA-13 monosulfate salt.

**Table 5: Form 1 Peak List**

| Pos. [°2θ] | Height [cts] |
|---|---|
| 3.4821 | 10149.73 |
| 4.5781 | 2575.83 |
| 5.2611 | 3237.31 |
| 5.7349 | 1648.87 |
| 7.3569 | 1698.68 |
| 11.5038 | 2272.18 |
| 11.7280 | 1524.92 |
| 13.3929 | 1827.59 |
| 13.9766 | 1554.22 |
| 17.3642 | 1944.76 |
| 17.9760 | 2308.27 |
| 19.0918 | 2416.90 |
| 21.2289 | 2687.24 |

The CSA-13 monosulfate salt formed as in Salt No. 3 or No. 4 is subjected to XRPD analysis and the pattern shown in Figure 2 and tabulated in Table 6 is obtained. This material is described as the Form 3 polymorph of the CSA-13 monosulfate salt.

**Table 6: Form 3 Peak List**

| Pos. [°2θ] | Height [cts] |
|---|---|
| 4.3665 | 3372.09 |
| 4.7145 | 3615.42 |
| 4.9167 | 11204.68 |
| 6.0934 | 2707.50 |
| 6.2547 | 5888.55 |
| 9.4794 | 4141.07 |
| 9.8539 | 2347.16 |
| 10.2449 | 3408.60 |
| 12.8438 | 6130.97 |
| 13.3815 | 3634.65 |
| 14.7948 | 3394.60 |
| 15.9971 | 1975.64 |
| 16.5681 | 1684.32 |
| 18.2047 | 2482.62 |
| 18.3891 | 2854.19 |
| 19.3919 | 2570.58 |
| 20.6269 | 2699.97 |
| 20.8990 | 2262.26 |
| 21.1318 | 2286.23 |

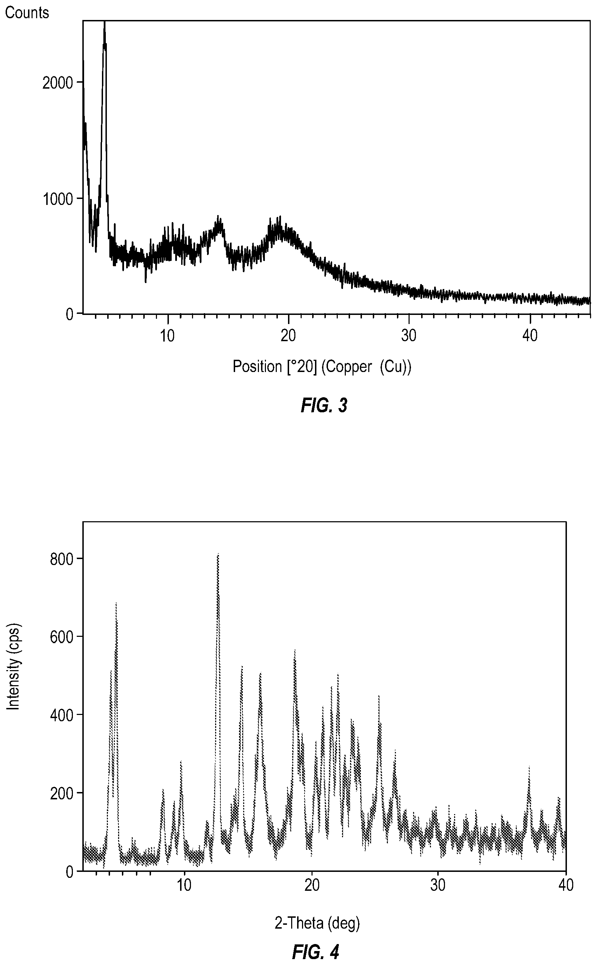

The CSA-13 monosulfate salt prepared as described in Salt No. 5 is subjected to XRPD analysis and the pattern shown in Figure 3 is obtained, indicating the sample is predominantly amorphous.

The di-NDSA salt prepared as in Salt No. 6 is subjected to XRPD analysis and the pattern shown in Figure 4 and tabulated in Table 7 is obtained.

**Table 7: Peak List**

| 2-theta(deg) | Height(cps ) |
|---|---|
| 4.216(9) | 252(29) |
| 4.629(8) | 344(34) |
| 8.29(2) | 88(17) |
| 9.13(2) | 61(14) |
| 9.739(17) | 115(20) |
| 12.641(9) | 464(39) |
| 14.457(14) | 273(30) |
| 15.864(19) | 217(27) |
| 18.610(18) | 190(25) |
| 19.200(8) | 144(22) |
| 20.242(18) | 129(21) |
| 20.803(14) | 187(25) |
| 21.512(15) | 206(26) |
| 22.014(13) | 255(29) |
| 22.57(2) | 115(20) |
| 23.169(19) | 168(24) |
| 23.63(3) | 133(21) |
| 25.227(18) | 183(25) |
| 26.44(3) | 118(20) |
| 37.05(4) | 82(16) |
| 39.33(5) | 59(14) |

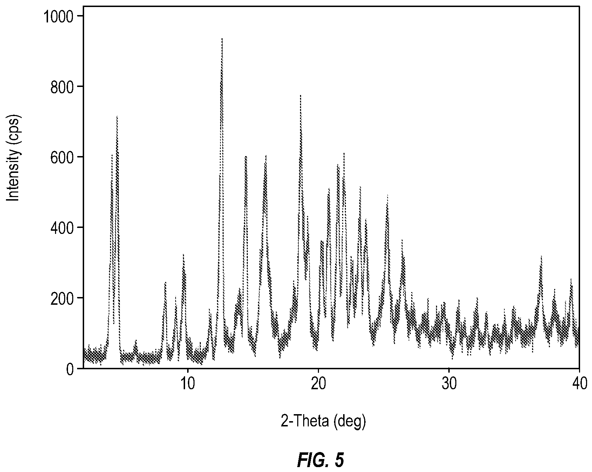

The di-NDSA salt prepared as in Salt No. 8 is subjected to XRPD analysis and the pattern shown in Figure 5 and tabulated in Table 8 is obtained.

**Table 8: Peak List**

| 2-theta(deg) | Height(cps ) |
|---|---|
| 4.200(7) | 298(31) |
| 4.606(6) | 384(36) |
| 8.292(13) | 125(20) |
| 9.113(15) | 87(17) |
| 9.728(14) | 155(23) |
| 11.71(2) | 59(14) |
| 12.625(7) | 511(41) |
| 13.95(2) | 83(17) |
| 14.444(9) | 324(33) |
| 15.826(19) | 258(29) |
| 18.622(7) | 324(33) |
| 19.20(2) | 180(24) |
| 20.22(2) | 143(22) |
| 20.767(16) | 221(27) |
| 21.482(16) | 251(29) |
| 21.958(17) | 264(30) |
| 22.53(3) | 91(17) |
| 23.12(2) | 185(25) |
| 23.61(3) | 151(22) |
| 25.26(3) | 187(25) |
| 26.55(6) | 100(18) |
| 37.01(4) | 92(17) |

Surprisingly it was found that the formation of the di-NDSA salt can be used to provide significantly improved purity with less pure CSA-13 free base. The di-NDSA salt can then be converted back to the free base. The purified CSA-13 free base can then be converted to the monosulfate salt as described herein.

### Summary of Data for CSA-13 Salts:

The following table summarizes the purity for select CSA-13 salts under various conditions:

**Table 9**

| **Salt** | **Conditions** | **Purity (%)** |
|---|---|---|
| 1,5-naphthalenedisulfonate salt | Starting Purity | 98.52 |
| | 40°C/75% RH | 97.93 |
| | 80°C | 98.16 |
| | Ambient light | 98.57 |
| Sulfate salt | Starting Purity | 99.34 |
| | 40°C/75% RH | 97.50 |
| | 80°C | 97.96 |
| | Ambient light | 99.76 |

Based upon the experiments for CSA-13, described above, it was unexpectedly found that the 1,5-naphthalenedisulfonate salt had favorable solid state properties and scalability amongst the measured counterions. The sulfate salt of CSA-13 also provided unexpected and favorable properties, including improved solubility.

### CSA-131

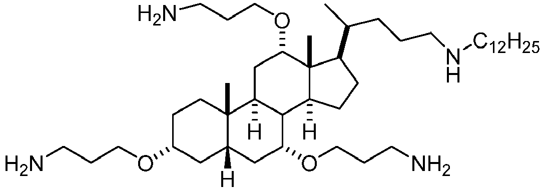

The free base of CSA-13 is obtained by neutralizing the hydrochloride salt as described in U.S. Patent No. 6,350,738.

CSA-131 has some structural similarities with CSA-13. As such, CSA-131 should have a similar pKa profile. Additionally, it was found that the di-NDSA salt of CSA-131 can be prepared, as was the case with CSA-13.

The free base of CSA-131 (146 g, with an area percent purity of 88.4%) was dissolved in EtOH (2.15 L, 200 proof) and filtered through a 0.20 µM frit into a 5L reaction flask. The solution was heated to 60-65 °C at which time 1,5-napthalenedisulfonic acid tetrahydrate (NDSA; 161.5 g, 448 mmoles, 2.25 eq.) was added as a solution in 1/1 EtOH/H₂O (900 mL) over 1.75 hours. When approximately 60% of the NDSA solution was added, a small amount of crystallization/precipitation was observed. At the end of the addition significant solids were present. No seeding was employed. The solution was slowly cooled to ambient temperature for an overnight stir period. The next morning the batch was cooled to 0-5 °C and filtered on a funnel to collect the product using ice-cold EtOH to aid in the transfer/provide first rinse of cake (200 mL). The cake was washed with ice-cold EtOH (2 x 225 mL), dried on the funnel under a latex dam until filtrates ceased, and then dried in a vacuum drying oven until constant weight to provide the CSA-131 di NDSA salt as a white solid: 197.2 g (75.7% yield) with an HPLC area percent purity of 97.7%.

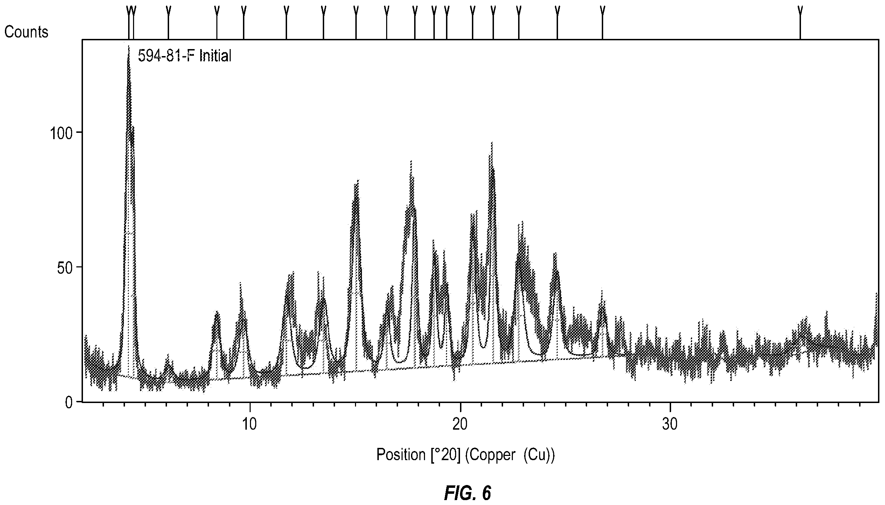

A sample of the CSA-131 2NDSA salt was analyzed by x-ray powder diffraction (XRPD) and the following spectrum was obtained (shown in Figure 6 and tabulated in Table 10), showing that the salt has a high degree of crystallinity.

**Table 10**

| **No.** | **Pos. [°2θ]** | **d-spacing [Å]** | **Height [cts]** | **Relative Height %** |
|---|---|---|---|---|
| 1 | 4.1922 | 21.07771 | 108.88 | 100.00 |
| 2 | 4.4257 | 19.96645 | 62.48 | 57.38 |
| 3 | 6.118 | 14.44666 | 5.30 | 4.87 |
| 4 | 8.3931 | 10.53507 | 21.73 | 19.96 |
| 5 | 9.6769 | 9.14015 | 19.44 | 17.85 |
| 6 | 11.7232 | 7.54887 | 26.83 | 24.64 |
| 7 | 13.4959 | 6.56107 | 24.59 | 22.58 |
| 8 | 15.0514 | 5.88631 | 59.18 | 54.35 |
| 9 | 16.5064 | 5.37059 | 20.03 | 18.40 |
| 10 | 17.8322 | 4.97418 | 54.03 | 49.62 |
| 11 | 18.7671 | 4.72842 | 38.17 | 35.06 |
| 12 | 19.3449 | 4.58848 | 26.69 | 24.51 |
| 13 | 20.596 | 4.31251 | 46.49 | 42.70 |
| 14 | 21.5538 | 4.12298 | 66.44 | 61.02 |
| 15 | 22.7706 | 3.90535 | 35.03 | 32.17 |
| 16 | 24.6057 | 3.61809 | 30.25 | 27.78 |
| 17 | 26.7689 | 3.33041 | 16.95 | 15.57 |
| 18 | 36.2048 | 2.48116 | 5.65 | 5.19 |

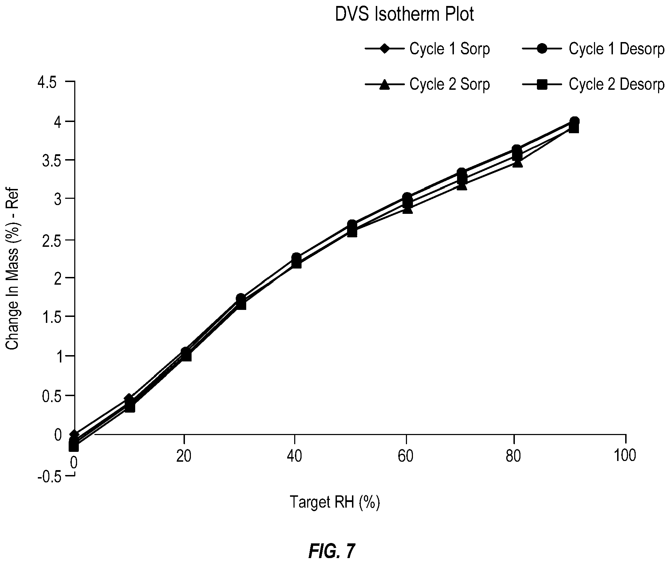

A sample of the CSA-131 2NDSA salt was subjected to a dynamic vapor sorption (DVS) analysis and results were obtained (Figure 7), showing that the salt shows minimal hysteresis.

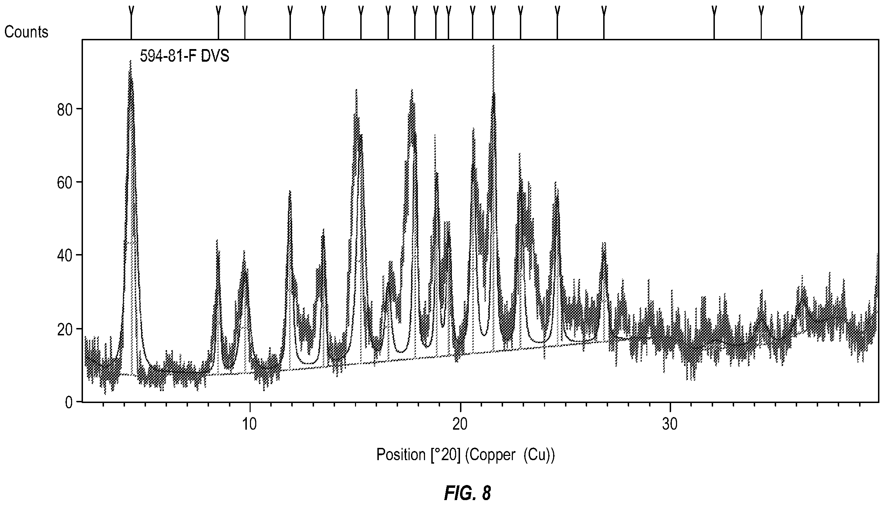

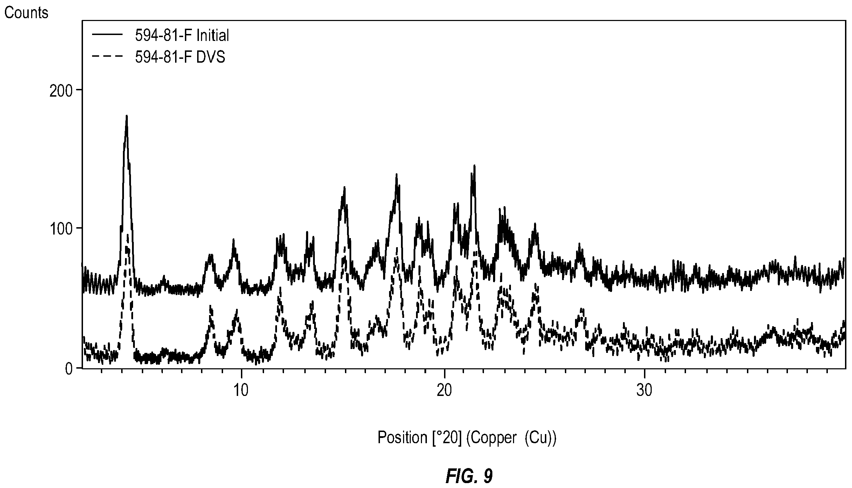

After being subjected to the DVS analysis, a sample was subjected to XRPD analysis and a spectrum was obtained (shown in Figure 8 and tabulated in Table 11), showing that the DVS analysis did not significantly impact crystallinity. Figure 9 provides an overlay of the XRPD spectrum pre- and post-DVS analysis.

**Table 11**

| **No.** | **Pos. [°2θ]** | **d-spacing [Å]** | **Height [cts]** | **Relative Height %** |
|---|---|---|---|---|
| 1 | 4.3296 | 20.40912 | 73.81 | 100.00 |
| 2 | 8.4622 | 10.44922 | 29.18 | 39.53 |
| 3 | 9.7475 | 9.0741 | 25.19 | 34.13 |
| 4 | 11.8734 | 7.45376 | 44.6 | 60.43 |
| 5 | 13.482 | 6.56779 | 32.94 | 44.63 |
| 6 | 15.249 | 5.81049 | 57.24 | 77.55 |
| 7 | 16.5541 | 5.35522 | 18.92 | 25.63 |
| 8 | 17.8375 | 4.9727 | 57.2 | 77.50 |
| 9 | 18.8803 | 4.70033 | 46.14 | 62.51 |
| 10 | 19.4351 | 4.56739 | 29.82 | 40.40 |
| 11 | 20.5833 | 4.31514 | 47.3 | 64.08 |
| 12 | 21.5768 | 4.11863 | 66.11 | 89.57 |
| 13 | 22.8336 | 3.8947 | 41.26 | 55.90 |
| 14 | 24.6093 | 3.61756 | 38.51 | 52.17 |
| 15 | 26.8236 | 3.32374 | 22.52 | 30.51 |
| 16 | 32.1213 | 2.78664 | 2.08 | 2.82 |
| 17 | 34.323 | 2.61276 | 6.29 | 8.52 |
| 18 | 36.2506 | 2.47813 | 8.08 | 10.95 |

Tables 12 and 13 provide the method used to analyze purity of the CSA-131 2 NDSA salt using liquid chromatography with charged aerosol detection (LC-CAD). This method can also be applied to other CSAs, including CSA-13.

**Table 12**

| | | | |
|---|---|---|---|
| **Column**: : Thermo Betasil Phenyl-Hexyl, 50 x 3.0 mm, 3 µm, Part# 73003-053030 | | | |
| **Diluent:** MeCN / H₂O / TFA (50 / 50 / 0.5) | | | |
| **Sample Concentration**: 1.0 mg/mL for CSA-13 Bis-NDSA | | | |
| **Mobile Phase** A: H₂O / 0.1%TFA | | **Mobile Phase B:** MeCN / MeOH / TFA (80 / 20 / 0.1) | |
| **Column Temperature:** 20 °C | | **Injection Volume:** 10 µL | |
| **Sample Temperature:** ambient | | **Detection:** CAD (Nebulizer: 25 □C; N₂: 35 psi) CAD (Model: ESA Corona, Part# 70-6186A) | |

| **Gradient Elution Table** | | | |
|---|---|---|---|
| Time (min) | A% | B% | Flow Rate (mL/min) |
| 0 | 90 | 10 | 1.0 |
| 10 | 54 | 46 | 1.0 |
| 18 | 54 | 46 | 1.0 |
| 20 | 20 | 80 | 1.0 |
| 22 | 20 | 80 | 1.0 |
| 22.1 | 90 | 10 | 1.0 |
| 27 | 90 | 10 | 1.0 |

**Table 13**

| Method | CSA-PHex6D | | |
|---|---|---|---|
| Column | Thermo Betasil Phenyl-hexyl 50x3 mm, 3µm | | |
| Column Temp. | 30°C | | |
| Detector | CAD | | |
| Mobile phase | A: H₂O, 0.1% TFA | | |
| | B: 80% MeCN, 20% MeOH, 0.1% TFA | | |
| Gradient | | A | B |
| | 0.00 min | 80 | 20 |
| | 10.00 min | 15 | 85 |
| | 20.00 min | 15 | 85 |
| | 22.00 min | 10 | 90 |
| | 23.00 min | 80 | 20 |
| | 26.00 min | 80 | 20 |
| Flow rate | 1.0 mL/min | | |

Surprisingly it was found that the formation of the di-NDSA salt can be used to provide significantly improved purity with less pure CSA-131 free base.

### CSA-44

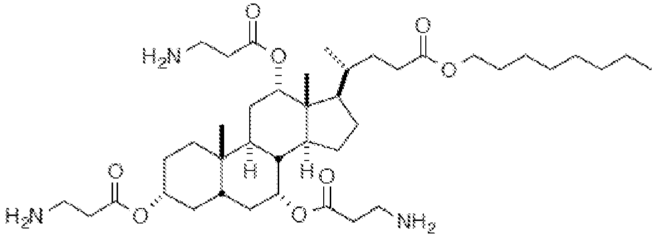

The free base of CSA-44 is obtained by neutralizing the hydrochloride salt as described in U.S. Patent No. 7,598,234.

### pKa Measurements of CSA-44

CSA-44 has three basic functional groups. pKa analysis was performed using the pH-metric method, with the sample being titrated in a triple titration from pH 2.0 to 12.0. CSA-44 pKa values were measured as 9.15 ± 0.06, 8.63 ± 0.09, and 7.75 ± 0.09.

### Solvent Solubility Test

Preliminary solubility tests were performed on the free base of CSA-44, reported in Table 14 below:

**Table 14**

| **Solvent** | **Approximate Solubility (mg/mL)** | **Observations** |
|---|---|---|
| Acetone | < 10 | Initial gum-like material converted to a white solid after 100 µL. After 100 vol., the mixture was cloudy. |
| Acetonitrile | < 10 | Initial gum-like material converted to a white solid after 100 µL. After 100 vol., the mixture was cloudy. |
| 1-Butanol | < 10 | Initial gum-like material converted to a white solid after 100 µL. After 100 vol., the mixture was cloudy. |
| Cyclohexane | *ca.*104 | Dissolution was observed. |
| Dichloromethane | *ca.*421 | Dissolution was observed. |
| Diisopropyl ether | < 10 | Initial gum-like material converted to a white solid after 100 µL. After 100 vol., the mixture was cloudy. |
| Dimethylformamide | *ca.*206 | Dissolution was observed. |
| Dimethylsulfoxide | *ca.*208 | Dissolution was observed. |
| 1,4-Dioxane | < 10 | Initial gum-like material converted to a white solid after 60 µL. After 100 vol., the mixture was cloudy. |
| Ethanol | < 10 | Initial gum-like material converted to a white solid after 100 µL. After 100 vol., the mixture was cloudy. |
| Ethyl acetate | < 10 | Initial gum-like material converted to a white solid after 100 µL. After 100 vol., the mixture was cloudy. |
| Heptane | < 10 | Dissolution was not observed. |
| Isopropyl acetate | < 10 | Initial gum-like material converted to a white solid after 100 µL. After 100 vol., the mixture was cloudy. |
| Methanol | < 10 | Initial gum-like material converted to a white solid after 100 µL. After 100 vol., the mixture was cloudy. |
| Methyl ethyl ketone | < 10 | Initial gum-like material converted to a white solid after 100 µL. After 100 vol., the mixture was cloudy. |
| Methyl isobutyl ketone | < 10 | Initial gum-like material converted to a white solid after 100 µL. After 100 vol., the mixture was cloudy. |
| *N*-Methyl-2-pyrrolidone | *ca.*107 | Dissolution was observed. |
| Nitromethane | < 10 | Initial gum-like material converted to a white solid after 50 µL. After 100 vol., the mixture was cloudy. |
| 2-Propanol | < 10 | Initial gum-like material converted to a white solid after 100 µL. After 100 vol., the mixture was cloudy. |
| *tert*-Butylmethyl ether | < 10 | Initial gum-like material converted to a white solid after 100 µL. After 100 vol., the mixture was cloudy. |
| Tetrahydrofuran | *ca.*215 | Dissolution was observed. |
| Toluene | *ca.*424 | Dissolution was observed. |
| Water | < 10 | Initial gum-like material converted to a white solid after 100 µL. After 100 vol., the mixture was cloudy. |
| Acetonitrile: Water (10%) | < 10 | Initial gum-like material converted to a white solid after 200 µL. After 100 vol., the mixture was cloudy. |

Solubility values were estimated by a solvent addition technique, based on the following protocol: CSA-44 (20 mg) was weighed and individually distributed to 24 vials. Each solvent was added to the appropriate vial in 10 aliquots of 10 µL, 5 aliquots of 20 µL, 3 aliquots of 100 µL, and 1 aliquot of 500 µL. If complete dissolution was observed, the additions were stopped. Between additions, the sample was stirred to further encourage dissolution. If 2000 µL of solvent was added without dissolution, the solubility was calculated to be below this point. Polarized light microscopy analysis was performed on solids obtained from acetone, acetonitrile, 1,4-dioxane, ethanol, ethyl acetate, and methanol.

Based upon the solubility, diversity, toxicity, and stability of CSA-44 in the preliminary solubility tests, the following ICH Class 2 solvents were selected for salt screening experiments: Acetonitrile: Water (10%), Cyclohexane, Tetrahydrofuran, and Toluene. Additionally, 2-Propanol and *tert*-Butylmethyl ether were also selected.

### Counterions for CSA-44 Salt Screening:

Counterions/acids for the proposed salt screening of CSA-44 were selected on the basis of the measured pKas of CSA-44, described above, and the likelihood of salt formation, which was estimated in part by a greater than about 2 pKa unit difference between the CSA pKA and the free acid pKa of the counterion. Table 15 below lists the counterions identified for preliminary salt screening experiments of CSA-44:

**Table 15**

| **Counterion/acid** | **Class** | **pKa 1** | **pKa 2** | **pKa 3** | **Equivalents** |
|---|---|---|---|---|---|
| Benzoic acid | 2 | 4.19 | - | - | 3 |
| Benzenesulphonic acid | 2 | 0.70 | - | - | 3 |
| Citric acid | 1 | 3.13 | 4.76 | 6.40 | 1 |
| Citric acid | 1 | 3.13 | 4.76 | 6.40 | 2 |
| Fumaric acid | 1 | 3.03 | 4.38 | - | 2 |
| Galactaric acid (Mucic Acid) | 1 | 3.08 | 3.63 | - | 2 |
| Hydrochloric acid | 1 | -6.10 | - | - | 2 |
| Hydrochloric acid | 1 | -6.10 | - | - | 3 |
| 1-Hydroxy-2-Naphthoic acid | 2 | 2.70 | 13.50 | - | 3 |
| L-Malic acid | 1 | 3.46 | 5.10 | - | 2 |
| 1,5-Naphthalenedisulfonic acid | 2 | -3.37 | -2.64 | - | 2 |
| Pamoic acid | 2 | 2.51 | 3.10 | - | 2 |
| Phosphoric acid | 1 | 1.96 | 7.12 | 12.32 | 3 |
| Succinic acid | 1 | 4.21 | 5.64 | - | 2 |
| Sulphuric acid | 1 | -3.00 | 1.92 | - | 2 |
| L-Tartaric acid | 1 | 3.02 | 4.36 | - | 2 |

Salt screening was carried out using the following protocol: CSA-44 (approximately 25 mg) was slurried or dissolved in the respective solvent, and then mixed with the appropriate equivalents of the acid counterion (specified in Table 15, above). The mixtures of CSA-44 / counterion / solvent were temperature cycled between 5° C and 25 C in four hour cycles for a period of approximately 48 hours. The following table summarizes the results of the primary salt screen:

**Table 16**

| **Counterion/acid** | **Equiv.** | **Solvent** | | | | | |
|---|---|---|---|---|---|---|---|
| | | **A** | **B** | **C** | **D** | **E** | **F** |
| Benzoic acid | 3 | Gum | AS | PSC | Gum | PSC* | PSC |
| Benzenesulphonic acid | 3 | Gum | Gum | PSC* | PSC- | PSC | Gum |
| Citric acid | 1 | Gum | Gum | AS | AS | PSC* | Gum |
| Citric acid | 2 | Gum | Gum | AS | AS | PSC* | Gel |
| Fumaric acid | 2 | AS | CC | AS | AS | Gum | CC |
| Mucic acid | 2 | AS | CC | CC | CC | CC | CC |
| Hydrochloric acid | 2 | Gum | Gum | Gum | Gum | Gum | Gum |
| Hydrochloric acid | 3 | Gum | Gum | Gum | PSC | Gum | Gum |
| L-Malic acid | 2 | Gum | PSC/ CC | Gum | AS | Gum | Gum |
| 1,5-Naphthalenedisul phonic acid | 2 | PSC* | PSC/ CC | PSC | PSC | PSC | PSC/ CC |
| Pamoic acid | 2 | CC | CC | CC | CC | Gum | CC |
| Succinic acid | 2 | Gum | CC | Gum | Gum | PSC* | Gum |
| 1-hydroxy-2-Naphthoic acid | 3 | Gum | FB | Gel | PSC* | Gum | PSC* |
| Phosphoric acid | 3 | PSC* | PSC* | PSC | Gum | PSC* | Gum |
| Sulphuric acid | 2 | Gum | PSC | PSC* | Gum | PSC* | Gum |
| L-Tartaric acid | 2 | Gum | PSC | PSC | PSC | PSC | Gel |
| L-Aspartic acid | 3 | Gum | CC | CC | CC | CC | CC |
| L-Arginine | 3 | CC | CC | CC | CC | CC | CC |
| L-tyrosine | 3 | CC | CC | CC | CC | CC | CC |
| Meglumine | 3 | CC | CC | CC | CC | CC | CC |
| Proline | 3 | Gum | PSC/ CC | PSC/ CC | PSC/ CC | PSC/ CC | Gum |
| Urea | 3 | Gum | Gum | CC | Gum | PSC/ CC | Gum |
| L-Glycine | 3 | CC/PSC* | CC/PSC* | CC/PSC* | PSC/ CC | CC/PSC* | PSC/ CC |
| Tromethamine | 3 | CC | CC | CC | CC | CC | CC |

In Table 16, solvents A-F were as follows: (A) Acetonitrile:water (10%); (B) cyclohezane; (C) 2-propanol; (D) TBME; (E) THF; and (F) toluene. Characterization of the resultant material from the primary screen was as follows: Gum; AS ("amorphous solid"); PSC ("potential salt/co-crystal"); PSC* ("potential salt/co-crystal" obtained with anti-solvent addition); PSC- ("potential salt/co-crystal" obtained by evaporation of solvent); Gel; CC ("counterion/co-former"); and FB ("free base").

According to the primary salt screen and provided data, certain samples indicated signs of co-crystal formation. Additional experiments of these samples were performed in which the number of equivalents was reduced from 3 mol to 2 mol and the same salt screening procedure was followed. Isolated material was in the form of a mixture of gum and crystalline solid, with PXRD analysis showing a mixture of PSC and CC.

Salt screening was also performed using 150 mg of CSA-44, finding that flowable solids could be obtained if material was isolated upon precipitation and without temperature cycling. For experiments resulting in the preparation of thin slurries, it was also found that anti-solvent addition would improve the yield. Amorphous solids were obtained from the following counterions, equivalents, and solvents: Benzoic acid, 3 equivalents, THF; 1,5-napthalenedisulphonic acid, 2 equivalents, 2-propanol; succinic acid, 2 equivalents, THF; phosphoric acid, 3 equivalents, THF; sulfuric acid, 2 equivalents, TBME; and L-tartaric acid, 2 equivalents, THF. Preliminary results suggested that crystalline material was obtained from the following counterions, equivalents, and solvents: benzenesulfonic acid, 3 equivalents, 2-propanol or THF; and hydrochloric acid, 3 equivalents, TBME. These experiments surprisingly indicated that 1,5-napthalenedisulphonic acid provided favorable properties such as a stable, flowable solid (from visual inspection).

To improve crystallinity, amorphous and crystalline solids obtained from the above-described screen were slurried in solvents such as 1,4-dioxane, dichloromethane, methanol, ethyl acetate, diisopropyl ether, and acetonitrile. The results of this experiment are summarized in Table 17:

**Table 17**

| **Counterion/Acid** | **1,4-D** | **DCM** | **M** | **EA** | **DIE** | **ACET** |
|---|---|---|---|---|---|---|
| Benzoic acid | C | C | A | C | A | CS |
| Benzenesulfonic acid | C | C | C | C | CS | CS |
| Benzenesulfonic acid | C | CS | C | C | C | C |
| Hydrochloric acid | CS | C | C | C | C | CS |
| 1,5-Naphthalenedisuolfonic acid | A | A | C | A | A | A |
| Succinic acid | CS | CS | CS | A | A | A |
| Phosphoric acid | CS | A | A | A | A | CS |
| Sulfuric acid | CS | A | CS | CS | CS | CS |
| L-Tartaric acid | A | A | A | A | A | A |

In Table 17, 1,4-D stands for "1,4-Dioxane"; DCM stands for "Dichloromethane"; M stands for "Methanol"; EA stands for "Ethyl Acetate"; DIE stands for "Diisopropyl ether"; ACET stands for "Acetonitrole"; C stands for "crystalline"; A stands for "amorphous"; and CS stands for "clear solution." Although a number of results indicated the formation of crystalline material, 1,5-naphthalenedisulfonic acid appeared to provide the most flowable solid after isolation. Potential salts from benzoic acid showed an improvement in crystallinity in 1,4-dioxane, dichloromethane, and ethyl acetate, but became gum-like upon isolation. Similar results were observed for benzenesulfonic acid and hydrochloric acid.

## Claims

1. A 1,5-naphthalenedisulfonic acid di-addition salt of a cationic steroidal antimicrobial (CSA), wherein the CSA is a compound of Formula III: 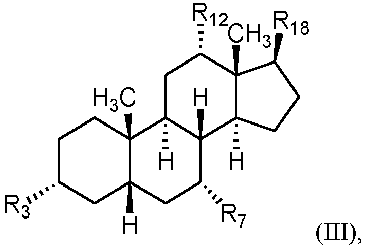 wherein,
R₃, R₇, and R₁₂, are independently selected from the group consisting of hydrogen, hydroxyl, (C₁-C₂₂) alkyl, (C₁-C₂₂) hydroxyalkyl, (C₁-C₂₂) alkyloxy-(C₁-C₂₂) alkyl, (C₁-C₂₂) alkylcarboxy-(C₁-C₂₂) alkyl, (C₁-C₂₂) alkylamino-(C₁-C₂₂)alkyl, (C₁-C₂₂) alkylamino-(C₁-C₂₂) alkylamino, (C₁-C₂₂) alkylamino-(C₁-C₂₂) alkylamino- (C₁-C₂₂) alkylamino, (C₁-C₂₂) aminoalkyl, aryl, arylamino-(C₁-C₂₂) alkyl, (C₁-C₂₂) haloalkyl, C₂-C₆ alkenyl, C₂-C₆ alkynyl, oxo, a linking group attached to a second steroid, (C₁-C₂₂) aminoalkyloxy, (C₁-C₂₂) aminoalkyloxy-(C₁-C₂₂) alkyl, (C₁-C₂₂) aminoalkylcarboxy, (C₁-C₂₂) aminoalkylaminocarbonyl, (C₁-C₂₂) aminoalkylcarboxamido, di(C₁-C₂₂ alkyl)aminoalkyl, H₂N-HC(Q₅)-C(O)-O-, H₂N-HC(Q₅)-C(O)-N(H)-, (C₁-C₂₂) azidoalkyloxy, (C₁-C₂₂) cyanoalkyloxy, P.G.-HN-HC(Q₅)-C(O)-O-, (C₁-C₂₂) guanidinoalkyloxy, (C₁-C₂₂) quaternary ammonium alkylcarboxy, and (C₁-C₂₂) guanidinoalkyl carboxy, where Q₅ is a side chain of any amino acid (including a side chain of glycine, i.e., H), and P.G. is an amino protecting group; and
R₁₈ is selected from the group consisting of hydrogen, hydroxyl, (C₁-C₂₂) alkyl, (C₁-C₂₂) hydroxyalkyl, (C₁-C₂₂) alkyloxy-(C₁-C₂₂) alkyl, (C₁-C₂₂) alkylcarboxy-(C₁-C₂₂) alkyl, (C₁-C₂₂) alkylamino-(C₁-C₂₂)alkyl, (C₁-C₂₂) alkylamino-(C₁-C₂₂) alkylamino, (C₁-C₂₂) alkylamino-(C₁-C₂₂) alkylamino- (C₁-C₂₂) alkylamino, (C₁-C₂₂) aminoalkyl, aryl, arylamino-(C₁-C₂₂) alkyl, (C₁-C₂₂) haloalkyl, C₂-C₆ alkenyl, C₂-C₆ alkynyl, oxo, a linking group attached to a second steroid, (C₁-C₂₂) aminoalkyloxy, (C₁-C₂₂) aminoalkyloxy-(C₁-C₂₂) alkyl, (C₁-C₂₂) aminoalkylcarboxy, (C₁-C₂₂) aminoalkylaminocarbonyl, (C₁-C₂₂) aminoalkyl-carboxamido, di(C₁-C₂₂ alkyl)aminoalkyl, H₂N-HC(Q₅)-C(O)-O-, H₂N-HC(Q₅)-C(O)-N(H)-, (C₁-C₂₂) azidoalkyloxy, (C₁-C₂₂) cyanoalkyloxy, P.G.-HN-HC(Q₅)-C(O)-O-, (C₁-C₂₂) guanidinoalkyloxy, (C₁-C₂₂) quaternary ammonium alkylcarboxy, (C₁-C₂₂) guanidinoalkyl carboxy, and a group having amide functionality in which the carbonyl group of the amide is positioned between the amido nitrogen of the amide and fused ring D of the steroidal backbone, where Q₅ is a side chain of any amino acid (including a side chain of glycine, i.e., H), and P.G. is an amino protecting group,
provided that at least two or three of R₃, R₇, R₁₂, and R₁₈ are independently selected from the group consisting of (C₁-C₂₂) aminoalkyl, (C₁-C₂₂) aminoalkyloxy, (C₁-C₂₂) alkylcarboxy-(Ci-C₂₂) alkyl, (C₁-C₂₂) alkylamino-(C₁-C₂₂) alkylamino, (C₁-C₂₂) alkylamino-(C₁-C₂₂) alkylamino (C₁-C₂₂) alkylamino, (C₁-C₂₂) aminoalkylcarboxy, arylamino (C₁-C₂₂) alkyl, (C₁-C₂₂) aminoalkyloxy (C₁-C₂₂) aminoalkylaminocarbonyl, (C₁-C₂₂) aminoalkylaminocarbonyl, (C₁-C₂₂) aminoalkylcarboxyamido, (C₁-C₂₂) quaternary ammonium alkylcarboxy, di(C₁-C₂₂ alkyl)aminoalkyl, H₂N-HC(Q₅)-C(O)-O-, H₂N-HC(Q₅)-C(O)-N(H)-, (C₁-C₂₂) azidoalkyloxy, (C₁-C₂₂) cyanoalkyloxy, P.G.-HN-HC(Q₅)-C(O)-O-, (C₁-C₂₂) guanidinoalkyloxy, and (C₁-C₂₂) guanidinoalkylcarboxy.

2. The salt of claim 1, wherein R₁₈ has the following structure:
-R₂₀-(C=O)-N-R₂₁R₂₂
wherein,
R₂₀ is omitted or a substituted or unsubstituted alkyl, alkenyl, alkynyl, or aryl; and
R₂₁ and R₂₂ are independently selected from the group consisting of hydrogen, a substituted or unsubstituted alkyl, a substituted or unsubstituted alkenyl, a substituted or unsubstituted alkynyl, or a substituted or unsubstituted aryl, such as where R₂₁ and R₂₂ are independently selected from the group consisting of hydrogen, optionally substituted C₁-C₂₄ alkyl, optionally substituted C₂-C₂₄ alkenyl, optionally substituted C₂-C₂₄ alkynyl, optionally substituted C₆ or C₁₀ aryl, optionally substituted 5 to 10 membered heteroaryl, optionally substituted 5 to 10 membered heterocyclyl, optionally substituted C₇₋₁₃ aralkyl, optionally substituted (5 to 10 membered heteroaryl)-C₁-C₆ alkyl, optionally substituted C₃₋₁₀ carbocyclyl, optionally substituted C₄₋₁₀ (carbocyclyl)alkyl, optionally substituted (5 to 10 membered heterocyclyl)-C₁-C₆ alkyl, optionally substituted amido, and a suitable amine protecting group, provided that at least one of R₂₁ and R₂₂ is not hydrogen, and optionally wherein R₂₁ and R₂₂, together with the atoms to which they are attached, form an optionally substituted 5 to 10 membered heterocyclyl ring.

3. The salt of claim 1 or 2, wherein
R₃, R₇, and R₁₂, are independently selected from the group consisting of hydrogen, an unsubstituted (C₁-C₁₈) alkyl, unsubstituted (C₁-C₁₈) hydroxyalkyl, unsubstituted (C₁-C₁₈) alkyloxy-(C₁-C₁₈) alkyl, unsubstituted (C₁-C₁₈) alkylcarboxy-(C₁-C₁₈) alkyl, unsubstituted (C₁-C₁₈) alkylamino-(C₁-C₁₈)alkyl, unsubstituted (C₁-C₁₈) alkylamino-(C₁-C₁₈) alkylamino, unsubstituted (C₁-C₁₈) alkylamino-(C₁-C₁₈) alkylamino- (C₁-C₁₈) alkylamino, an unsubstituted (C₁-C₁₈) aminoalkyl, an unsubstituted arylamino-(C₁-C₁₈) alkyl, an unsubstituted (C₁-C₁₈) aminoalkyloxy, an unsubstituted (C₁-C₁₈) aminoalkyloxy-(C₁-C₁₈) alkyl, an unsubstituted (C₁-C₁₈) aminoalkylcarboxy, an unsubstituted (C₁-C₁₈) aminoalkylaminocarbonyl, an unsubstituted (C₁-C₁₈) aminoalkylcarboxamido, an unsubstituted di(C₁-C₁₈ alkyl)aminoalkyl, unsubstituted (C₁-C₁₈) guanidinoalkyloxy, unsubstituted (C₁-C₁₈) quaternary ammonium alkylcarboxy, and unsubstituted (C₁-C₁₈) guanidinoalkyl carboxy; and
R₁₈ is independently selected from the group consisting of hydrogen, an unsubstituted (C₁-C₁₈) alkyl, unsubstituted (C₁-C₁₈) hydroxyalkyl, unsubstituted (C₁-C₁₈) alkyloxy-(C₁-C₁₈) alkyl, unsubstituted (C₁-C₁₈) alkylcarboxy-(C₁-C₁₈) alkyl, unsubstituted (C₁-C₁₈) alkylamino-(C₁-C₁₈)alkyl, unsubstituted (C₁-C₁₈) alkylamino-(C₁-C₁₈) alkylamino, unsubstituted (C₁-C₁₈) alkylamino-(C₁-C₁₈) alkylamino- (C₁-C₁₈) alkylamino, an unsubstituted (C₁-C₁₈) aminoalkyl, an unsubstituted arylamino-(C₁-C₁₈) alkyl, an unsubstituted (C₁-C₁₈) aminoalkyloxy, an unsubstituted (C₁-C₁₈) aminoalkyloxy-(C₁-C₁₈) alkyl, an unsubstituted (C₁-C₁₈) aminoalkylcarboxy, an unsubstituted (C₁-C₁₈) aminoalkylaminocarbonyl, an unsubstituted (C₁-C₁₈) aminoalkylcarboxamido, an unsubstituted di(C₁-C₁₈ alkyl)aminoalkyl, unsubstituted (C₁-C₁₈) guanidinoalkyloxy, unsubstituted (C₁-C₁₈) quaternary ammonium alkylcarboxy, unsubstituted (C₁-C₁₈) guanidinoalkyl carboxy, and a group having amide functionality in which the carbonyl group of the amide is positioned between the amido nitrogen of the amide and fused ring D of the steroidal backbone.

4. The salt of any of claims 1-3, wherein,
R₃, R₇, and R₁₂ are independently selected from the group consisting of hydrogen, an unsubstituted (C₁-C₆) alkyl, unsubstituted (C₁-C₆) hydroxyalkyl, unsubstituted (C₁-C₁₆) alkyloxy-(C₁-C₅) alkyl, unsubstituted (C₁-C₁₆) alkylcarboxy-(C₁-C₅) alkyl, unsubstituted (C₁-C₁₆) alkylamino-(C₁-C₅)alkyl, (C₁-C₁₆) alkylamino-(C₁-C₅) alkylamino, unsubstituted (C₁-C₁₆) alkylamino-(C₁-C₁₆) alkylamino-(C₁-C₅) alkylamino, an unsubstituted (C₁-C₁₆) aminoalkyl, an unsubstituted arylamino-(C₁-C₅) alkyl, an unsubstituted (C₁-C₅) aminoalkyloxy, an unsubstituted (C₁-C₁₆) aminoalkyloxy-(C₁-C₅) alkyl, an unsubstituted (C₁-C₅) aminoalkylcarboxy, an unsubstituted (C₁-C₅) aminoalkylaminocarbonyl, an unsubstituted (C₁-C₅) aminoalkylcarboxamido, an unsubstituted di(C₁-C₅ alkyl)amino-(C₁-C₅) alkyl, unsubstituted (C₁-C₅) guanidinoalkyloxy, unsubstituted (C₁-C₁₆) quaternary ammonium alkylcarboxy, and unsubstituted (C₁-C₁₆) guanidinoalkylcarboxy; and
R₁₈ is independently selected from the group consisting of hydrogen, an unsubstituted (C₁-C₆) alkyl, unsubstituted (C₁-C₆) hydroxyalkyl, unsubstituted (C₁-C₁₆) alkyloxy-(C₁-C₅) alkyl, unsubstituted (C₁-C₁₆) alkylcarboxy-(C₁-C₅) alkyl, unsubstituted (C₁-C₁₆) alkylamino-(Ci-Cs)alkyl, (C₁-C₁₆) alkylamino-(C₁-C₅) alkylamino, unsubstituted (C₁-C₁₆) alkylamino-(C₁-C₁₆) alkylamino-(C₁-C₅) alkylamino, an unsubstituted (C₁-C₁₆) aminoalkyl, an unsubstituted arylamino-(C₁-C₅) alkyl, an unsubstituted (C₁-C₅) aminoalkyloxy, an unsubstituted (C₁-C₁₆) aminoalkyloxy-(C₁-C₅) alkyl, an unsubstituted (C₁-C₅) aminoalkylcarboxy, an unsubstituted (C₁-C₅) aminoalkylaminocarbonyl, an unsubstituted (C₁-C₅) aminoalkylcarboxamido, an unsubstituted di(C₁-C₅ alkyl)amino-(C₁-C₅) alkyl, unsubstituted (C₁-C₅) guanidinoalkyloxy, unsubstituted (C₁-C₁₆) quaternary ammonium alkylcarboxy, unsubstituted (C₁-C₁₆) guanidinoalkylcarboxy, and a group having amide functionality in which the carbonyl group of the amide is positioned between the amido nitrogen of the amide and fused ring of the steroidal backbone.

5. The salt of any of claims 1-4, wherein
R₃, R₇, and R₁₂ are independently selected from the group consisting of aminoalkyloxy; aminoalkylcarboxy; alkylaminoalkyl; alkoxycarbonylalkyl; alkylcarbonylalkyl; di(alkyl)aminoalkyl; alkylcarboxyalkyl; and hydroxyalkyl, such as wherein R₃, R₇, and R₁₂ are independently selected from the group consisting of aminoalkyloxy and aminoalkylcarboxy, preferably where R₃, R₇, and R₁₂ are the same; and
R₁₈ is independently selected from the group consisting of aminoalkyloxy; aminoalkylcarboxy; alkylaminoalkyl; alkoxycarbonylalkyl; alkylcarbonylalkyl; di(alkyl)aminoalkyl; alkylcarboxyalkyl; hydroxyalkyl, and a group having amide functionality in which the carbonyl group of the amide is positioned between the amido nitrogen of the amide and fused ring D of the steroidal backbone, as wherein R₁₈ is alkylaminoalkyl or alkoxycarbonylalkyl.

6. The salt of any one of claims 1-5, wherein
R₃, R₇, and R₁₂ are independently selected from the group consisting of amino-C₃-alkyloxy; amino-C₃-alkyl-carboxy; C₈-alkylamino-C₅-alkyl; C₁₂-alkylamino-C₅-alkyl; C₁₃-alkylamino-C₅-alkyl; C₁₆-alkylamino-C₅-alkyl; di-(C₅-alkyl)amino-C₅-alkyl; C₆-alkoxy-carbonyl-C₄-alkyl; C₈-alkoxy-carbonyl-C₄-alkyl; C₁₀-alkoxy-carbonyl-C₄-alkyl; C₆-alkyl-carboxy-C₄-alkyl; C₈-alkylcarboxy-C₄-alkyl; and C₁₀-alkyl-carboxy-C₄-alkyl; and
preferably wherein R₃, R₇, and R₁₂ are independently selected from the group consisting of amino-C₃-alkyloxy; amino-C₃-alkyl-carboxy; C₈-alkylamino-C₅-alkyl; C₁₂-alkylamino-Cs-alkyl; C₁₃-alkylamino-C₅-alkyl; C₁₆-alkylamino-C₅-alkyl; di-(C₅-alkyl)amino-C₅-alkyl; C₆-alkoxy-carbonyl-C₄-alkyl; C₈-alkoxy-carbonyl-C₄-alkyl; and C₁₀-alkoxy-carbonyl-C₄-alkyl,
more preferably wherein wherein R₃, R₇, and R₁₂ are independently selected from the group consisting of amino-C₃-alkyloxy or amino-C₃-alkyl-carboxy; and
R₁₈ is independently selected from the group consisting of amino-C₃-alkyloxy; amino-C₃-alkyl-carboxy; C₈-alkylamino-C₅-alkyl; C₁₂-alkylamino-C₅-alkyl; C₁₃-alkylamino-C₅-alkyl; C₁₆-alkylamino-Cs-alkyl; di-(C₅-alkyl)amino-C₅-alkyl; C₆-alkoxy-carbonyl-C₄-alkyl; C₈-alkoxy-carbonyl-C₄-alkyl; C₁₀-alkoxy-carbonyl-C₄-alkyl; C₆-alkyl-carboxy-C₄-alkyl; C₈-alkyl-carboxy-C₄-alkyl; C10-alkyl-carboxy-C₄-alkyl; and a group having amide functionality in which the carbonyl group of the amide is positioned between the amido nitrogen of the amide and fused ring D of the steroidal backbone,
preferably wherein R₁₈ is independently selected from the group consisting of amino-C₃-alkyloxy; amino-C₃-alkyl-carboxy; C₈-alkylamino-C₅-alkyl; C₁₂-alkylamino-C₅-alkyl; C₁₃-alkylamino-C₅-alkyl; C₁₆-alkylamino-C₅-alkyl; di-(C₅-alkyl)amino-C₅-alkyl; C₆-alkoxy-carbonyl-C₄-alkyl; C₈-alkoxy-carbonyl-C₄-alkyl; C₁₀-alkoxy-carbonyl-C₄-alkyl; and a group having amide functionality in which the carbonyl group of the amide is positioned between the amido nitrogen of the amide and fused ring D of the steroidal backbone,
more preferably wherein R₁₈ is selected from the group consisting of C₈-alkylamino-Cs-alkyl or C₈-alkoxy-carbonyl-C₄-alkyl.

7. The salt of any one of Claims 1-6, wherein the CSA is selected from the group consisting of: 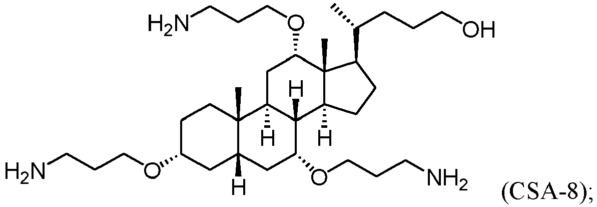 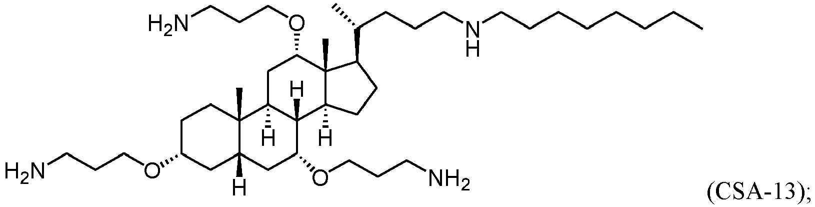 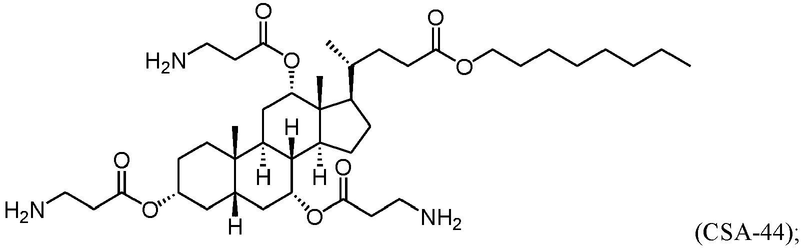 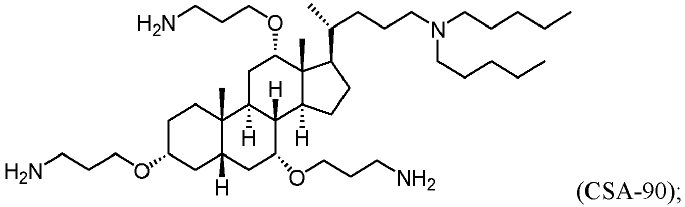 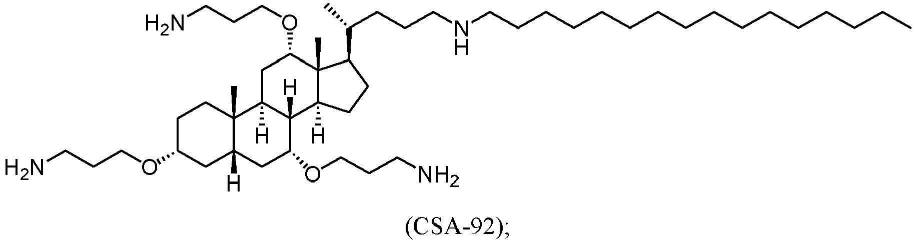 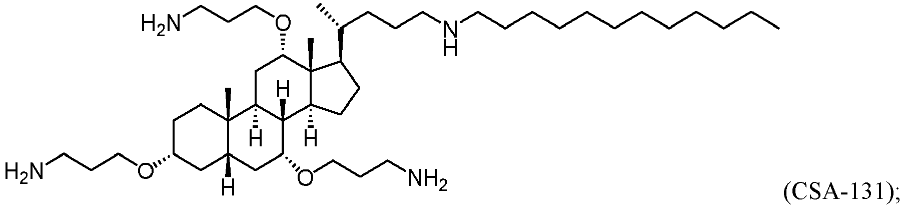 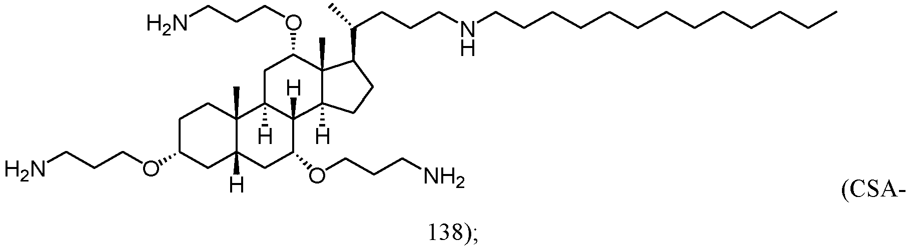 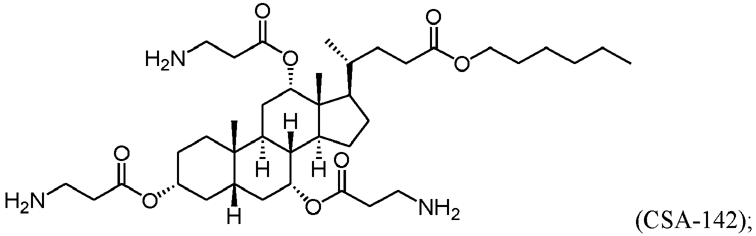 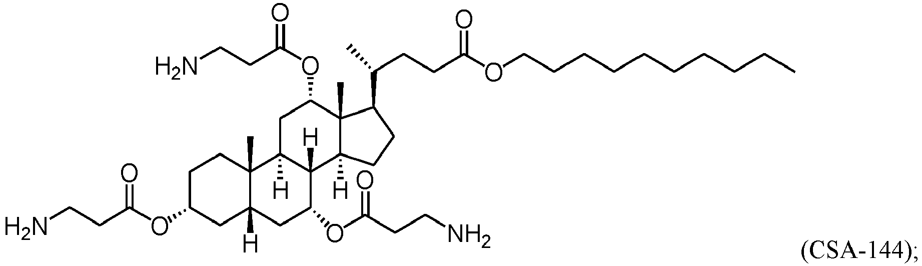 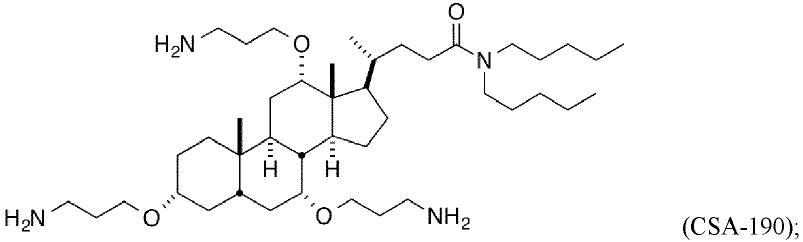 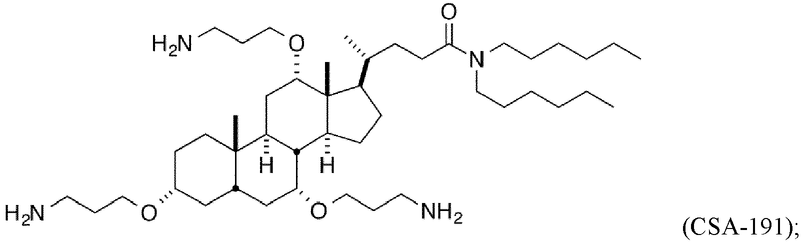 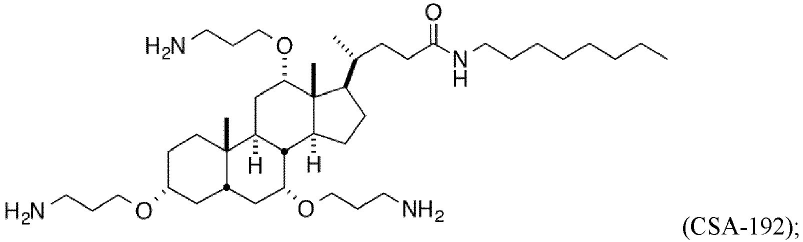 and

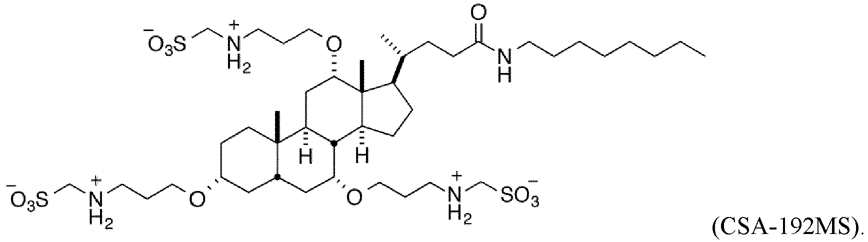

8. The salt of any one of claims 1-7, wherein the salt is a solid, such as a flowable solid, preferably crystalline and/or wherein the salt is storage stable and/or micronized.

9. The salt of any one of claims 1-8, wherein the salt is a 1,5-naphthalenedisulfonic acid di-addition salt of CSA-13 and is **characterized by**:
an x-ray powder diffraction pattern with the following 2θ values (± 0.2): 4.216; 4.629; 8.29; 9.13; 9.739; 12.641; 14.457; 15.864; 18.610; 19.200; 20.242; 20.803; 21.512; 22.014; 22.57; 23.169; 23.63; 25.227; 26.44; 37.05; and 39.33,
an x-ray powder diffraction pattern with the following 2θ values (± 0.2): 4.200; 4.606; 8.292; 9.113; 9.728; 11.71; 12.625; 13.95; 14.444; 15.826; 18.622; 19.20; 20.22; 20.767; 21.482; 21.958; 22.53; 23.12; 23.61; 25.26; 26.55; and 37.01, or
wherein the salt is a 1,5-naphthalenedisulfonic acid di-addition salt of CSA-131 and is **characterized by**:
an x-ray powder diffraction pattern with the following 2θ values (± 0.2): 4.1922; 4.4257, 6.118, 8.3931, 9.6769, 11.7232, 13.4959, 15.0514, 16.5064, 17.8322, 18.7671, 19.3449, 20.596, 21.5538, 22.7706, 24.6057, 26.7689, and 36.2048.

10. A formulation, comprising: a salt of any one of claims 1-8 and a pharmaceutically acceptable excipient.

11. A process for preparing the salt of any one of Claim 1-10, comprising:
diluting a starting free base of a CSA with a solvent;
adding at least one equivalent of an acid to the diluted CSA in solvent to afford a reaction mixture;
precipitating or temperature cycling the reaction mixture, such as temperature cycling for at least about 48 hours; and
isolating a CSA salt.

12. The process of claim 11, further comprising utilizing an anti-solvent or evaporation of solvent when isolating the CSA salt.

13. The process of claim 11 or 12, wherein the CSA salt is a solid, such as where the CSA salt is crystalline or amorphous, storage stable, flowable, and/or micronized.

## Patentansprüche

1. 1,5-Naphthalindisulfonsäure-Diadditionssalz eines kationischen steroidalen antimikrobiellen Mittels (CSA), wobei das CSA eine Verbindung der Formel III ist: 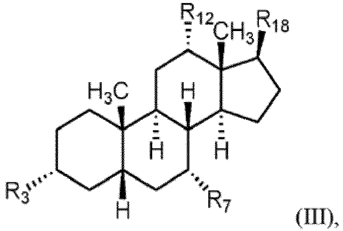 wobei
R₃, R₇ und R₁₂ unabhängig voneinander ausgewählt sind aus der Gruppe, bestehend aus Wasserstoff, Hydroxyl, (C₁-C₂₂)-Alkyl, (C₁-C₂₂)-Hydroxyalkyl, (C₁-C₂₂)-Alkyloxy-(C₁-C₂₂)-alkyl, (C₁-C₂₂)-Alkylcarboxy-(C₁-C₂₂)-alkyl, (C₁-C₂₂)-Alkylamino-(C₁-C₂₂)-alkyl, (C₁-C₂₂)-Alkylamino-(C₁-C₂₂)-alkylamino, (C₁-C₂₂)-Alkylamino-(C₁-C₂₂)-alkylamino-(C₁-C₂₂)-alkylamino, (C₁-C₂₂)-Aminoalkyl, Aryl, Arylamino-(C₁-C₂₂)-alkyl, (C₁-C₂₂)-Halogenalkyl, C₂-C₆-Alkenyl, C₂-C₆-Alkinyl, Oxo, einer an ein zweites Steroid gebundenen Verknüpfungsgruppe, (C₁-C₂₂)-Aminoalkyloxy, (C₁-C₂₂)-Aminoalkyloxy-(C₁-C₂₂)-alkyl, (C₁-C₂₂)-Aminoalkylcarboxy, (C₁-C₂₂)-Aminoalkylaminocarbonyl, (C₁-C₂₂)-Aminoalkylcarboxamido, Di(C₁-C₂₂-alkyl)aminoalkyl, H₂N-HC(Q₅)-C(O)-O-, H₂N-HC(Q₅)-C(O)-N(H)-, (C₁-C₂₂)-Azidoalkyloxy, (C₁-C₂₂)-Cyanoalkyloxy, P.G.-HN-HC(Q₅)-C(O)-O-, (C₁-C₂₂)-Guanidinoalkyloxy, quaternärem (C₁-C₂₂)-Ammoniumalkylcarboxy und (C₁-C₂₂)-Guanidinoalkylcarboxy, wobei Q₅ eine Seitenkette einer beliebigen Aminosäure (einschließlich einer Seitenkette von Glycin, d.h., H) ist und P.G. eine Aminoschutzgruppe ist; und
R₁₈ ausgewählt ist aus der Gruppe, bestehend aus Wasserstoff, Hydroxyl, (C₁-C₂₂)-Alkyl, (C₁-C₂₂)-Hydroxyalkyl, (C₁-C₂₂)-Alkyloxy-(C₁-C₂₂)-alkyl, (C₁-C₂₂)-Alkylcarboxy-(C₁-C₂₂)-alkyl, (C₁-C₂₂)-Alkylamino-(C₁-C₂₂)-alkyl, (C₁-C₂₂)-Alkylamino-(C₁-C₂₂)-alkylamino, (C₁-C₂₂)-Alkylamino-(C₁-C₂₂)-alkylamino-(C₁-C₂₂)-alkylamino, (C₁-C₂₂)-Aminoalkyl, Aryl, Arylamino-(C₁-C₂₂)-alkyl, (C₁-C₂₂)-Halogenalkyl, C₂-C₆-Alkenyl, C₂-C₆-Alkinyl, Oxo, einer an ein zweites Steroid gebundenen Verknüpfungsgruppe, (C₁-C₂₂)-Aminoalkyloxy, (C₁-C₂₂)-Aminoalkyloxy-(C₁-C₂₂)-alkyl, (C₁-C₂₂)-Aminoalkylcarboxy, (C₁-C₂₂)-Aminoalkylaminocarbonyl, (C₁-C₂₂)-Aminoalkylcarboxamido, Di(C₁-C₂₂-alkyl)-aminoalkyl, H₂N-HC(Q₅)-C(O)-O-, H₂N-HC(Q₅)-C(O)-N(H)-, (C₁-C₂₂)-Azidoalkyloxy, (C₁-C₂₂)-Cyanoalkyloxy, P.G.-HN-HC(Q₅)-C(O)-O-, (C₁-C₂₂)-Guanidinoalkyloxy, quaternärem (C₁-C₂₂)-Ammoniumalkylcarboxy, (C₁-C₂₂)-Guanidinoalkylcarboxy und einer Gruppe mit Amidfunktionalität, in der die Carbonylgruppe des Amids zwischen dem Amidostickstoff des Amids und dem kondensierten Ring D des Steroid-Grundgerüsts positioniert ist, wobei Q₅ eine Seitenkette einer beliebigen Aminosäure (einschließlich einer Seitenkette von Glycin, d.h., H) ist und P.G. eine Aminoschutzgruppe ist,
vorausgesetzt, dass mindestens zwei oder drei von R₃, R₇, R₁₂ und R₁₈ unabhängig voneinander ausgewählt sind aus der Gruppe, bestehend aus (C₁-C₂₂)-Aminoalkyl, (C₁-C₂₂)-Aminoalkyloxy, (C₁-C₂₂)-Alkylcarboxy-(C₁-C₂₂)-alkyl, (C₁-C₂₂)-Alkylamino-(C₁-C₂₂)-alkylamino, (C₁-C₂₂)-Alkylamino-(C₁-C₂₂)-alkylamino-(C₁-C₂₂)-alkylamino, (C₁-C₂₂)-Aminoalkylcarboxy, Arylamino-(C₁-C₂₂)-alkyl, (C₁-C₂₂)-Aminoalkyloxy-(C₁-C₂₂)-aminoalkylaminocarbonyl, (C₁-C₂₂)-Aminoalkylaminocarbonyl, (C₁-C₂₂)-Aminoalkylcarboxyamido, quaternärem (C₁-C₂₂)-Ammoniumalkylcarboxy, Di(C₁-C₂₂-alkyl)aminoalkyl, H₂N-HC(Q₅)-C(O)-O-, H₂N-HC(Q₅)-C(O)-N(H)-, (C₁-C₂₂)-Azidoalkyloxy, (C₁-C₂₂)-Cyanoalkyloxy, P.G.-HN-HC(Q₅)-C(O)-O-, (C₁-C₂₂)-Guanidinoalkyloxy und (C₁-C₂₂)-Guanidinoalkylcarboxy.

2. Salz nach Anspruch 1, wobei R₁₈ die folgende Struktur aufweist:
-R₂₀-(C=O)-N-R₂₁R₂₂
wobei
R₂₀ weggelassen ist oder ein substituiertes oder unsubstituiertes Alkyl, Alkenyl, Alkinyl oder Aryl ist; und
R₂₁ und R₂₂ unabhängig voneinander ausgewählt sind aus der Gruppe, bestehend aus Wasserstoff, einem substituierten oder unsubstituierten Alkyl, einem substituierten oder unsubstituierten Alkenyl, einem substituierten oder unsubstituierten Alkinyl oder einem substituierten oder unsubstituierten Aryl, z.B. wobei R₂₁ und R₂₂ unabhängig voneinander ausgewählt sind aus der Gruppe, bestehend aus Wasserstoff, gegebenenfalls substituiertem C₁-C₂₄-Alkyl, gegebenenfalls substituiertem C₂-C₂₄-Alkenyl, gegebenenfalls substituiertem C₂-C₂₄-Alkinyl, gegebenenfalls substituiertem C₆- oder C₁₀-Aryl, gegebenenfalls substituiertem 5- bis 10-gliedrigem Heteroaryl, gegebenenfalls substituiertem 5- bis 10-gliedrigem Heterocyclyl, gegebenenfalls substituiertem C₇₋₁₃-Aralkyl, gegebenenfalls substituiertem (5-bis 10-gliedrigem Heteroaryl)-C₁-C₆-alkyl, gegebenenfalls substituiertem C₃₋₁₀-Carbocyclyl, gegebenenfalls substituiertem C₄₋₁₀-(Carbocyclyl)alkyl, gegebenenfalls substituiertem (5- bis 10-gliedrigem Heterocyclyl)-C₁-C₆-alkyl, gegebenenfalls substituiertem Amido und einer geeigneten Aminschutzgruppe, vorausgesetzt, dass mindestens eines von R₂₁ und R₂₂ nicht Wasserstoff ist, und wobei R₂₁ und R₂₂ gegebenenfalls zusammen mit den Atomen, an die sie gebunden sind, einen gegebenenfalls substituierten 5- bis 10-gliedrigen Heterocyclylring bilden.

3. Salz nach Anspruch 1 oder 2, wobei
R₃, R₇ und R₁₂ unabhängig voneinander ausgewählt sind aus der Gruppe, bestehend aus Wasserstoff, einem unsubstituierten (C₁-C₁₈)-Alkyl, unsubstituiertem (C₁-C₁₈)-Hydroxyalkyl, unsubstituiertem (C₁-C₁₈)-Alkyloxy-(C₁-C₁₈)-alkyl, unsubstituiertem (C₁-C₁₈)-Alkylcarboxy-(C₁-C₁₈)-alkyl, unsubstituiertem (C₁-C₁₈)-Alkylamino-(C₁-C₁₈)-alkyl, unsubstituiertem (C₁-C₁₈)-Alkylamino-(C₁-C₁₈)-alkylamino, unsubstituiertem (C₁-C₁₈)-Alkylamino-(C₁-C₁₈)-alkylamino-(C₁-C₁₈)-alkylamino, einem unsubstituierten (C₁-C₁₈)-Aminoalkyl, einem unsubstituierten Arylamino-(C₁-C₁₈)-alkyl, einem unsubstituierten (C₁-C₁₈)-Aminoalkyloxy, einem unsubstituierten (C₁-C₁₈)-Aminoalkyloxy-(C₁-C₁₈)-alkyl, einem unsubstituierten (C₁-C₁₈)-Aminoalkylcarboxy, einem unsubstituierten (C₁-C₁₈)-Aminoalkylaminocarbonyl, einem unsubstituierten (C₁-C₁₈)-Aminoalkylcarboxamido, einem unsubstituierten Di(C₁-C₁₈-alkyl)aminoalkyl, unsubstituiertem (C₁-C₁₈)-Guanidinoalkyloxy, unsubstituiertem quaternärem (C₁-C₁₈)-Ammoniumalkylcarboxy und unsubstituiertem (C₁-C₁₈)-Guanidinoalkylcarboxy; und
R₁₈ unabhängig ausgewählt ist aus der Gruppe, bestehend aus Wasserstoff, einem unsubstituierten (C₁-C₁₈)-Alkyl, unsubstituiertem (C₁-C₁₈)-Hydroxyalkyl, unsubstituiertem (C₁-C₁₈)-Alkyloxy-(C₁-C₁₈)-alkyl, unsubstituiertem (C₁-C₁₈)-Alkylcarboxy-(C₁-C₁₈)-alkyl, unsubstituiertem (C₁-C₁₈)-Alkylamino-(C₁-C₁₈)-alkyl, unsubstituiertem (C₁-C₁₈)-Alkylamino-(C₁-C₁₈)-alkylamino, unsubstituiertem (C₁-C₁₈)-Alkylamino-(C₁-C₁₈)-alkylamino-(C₁-C₁₈)-alkylamino, einem unsubstituierten (C₁-C₁₈)-Aminoalkyl, einem unsubstituierten Arylamino-(C₁-C₁₈)-alkyl, einem unsubstituierten (C₁-C₁₈)-Aminoalkyloxy, einem unsubstituierten (C₁-C₁₈)-Aminoalkyloxy-(C₁-C₁₈)-alkyl, einem unsubstituierten (C₁-C₁₈)-Aminoalkylcarboxy, einem unsubstituierten (C₁-C₁₈)-Aminoalkylaminocarbonyl, einem unsubstituierten (C₁-C₁₈)-Aminoalkylcarboxamido, einem unsubstituierten Di(C₁-C₁₈-alkyl)aminoalkyl, unsubstituiertem (C₁-C₁₈)-Guanidinoalkyloxy, unsubstituiertem quaternärem (C₁-C₁₈)-Ammoniumalkylcarboxy, unsubstituiertem (C₁-C₁₈)-Guanidinoalkylcarboxy und einer Gruppe mit Amidfunktionalität, in der die Carbonylgruppe des Amids zwischen dem Amidostickstoff des Amids und dem kondensierten Ring D des Steroid-Grundgerüsts positioniert ist.

4. Salz nach einem der Ansprüche 1-3, wobei
R₃, R₇ und R₁₂ unabhängig voneinander ausgewählt sind aus der Gruppe, bestehend aus Wasserstoff, einem unsubstituierten (C₁-C₆)-Alkyl, unsubstituiertem (C₁-C₆)-Hydroxyalkyl, unsubstituiertem (C₁-C₁₆)-Alkyloxy-(C₁-C₅)-alkyl, unsubstituiertem (C₁-C₁₆)-Alkylcarboxy-(C₁-C₅)-alkyl, unsubstituiertem (C₁-C₁₆)-Alkylamino-(C₁-C₅)-alkyl, (C₁-C₁₆)-Alkylamino-(C₁-C₅)-alkylamino, unsubstituiertem (C₁-C₁₆)-Alkylamino-(C₁-C₁₆)-alkylamino-(C₁-C₅)-alkylamino, einem unsubstituierten (C₁-C₁₆)-Aminoalkyl, einem unsubstituierten Arylamino-(C₁-C₅)-alkyl, einem unsubstituierten (C₁-C₅)-Aminoalkyloxy, einem unsubstituierten (C₁-C₁₆)-Aminoalkyloxy-(C₁-C₅)-alkyl, einem unsubstituierten (C₁-C₅)-Aminoalkylcarboxy, einem unsubstituierten (C₁-C₅)-Aminoalkylaminocarbonyl, einem unsubstituierten (C₁-C₅)-Aminoalkylcarboxamido, einem unsubstituierten Di(C₁-C₅-alkyl)amino-(C₁-C₅)-alkyl, unsubstituiertem (C₁-C₅)-Guanidinoalkyloxy, unsubstituiertem quaternärem (C₁-C₁₆)-Ammoniumalkylcarboxy und unsubstituiertem (C₁-C₁₆)-Guanidinoalkylcarboxy; und
R₁₈ unabhängig ausgewählt ist aus der Gruppe, bestehend aus Wasserstoff, einem unsubstituierten (C₁-C₆)-Alkyl, unsubstituiertem (C₁-C₆)-Hydroxyalkyl, unsubstituiertem (C₁-C₁₆)-Alkyloxy-(C₁-C₅)-alkyl, unsubstituiertem (C₁-C₁₆)-Alkylcarboxy-(C₁-C₅)-alkyl, unsubstituiertem (C₁-C₁₆)-Alkylamino-(C₁-C₅)-alkyl, (C₁-C₁₆)-Alkylamino-(C₁-C₅)-alkylamino, unsubstituiertem (C₁-C₁₆)-Alkylamino-(C₁-C₁₆)-alkylamino-(C₁-C₅)-alkylamino, einem unsubstituierten (C₁-C₁₆)-Aminoalkyl, einem unsubstituierten Arylamino-(C₁-C₅)-alkyl, einem unsubstituierten (C₁-C₅)-Aminoalkyloxy, einem unsubstituierten (C₁-C₁₆)-Aminoalkyloxy-(C₁-C₅)-alkyl, einem unsubstituierten (C₁-C₅)-Aminoalkylcarboxy, einem unsubstituierten (C₁-C₅)-Aminoalkylaminocarbonyl, einem unsubstituierten (C₁-C₅)-Aminoalkylcarboxamido, einem unsubstituierten Di(C₁-C₅-alkyl)amino-(C₁-C₅)-alkyl, unsubstituiertem (C₁-C₅)-Guanidinoalkyloxy, unsubstituiertem quaternärem (C₁-C₁₆)-Ammoniumalkylcarboxy, unsubstituiertem (C₁-C₁₆)-Guanidinoalkylcarboxy und einer Gruppe mit Amidfunktionalität, in der die Carbonylgruppe des Amids zwischen dem Amidostickstoff des Amids und dem kondensierten Ring des Steroid-Grundgerüsts positioniert ist.

5. Salz nach einem der Ansprüche 1-4, wobei
R₃, R₇ und R₁₂ unabhängig voneinander ausgewählt sind aus der Gruppe, bestehend aus Aminoalkyloxy; Aminoalkylcarboxy; Alkylaminoalkyl; Alkoxycarbonylalkyl; Alkylcarbonylalkyl; Di(alkyl)aminoalkyl; Alkylcarboxyalkyl; und Hydroxyalkyl, z.B. wobei R₃, R₇ und R₁₂ unabhängig voneinander ausgewählt sind aus der Gruppe, bestehend aus Aminoalkyloxy und Aminoalkylcarboxy, wobei R₃, R₇ und R₁₂ vorzugsweise gleich sind; und
R₁₈ unabhängig ausgewählt ist aus der Gruppe, bestehend aus Aminoalkyloxy; Aminoalkylcarboxy; Alkylaminoalkyl; Alkoxycarbonylalkyl; Alkylcarbonylalkyl; Di(alkyl)aminoalkyl; Alkylcarboxyalkyl; Hydroxyalkyl und einer Gruppe mit Amidfunktionalität, in der die Carbonylgruppe des Amids zwischen dem Amidostickstoff des Amids und dem kondensierten Ring D des Steroid-Grundgerüsts positioniert ist, z.B. wobei R₁₈ Alkylaminoalkyl oder Alkoxycarbonylalkyl ist.

6. Salz nach einem der Ansprüche 1-5, wobei
R₃, R₇ und R₁₂ unabhängig voneinander ausgewählt sind aus der Gruppe, bestehend aus Amino-C₃-alkyloxy; Amino-C₃-alkylcarboxy; C₈-Alkylamino-C₅-alkyl; C₁₂-Alkylamino-C₅-alkyl; C₁₃-Alkylamino-C₅-alkyl; C₁₆-Alkylamino-C₅-alkyl, Di-(C₅-alkyl)amino-C₅-alkyl; C₆-Alkoxycarbonyl-C₄-alkyl; C₈-Alkoxycarbonyl-C₄-alkyl; C₁₀-Alkoxycarbonyl-C₄-alkyl; C₆-Alkylcarboxy-C₄-alkyl; C₈-Alkylcarboxy-C₄-alkyl, und C₁₀-Alkylcarboxy-C₄-alkyl; und
wobei R₃, R₇ und R₁₂ vorzugsweise unabhängig voneinander ausgewählt sind aus der Gruppe, bestehend aus Amino-C₃-alkyloxy; Amino-C₃-alkylcarboxy; C₈-Alkylamino-C₅-alkyl; C₁₂-Alkylamino-C₅-alkyl; C₁₃-Alkylamino-C₅-alkyl; C₁₆-Alkylamino-C₅-alkyl; Di-(C₅-alkyl)amino-C₅-alkyl; C₆-Alkoxycarbonyl-C₄-alkyl; C₈-Alkoxycarbonyl-C₄-alkyl; und C₁₀-Alkoxycarbonyl-C₄-alkyl,
wobei R₃, R₇ und R₁₂ stärker bevorzugt unabhängig voneinander ausgewählt sind aus der Gruppe, bestehend aus Amino-C₃-alkyloxy oder Amino-C₃-alkylcarboxy; und
R₁₈ unabhängig ausgewählt ist aus der Gruppe, bestehend aus Amino-C₃-alkyloxy; Amino-C₃-alkylcarboxy; C₈-Alkylamino-C₅-alkyl; C₁₂-Alkylamino-C₅-alkyl, C₁₃-Alkylamino-C₅-alkyl; C₁₆-Alkylamino-C₅-alkyl; Di-(C₅-alkyl)amino-C₅-alkyl; C₆-Alkoxycarbonyl-C₄-alkyl; C₈-Alkoxycarbonyl-C₄-alkyl; C₁₀-Alkoxycarbonyl-C₄-alkyl; C₆-Alkylcarboxy-C₄-alkyl; C₈-Alkylcarboxy-C₄-alkyl, C₁₀-Alkylcarboxy-C₄-alkyl; und einer Gruppe mit Amidfunktionalität, in der die Carbonylgruppe des Amids zwischen dem Amidostickstoff des Amids und dem kondensierten Ring D des Steroid-Grundgerüsts positioniert ist,
wobei R₁₈ vorzugsweise unabhängig ausgewählt ist aus der Gruppe, bestehend aus Amino-C₃-alkyloxy; Amino-C₃-alkylcarboxy; C₈-Alkylamino-C₅-alkyl; C₁₂-Alkylamino-C₅-alkyl; C₁₃-Alkylamino-C₅-alkyl; C₁₆-Alkylamino-C₅-alkyl; Di-(C₅-alkyl)amino-C₅-alkyl; C₆-Alkoxycarbonyl-C₄-alkyl; C₈-Alkoxycarbonyl-C₄-alkyl; C₁₀-Alkoxycarbonyl-C₄-alkyl; und einer Gruppe mit Amidfunktionalität, in der die Carbonylgruppe des Amids zwischen dem Amidostickstoff des Amids und dem kondensierten Ring D des Steroid-Grundgerüsts positioniert ist,
wobei R₁₈ stärker bevorzugt ausgewählt ist aus der Gruppe, bestehend aus C₈-Alkylamino-Cs-alkyl oder C₈-Alkoxycarbonyl-C₄-alkyl.

7. Salz nach einem der Ansprüche 1-6, wobei das CSA ausgewählt ist aus der Gruppe, bestehend aus: 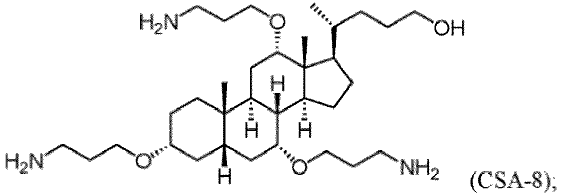 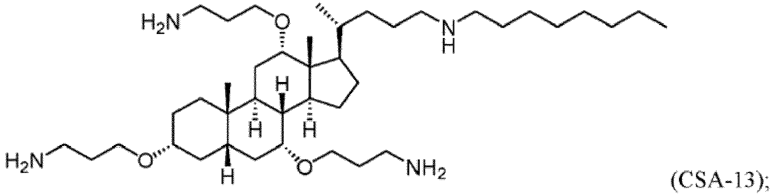 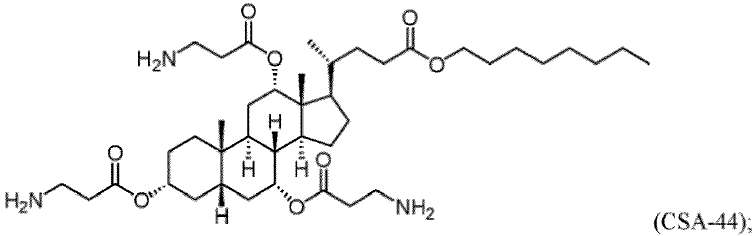 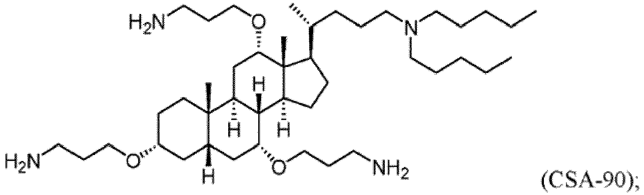 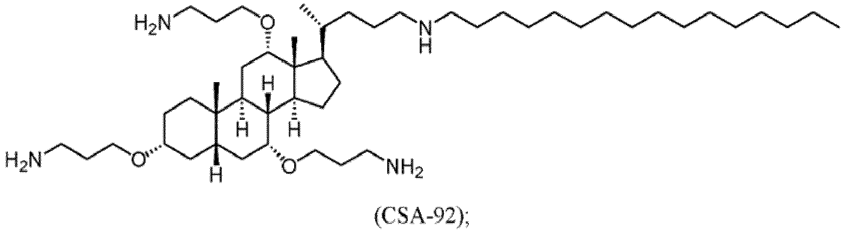 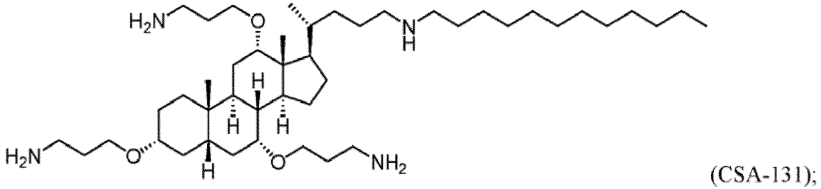 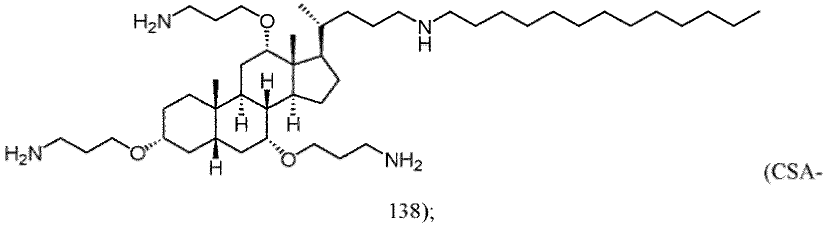 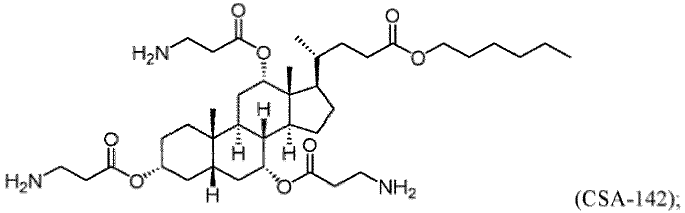 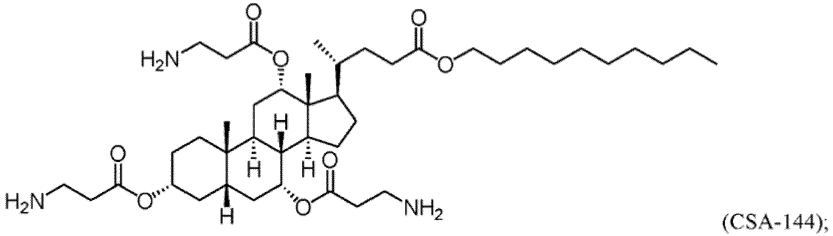 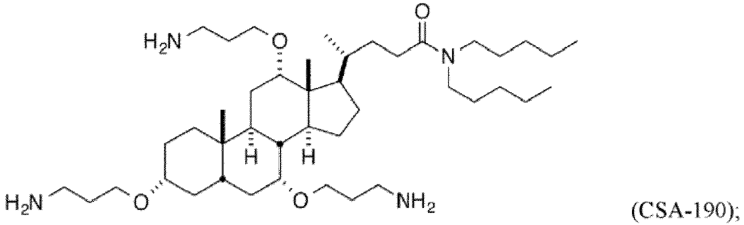 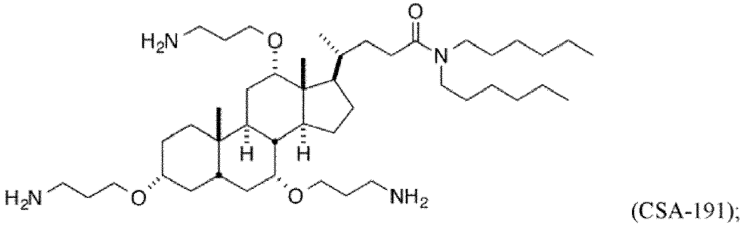 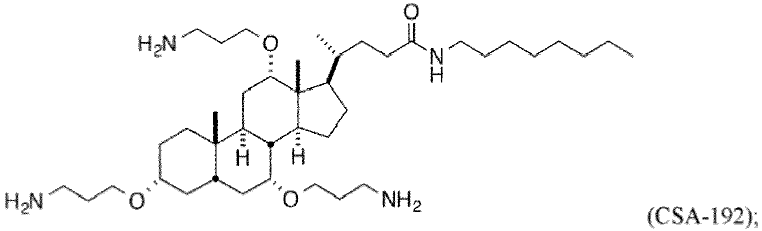 und

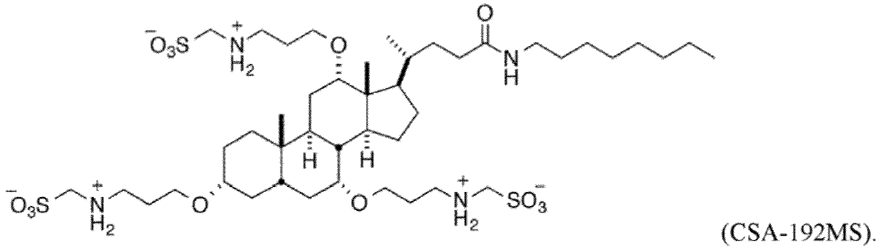

8. Salz nach einem der Ansprüche 1-7, wobei es sich bei dem Salz um einen Feststoff handelt, wie z.B. einen fließfähigen Feststoff, der vorzugsweise kristallin ist, und/oder wobei das Salz lagerstabil und/oder mikronisiert ist.

9. Salz nach einem der Ansprüche 1-8, wobei das Salz ein 1,5-Naphthalindisulfonsäure-Diadditionssalz von CSA-13 ist und **gekennzeichnet ist durch**:
ein Röntgenpulverbeugungsmuster mit den folgenden 2θ-Werten (± 0,2): 4,216; 4,629; 8,29; 9,13; 9,739; 12,641; 14,457; 15,864; 18,610; 19,200; 20,242; 20,803; 21,512; 22,014; 22,57; 23,169; 23,63; 25,227; 26,44; 37,05; und 39,33,
ein Röntgenpulverbeugungsmuster mit den folgenden 2θ-Werten (± 0,2): 4,200; 4,606; 8,292; 9,113; 9,728; 11,71; 12,625; 13,95; 14,444; 15,826; 18,622; 19,20; 20,22; 20,767; 21,482; 21,958; 22,53; 23,12; 23,61; 25,26; 26,55; und 37.01, oder
wobei das Salz ein 1,5-Naphthalindisulfonsäure-Diadditionssalz von CSA-131 ist und **gekennzeichnet ist durch**:
ein Röntgenpulverbeugungsmuster mit den folgenden 2θ-Werten (± 0,2): 4,1922; 4,4257, 6,118, 8,3931, 9,6769, 11,7232, 13,4959, 15,0514, 16,5064, 17,8322, 18,7671, 19,3449, 20,596, 21,5538, 22,7706, 24,6057, 26,7689 und 36,2048.

10. Formulierung, umfassend: ein Salz nach einem der Ansprüche 1-8 und einen pharmazeutisch verträglichen Hilfsstoff.

11. Verfahren zur Herstellung des Salzes nach einem der Ansprüche 1-10, umfassend:
das Verdünnen einer freien Ausgangsbase eines CSA mit einem Lösungsmittel;
das Hinzufügen von mindestens einem Äquivalent einer Säure zu dem verdünnten CSA im Lösungsmittel, um ein Reaktionsgemisch bereitzustellen;
das Ausfällen oder einen Temperaturwechsel des Reaktionsgemisches, wie z.B. einen Temperaturwechsel für zumindest etwa 48 Stunden; und
das Isolieren eines CSA-Salzes.

12. Verfahren nach Anspruch 11, ferner umfassend das Verwenden eines Antilösungsmittels oder das Eindampfen des Lösungsmittels beim Isolieren des CSA-Salzes.

13. Verfahren nach Anspruch 11 oder 12, wobei das CSA-Salz ein Feststoff ist, z.B. wobei das CSA-Salz kristallin oder amorph, lagerstabil, fließfähig und/oder mikronisiert ist.

## Revendications

1. Sel de di-addition d'acide 1,5-naphtalènedisulfonique d'un composé antimicrobien stéroïdien cationique (CSA), dans lequel le CSA est un composé de Formule III : 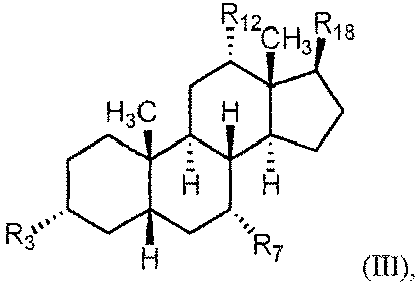 où,
R₃, R₇ et R₁₂ sont indépendamment choisis parmi le groupe constitué d'hydrogène, hydroxyle, (C₁-C₂₂) alkyle, (C₁-C₂₂) hydroxyalkyle, (C₁-C₂₂) alkyloxy-(C₁-C₂₂) alkyle, (C₁-C₂₂) alkylcarboxy-(C₁-C₂₂) alkyle, (C₁-C₂₂) alkylamino-(C₁-C₂₂) alkyle, (C₁-C₂₂) alkylamino-(C₁-C₂₂) alkylamino, (C₁-C₂₂) alkylamino-(C₁-C₂₂) alkylamino-(C₁-C₂₂) alkylamino, (C₁-C₂₂) aminoalkyl, aryle, arylamino-(C₁-C₂₂) alkyle, (C₁-C₂₂) haloalkyle, alcényle en C₂-C₆, alcynyle en C₂-C₆, oxo, un groupe de liaison attaché à un second stéroïde, (C₁-C₂₂) aminoalkyloxy, (C₁-C₂₂) aminoalkyloxy-(C₁-C₂₂) alkyle, (C₁-C₂₂) aminoalkylcarboxy, (C₁-C₂₂) aminoalkylaminocarbonyle, (C₁-C₂₂) aminoalkylcarboxamido, di(alkyle en C₁-C₂₂)aminoalkyle, H₂N-HC(Q₅)-C(O)-O-, H₂N-HC(Q₅)-C(O)-N(H)-, (C₁-C₂₂) azidoalkyloxy, (C₁-C₂₂) cyanoalkyloxy, P.G.-HN-HC(Q₅)-C(O)-O-, (C₁-C₂₂) guanidinoalkyloxy, (C₁-C₂₂) alkylcarboxy d'ammonium quaternaire, et (C₁-C₂₂) guanidinoalkyle carboxy, où Q₅ est une chaîne latérale de tout acide aminé (y compris une chaîne latérale de glycine, c'est-à-dire H) et P.G. est un groupe protecteur aminé ; et
R₁₈ est choisi parmi le groupe constitué d'hydrogène, hydroxyle, (C₁-C₂₂) alkyle, (C₁-C₂₂) hydroxyalkyle, (C₁-C₂₂) alkyloxy-(C₁-C₂₂) alkyle, (C₁-C₂₂) alkylcarboxy-(Ci-C₂₂) alkyle, (C₁-C₂₂) alkylamino-(C₁-C₂₂) alkyle, (C₁-C₂₂) alkylamino-(C₁-C₂₂) alkylamino, (C₁-C₂₂) alkylamino-(C₁-C₂₂) alkylamino-(C₁-C₂₂) alkylamino, (C₁-C₂₂) aminoalkyl, aryle, arylamino-(C₁-C₂₂) alkyle, (C₁-C₂₂) haloalkyle, alcényle en C₂-C₆, alcynyle en C₂-C₆, oxo, un groupe de liaison attaché à un second stéroïde, (C₁-C₂₂) aminoalkyloxy, (C₁-C₂₂) aminoalkyloxy-(C₁-C₂₂) alkyle, (C₁-C₂₂) aminoalkylcarboxy, (C₁-C₂₂) aminoalkylaminocarbonyle, (C₁-C₂₂) aminoalkyl-carboxamido, di(alkyle en C₁-C₂₂)aminoalkyle, H₂N-HC(Q₅)-C(O)-O-, H₂N-HC(Q₅)-C(O)-N(H)-, (C₁-C₂₂) azidoalkyloxy, (C₁-C₂₂) cyanoalkyloxy, P.G.-HN-HC(Q₅)-C(O)-O-, (C₁-C₂₂) guanidinoalkyloxy, (C₁-C₂₂) alkylcarboxy d'ammonium quaternaire, (C₁-C₂₂) guanidinoalkyle carboxy, et un groupe ayant une fonctionnalité amide dans lequel le groupe carbonyle de l'amide est positionné entre l'azote amido de l'amide et un cycle fusionné D du squelette stéroïdien, où Q₅ est une chaîne latérale de tout acide aminé (y compris une chaîne latérale de glycine, c'est-à-dire H), et P.G. est un groupe protecteur aminé,
à condition qu'au moins deux ou trois parmi R₃, R₇, R₁₂, et R₁₈ soient choisis indépendamment parmi le groupe constitué de (C₁-C₂₂) aminoalkyle, (C₁-C₂₂) aminoalkyloxy, (C₁-C₂₂) alkylcarboxy-(C₁-C₂₂) alkyle, (C₁-C₂₂) alkylamino-(C₁-C₂₂) alkylamino, (C₁-C₂₂) alkylamino-(C₁-C₂₂) alkylamino (C₁-C₂₂) alkylamino, (C₁-C₂₂) aminoalkylcarboxy, arylamino (C₁-C₂₂) alkyle, (C₁-C₂₂) aminoalkyloxy (C₁-C₂₂) aminoalkylaminocarbonyle, (C₁-C₂₂) aminoalkylaminocarbonyle, (C₁-C₂₂) aminoalkylcarboxyamido, (C₁-C₂₂) alkylcarboxy d'ammonium quaternaire, di(alkyle en C₁-C₂₂)aminoalkyle, H₂N-HC(Q₅)-C(O)-O-, H₂N-HC(Q₅)-C(O)-N(H)-, (C₁-C₂₂) azidoalkyloxy, (C₁-C₂₂) cyanoalkyloxy, P.G.-HN-HC(Q₅)-C(O)-O-, (C₁-C₂₂) guanidinoalkyloxy, et (C₁-C₂₂) guanidinoalkylcarboxy.

2. Sel selon la revendication 1, dans lequel R₁₈ a la structure suivante :
-R₂₀-(C=O)-N-R₂₁R₂₂
où,
R₂₀ est omis ou est un alkyle, alcényle, alcynyle, ou aryle substitué ou non substitué ; et
R₂₁ et R₂₂ sont indépendamment choisis parmi le groupe constitué d'hydrogène, d'un alkyle substitué ou non substitué, d'un alcényle substitué ou non substitué, d'un alcynyle substitué ou non substitué, ou d'un aryle substitué ou non substitué, tel que dans lequel R₂₁ et R₂₂ sont indépendamment choisis parmi le groupe constitué d'hydrogène, alkyle en C₁-C₂₄ facultativement substitué, alcényle en C₂-C₂₄ facultativement substitué, alcynyle en C₂-C₂₄ facultativement substitué, aryle en C₆ ou C₁₀ facultativement substitué, hétéroaryle de 5 à 10 éléments facultativement substitué, hétérocyclyle de 5 à 10 éléments facultativement substitué, aralkyle en C₇₋₁₃ facultativement substitué, (hétéroaryle de 5 à 10 éléments)facultativement substitué-alkyle en C₁-C₆, carbocyclyle en C₃₋₁₀ facultativement substitué, (carbocyclyl)alkyle en C₄₋₁₀ facultativement substitué, (hétérocyclyle de 5 à 10 éléments)-alkyle en C₁-C₆ facultativement substitué, amido facultativement substitué, et un groupe protecteur amine approprié, à condition qu'au moins l'un parmi R₂₁ et R₂₂ ne soit pas de l'hydrogène,
et facultativement dans lequel R₂₁ et R₂₂, conjointement avec les atomes auxquels ils sont liés, forment un cycle hétérocyclyle de 5 à 10 éléments facultativement substitué.

3. Sel selon la revendication 1 ou 2, dans lequel
R₃, R₇ et R₁₂ sont choisis indépendamment parmi le groupe constitué d'hydrogène, (C₁-C₁₈) alkyle non substitué, (C₁-C₁₈) hydroxyalkyle non substitué, (C₁-C₁₈) alkyloxy-(C₁-C₁₈) alkyle non substitué, (C₁-C₁₈) alkylcarboxy-(C₁-C₁₈) alkyle non substitué, (C₁-C₁₈) alkylamino-(C₁-C₁₈) alkyle non substitué, (C₁-C₁₈) alkylamino-(C₁-C₁₈) alkylamino non substitué, (C₁-C₁₈) alkylamino-(C₁-C₁₈) alkylamino-(C₁-C₁₈) alkylamino non substitué, (C₁-C₁₈) aminoalkyle non substitué, arylamino-(C₁-C₁₈) alkyle non substitué, (C₁-C₁₈) aminoalkyloxy non substitué, (C₁-C₁₈) aminoalkyloxy-(C₁-C₁₈) alkyle non substitué, (C₁-C₁₈) aminoalkylcarboxy non substitué, (C₁-C₁₈) aminoalkylaminocarbonyle non substitué, (Ci-Cis) aminoalkylcarboxamido non substitué, di(alkyle en C₁-C₁₈)aminoalkyle non substitué, (C₁-C₁₈) guanidinoalkyloxy non substitué, (C₁-C₁₈) alkylcarboxy ammonium quaternaire non substitué, et (C₁-C₁₈) guanidinoalkyle carboxy non substitué ; et
R₁₈ est indépendamment choisi parmi le groupe constitué d'hydrogène, (C₁-C₁₈) alkyle non substitué, (C₁-C₁₈) hydroxyalkyle non substitué, (C₁-C₁₈) alkyloxy-(C₁-C₁₈) alkyle non substitué, (C₁-C₁₈) alkylcarboxy-(C₁-C₁₈) alkyle non substitué, (C₁-C₁₈) alkylamino-(C₁-C₁₈) alkyle non substitué, (C₁-C₁₈) alkylamino-(C₁-C₁₈) alkylamino non substitué, (C₁-C₁₈) alkylamino-(C₁-C₁₈) alkylamino-(C₁-C₁₈) alkylamino non substitué, (C₁-C₁₈) aminoalkyle non substitué, arylamino-(C₁-C₁₈) alkyle non substitué, (C₁-C₁₈) aminoalkyloxy non substitué, (C₁-C₁₈) aminoalkyloxy-(C₁-C₁₈) alkyle non substitué, (C₁-C₁₈) aminoalkylcarboxy non substitué, (C₁-C₁₈) aminoalkylaminocarbonyle non substitué, (C₁-C₁₈) aminoalkylcarboxamido non substitué, di(alkyle en C₁-C₁₈)aminoalkyle non substitué, (C₁-C₁₈) guanidinoalkyloxy non substitué, (C₁-C₁₈) alkylcarboxy ammonium quaternaire non substitué, (C₁-C₁₈) guanidinoalkyle carboxy non substitué, et un groupe ayant une fonctionnalité amide dans lequel le groupe carbonyle de l'amide est positionné entre l'azote amido de l'amide et un cycle fusionné D du squelette stéroïdien.

4. Sel selon l'une quelconque des revendications 1 à 3, dans lequel,
R₃, R₇ et R₁₂ sont indépendamment choisis parmi le groupe constitué d'hydrogène, (C₁-C₆) alkyle non substitué, (C₁-C₆) hydroxyalkyle non substitué, (C₁-C₆) alkyloxy-(C₁-C₅) alkyle non substitué, (C₁-C₁₆) alkylcarboxy-(C₁-C₅) alkyle non substitué, (C₁-C₁₆) alkylamino-(C₁-C₅) alkyle non substitué, (C₁-C₁₆) alkylamino-(C₁-C₅) alkylamino, (C₁-C₁₆) alkylamino-(C₁-C₁₆) alkylamino-(C₁-C₅) alkylamino non substitué, (C₁-C₁₆) aminoalkyle non substitué, arylamino-(C₁-C₅) alkyle non substitué, (C₁-C₅) aminoalkyloxy non substitué, (C₁-C₁₆) aminoalkyloxy-(C₁-C₅) alkyle non substitué, (C₁-C₅) aminoalkylcarboxy non substitué, (C₁-C₅) aminoalkylaminocarbonyle non substitué, (C₁-C₅) aminoalkylcarboxamido non substitué, di(alkyle en C₁-C₅)amino-(C₁-C₅) alkyle non substitué, (C₁-C₅) guanidinoalkyloxy non substitué, (C₁-C₁₆) alkylcarboxy d'ammonium quaternaire non substitué, et (C₁-C₁₆) guanidinoalkylcarboxy non substitué ; et
R₁₈ est indépendamment choisi parmi le groupe constitué d'hydrogène, (C₁-C₆) alkyle non substitué, (C₁-C₆) hydroxyalkyle non substitué, (C₁-C₆) alkyloxy-(C₁-C₅) alkyle non substitué, (C₁-C₁₆) alkylcarboxy-(C₁-C₅) alkyle non substitué, (C₁-C₁₆) alkylamino-(C₁-C₅) alkyle non substitué, (C₁-C₁₆) alkylamino-(C₁-C₅) alkylamino, (C₁-C₁₆) alkylamino-(C₁-C₁₆) alkylamino-(C₁-C₅) alkylamino non substitué, (C₁-C₁₆) aminoalkyle non substitué, arylamino-(C₁-C₅) alkyle non substitué, (C₁-C₅) aminoalkyloxy non substitué, (C₁-C₁₆) aminoalkyloxy-(C₁-C₅) alkyle non substitué, (C₁-C₅) aminoalkylcarboxy non substitué, (C₁-C₅) aminoalkylaminocarbonyle non substitué, (C₁-C₅) aminoalkylcarboxamido non substitué, di(alkyle en C₁-C₅)amino-(C₁-C₅) alkyle non substitué, (C₁-C₅) guanidinoalkyloxy non substitué, (C₁-C₁₆) alkylcarboxy d'ammonium quaternaire non substitué, et (C₁-C₁₆) guanidinoalkylcarboxy non substitué, et un groupe ayant une fonctionnalité amide dans lequel le groupe carbonyle de l'amide est positionné entre l'azote amido de l'amide et un cycle fusionné du squelette stéroïdien.

5. Sel selon l'une quelconque des revendications 1 à 4, dans lequel
R₃, R₇ et R₁₂ sont choisis indépendamment parmi le groupe constitué d'aminoalkyloxy ; aminoalkylcarboxy ; alkylaminoalkyle ; alcoxycarbonylalkyle ; alkylcarbonylalkyle ; di(alkyl)aminoalkyle ; alkylcarboxyalkyle ; et hydroxyalkyle, tel que dans lequel R₃, R₇ et R₁₂ sont indépendamment choisis parmi le groupe constitué d'aminoalkyloxy et aminoalkylcarboxy, de préférence où R₃, R₇ et R₁₂ sont identiques ; et
R18 est indépendamment sélectionné parmi le groupe constitué d'aminoalkyloxy ; aminoalkylcarboxy ; alkylaminoalkyle ; alcoxycarbonylalkyle ; alkylcarbonylalkyle ; di(alkyl)aminoalkyle ; alkylcarboxyalkyle ; hydroxyalkyle, et un groupe ayant une fonctionnalité amide dans lequel le groupe carbonyle de l'amide est positionné entre l'azote amido de l'amide et un cycle fusionné D du squelette stéroïdien, tel que dans lequel R₁₈ est un alkylaminoalkyle ou un alcoxycarbonylalkyle.

6. Sel selon l'une quelconque des revendications 1 à 5, dans lequel
R₃, R₇ et R₁₂ sont choisis indépendamment parmi le groupe constitué d'amino-C₃-alkyloxy ; amino-C₃-alkyl-carboxy ; C₈-alkylamino-C₅-alkyle ; C₁₂-alkylamino-C₅-alkyle ; C₁₃-alkylamino-C₅-alkyle ; C₁₆-alkylamino-C₅-alkyle ; di-(C₅-alkyle)amino-C₅-alkyle ; C₆-alcoxy-carbonyl-C₄-alkyle ; C₈-alcoxy-carbonyl-C₄-alkyle ; C₁₀-alcoxy-carbonyl-C₄-alkyle ; C₆-alkyl-carboxy-C₄-alkyle ; C₈-alkyl-carboxy-C₄-alkyle ; et C₁₀-alkyl-carboxy-C₄-alkyle ; et
de préférence dans lequel R₃, R₇ et R₁₂ sont choisis indépendamment parmi le groupe constitué d'amino-C₃-alkyloxy ; amino-C₃-alkyl-carboxy ; C₈-alkylamino-C₅-alkyle ; C₁₂-alkylamino-C₅-alkyle ; C₁₃-alkylamino-C₅-alkyle ; C₁₆-alkylamino-C₅-alkyle ; di-(C₅-alkyle)amino-C₅-alkyle ; C₆-alcoxy-carbonyl-C₄-alkyle ; C₈-alcoxy-carbonyl-C₄-alkyle ; et C₁₀-alcoxy-carbonyl-C₄-alkyle,
de manière plus préférée dans lequel où R₃, R₇ et R₁₂ sont choisis indépendamment parmi le groupe constitué d'amino-C3-alkyloxy ou amino-C₃-alkyl-carboxy ; et
R₁₈ est choisi indépendamment parmi le groupe constitué d'amino-C₃-alkyloxy ; amino-C₃-alkyl-carboxy ; C₈-alkylamino-C₅-alkyle ; C₁₂-alkylamino-C₅-alkyle ; C₁₃-alkylamino-C₅-alkyle ; C₁₆-alkylamino-C₅-alkyle ; di-(C₅-alkyle)amino-C₅-alkyle ; C₆-alcoxy-carbonyl-C₄-alkyle ; C₈-alcoxy-carbonyl-C₄-alkyle ; C₁₀-alcoxy-carbonyl-C₄-alkyle ; C₆-alkyl-carboxy-C₄-alkyle ; C₈-alkyl-carboxy-C₄-alkyle ; C₁₀-alkyl-carboxy-C₄-alkyle ; et un groupe ayant une fonctionnalité amide dans lequel le groupe carbonyle de l'amide est positionné entre l'azote amido de l'amide et un cycle fusionné D du squelette stéroïdien,
de préférence dans lequel R₁₈ est choisi indépendamment parmi le groupe constitué d'amino-C₃-alkyloxy ; amino-C₃-alkyl-carboxy ; C₈-alkylamino-C₅-alkyle ; C₁₂-alkylamino-C₅-alkyle ; C₁₃-alkylamino-C₅-alkyle ; C₁₆-alkylamino-C₅-alkyle ; di-(C₅-alkyle)amino-C₅-alkyle ; C₆-alcoxy-carbonyl-C₄-alkyle ; C₈-alcoxy-carbonyl-C₄-alkyle ; C₁₀-alcoxy-carbonyl-C₄-alkyle ; et un groupe ayant une fonctionnalité amide dans lequel le groupe carbonyle de l'amide est positionné entre l'azote amido de l'amide et un cycle fusionné D du squelette stéroïdien,
de manière plus préférée dans lequel R₁₈ est choisi parmi le groupe constitué de C₈-alkylamino-C₅-alkyle ou C₈-alcoxy-carbonyl-C₄-alkyle.

7. Sel selon l'une quelconque des revendications 1 à 6, dans lequel le CSA est choisi parmi le groupe constitué de :

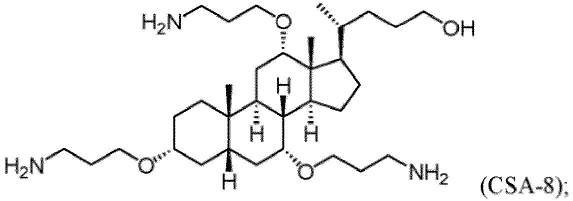

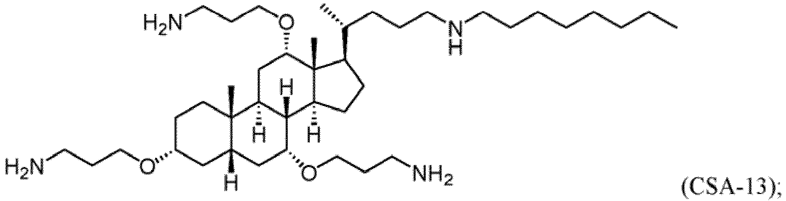

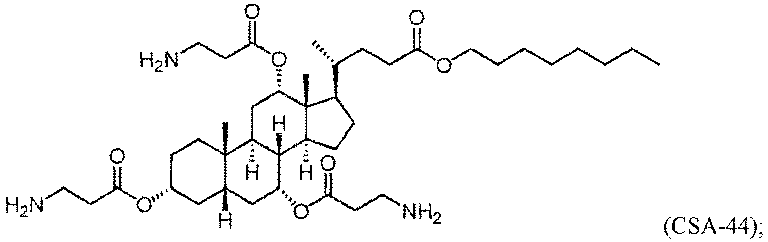

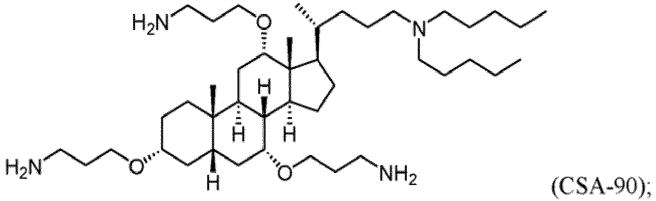

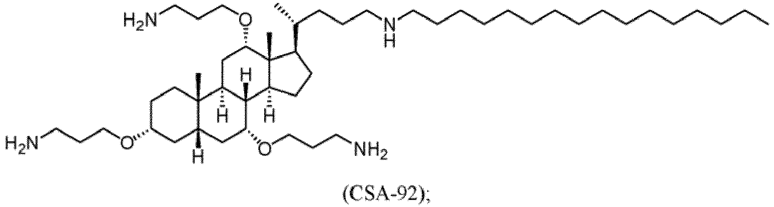

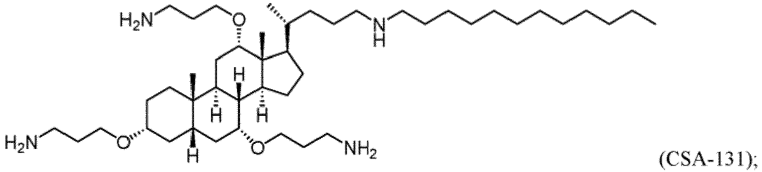

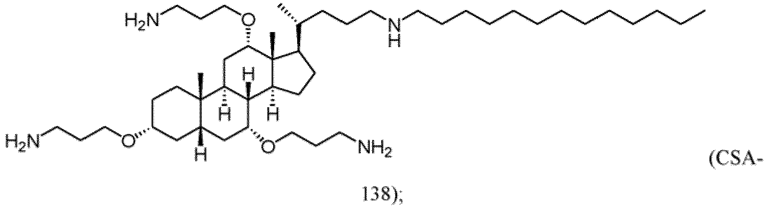

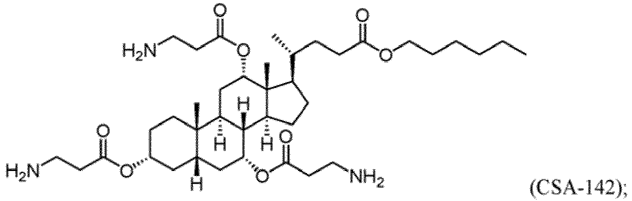

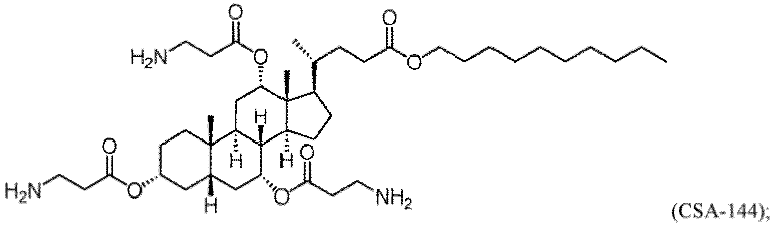

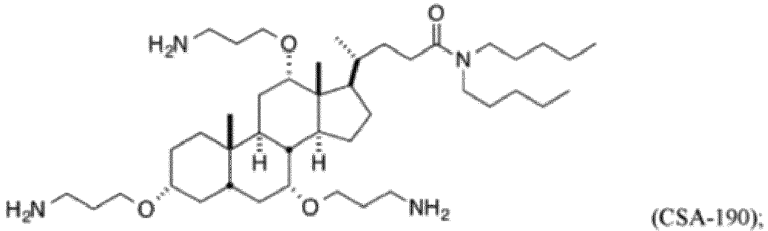

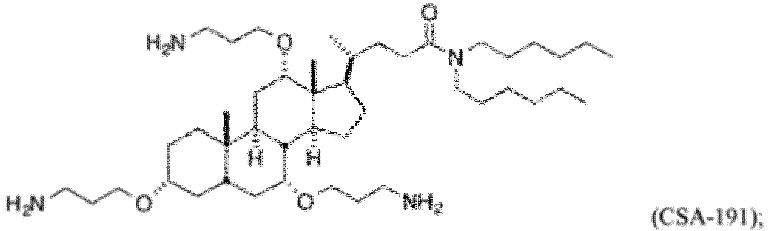

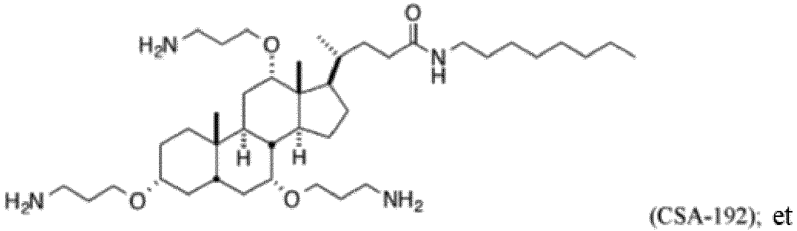

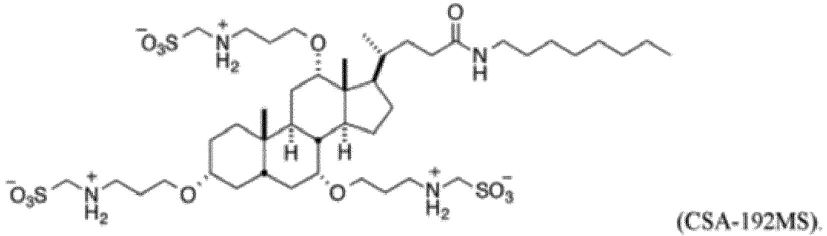

8. Sel selon l'une quelconque des revendications 1 à 7, dans lequel le sel est un solide, tel qu'un solide fluide, de préférence cristallin et/ou dans lequel le sel est stable au stockage et/ou micronisé.

9. Sel selon l'une quelconque des revendications 1 à 8, dans lequel le sel est un sel de di-addition d'acide 1,5-naphtalènedisulfonique de CSA-13 et est **caractérisé par** :
un diagramme de diffraction des rayons X sur poudre ayant les valeurs 2θ suivantes (± 0,2) : 4,216 ; 4,629 ; 8,29 ; 9,13 ; 9,739 ; 12,641 ; 14,457 ; 15,864 ; 18,610 ; 19,200 ; 20,242 ; 20,803 ; 21,512 ; 22,014 ; 22,57 ; 23,169 ; 23,63 ; 25,227 ; 26,44 ; 37,05 ; et 39,33,
un diagramme de diffraction des rayons X sur poudre ayant les valeurs 2θ suivantes (± 0,2) : 4,200 ; 4,606 ; 8,292 ; 9,113 ; 9,728 ; 11,71 ; 12,625 ; 13,95 ; 14,444 ; 15,826 ; 18,622 ; 19,20 ; 20,22 ; 20,767 ; 21,482 ; 21,958 ; 22,53 ; 23,12 ; 23,61 ; 25,26 ; 26,55 ; et 37,01, ou
dans lequel le sel est un sel de di-addition d'acide 1,5-naphtalènedisulfonique de CSA-131 et est **caractérisé par** :
un diagramme de diffraction des rayons X sur poudre ayant les valeurs 2θ suivantes (± 0,2) : 4,1922 ; 4,4257, 6,118, 8,3931, 9,6769, 11,7232, 13,4959, 15,0514, 16,5064, 17,8322, 18,7671, 19,3449, 20,596, 21,5538, 22,7706, 24,6057, 26,7689 et 36,2048.

10. Formulation, comprenant : un sel selon l'une quelconque des revendications 1 à 8 et un excipient pharmaceutiquement acceptable.

11. Procédé de préparation du sel selon l'une quelconque des revendications 1 à 10, comprenant les étapes consistant à :
diluer une base libre de départ d'un CSA avec un solvant ;
ajouter au moins un équivalent d'un acide au CSA dilué dans un solvant pour donner un mélange réactionnel ;
précipiter ou cycler en température le mélange réactionnel, tel qu'un cyclage en température pendant au moins 48 heures environ ; et
isoler un sel de CSA.

12. Procédé selon la revendication 11, comprenant en outre l'utilisation d'un anti-solvant ou d'une évaporation de solvant lors de l'isolement du sel de CSA.

13. Procédé selon la revendication 11 ou 12, dans lequel le sel de CSA est un solide, tel que dans lequel le sel de CSA est cristallin ou amorphe, stable au stockage, fluide et/ou micronisé.
